# EUROPEAN PATENT SPECIFICATION

(11) **EP 2 672 835 B1**
(45) Date of publication and mention of the grant of the patent: **08.04.2015**
(21) Application number: 12703250.6
(22) Date of filing: 07.02.2012
(51) Int. Cl.: C07C 259/06, A23L 1/03, A23L 2/60, A61Q 11/00, A23G 1/32

(54) **SWEETNESS ENHANCERS, SWEETENER COMPOSITIONS, METHODS OF MAKING THE SAME AND CONSUMABLES CONTAINING THE SAME**
SÜßKRAFTVERSTÄRKER, SÜßSTOFFZUSAMMENSETZUNGEN, VERFAHREN ZU IHRER HERSTELLUNG UND DIESE ENTHALTENE PRODUKTE FÜR DEN VERZEHR
EXHAUSTEURS DE GOÛT SUCRÉ, COMPOSITIONS ÉDULCORANTES, PROCÉDÉS PERMETTANT DE LES FABRIQUER ET PRODUITS ALIMENTAIRES LES CONTENANT

(30) Priority: 08.02.2011 EP 11001010; 08.02.2011 US 201161440821 P
(43) Date of publication of application: 18.12.2013
(73) Proprietor: Nutrinova Nutrition Specialties & Food Ingredients GmbH, 65926 Frankfurt am Main (DE)
(72) Inventor: KROHN, Michael, 64653 Lorsch (DE); SEIBERT, Simon, 64289 Darmstadt (DE); KLEBER, Alice, 64625 Bensheim (DE); WONSCHIK, Johann, 37647 Brevörde (DE); SIEMS, Karsten, 14552 Michendorf (DE); MÜLLER, Jens-Peter, 12161 Berlin (DE); HAUSTEDT, Lars, Ole, 14471 Potsdam (DE); SCHWARZ, Oliver, 15711 Königs Wusterhausen (DE)
(74) Representative: Lahrtz, Fritz
(86) International application number: PCT/EP2012/000537
(87) International publication number: WO 2012/107201

(56) References cited:
- WO-A1-2007/110115
- WO-A1-2008/147726

## Description

### Field of the Invention

The present invention relates to novel sweetness enhancer compounds of the formula (I), 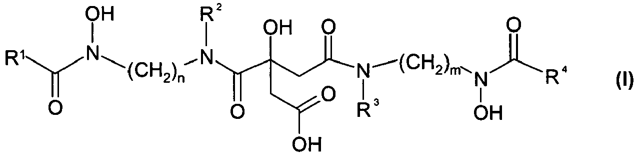 wherein R¹, R², R³, R⁴, n and m have the designations cited below or a stereoisomer or a salt or a hydrate thereof, to the use of a compound of formula (I) or a stereoisomer or a salt or a hydrate thereof as a sweetness enhancer, to sweetener compositions comprising at least one sweetener and a compound of formula (I) or a stereoisomer or a salt or a hydrate thereof, and optionally a pregelatinized starch, to a method for the manufacture of a compound of formula (I) or a stereoisomer or a salt or a hydrate thereof, to methods of making the sweetener compositions and to tabletop sweetener compositions comprising a compound of formula (I) or a stereoisomer or a salt or a hydrate thereof. Further, the invention relates to consumables comprising a consumable product and a compound of formula (I) or a stereoisomer or a salt or a hydrate thereof. Preferred consumable products are water-based consumables, solid dry consumables, dairy products, dairy-derived products and dairy-alternative products.

### Background of the Invention

The use of non-caloric high-intensity sweeteners is increasing due to health concerns raised over childhood obesity, type II diabetes, and related illnesses. Thus, a demand exists for sweeteners having sweetnesses significantly higher than those of conventional sweeteners, such as granulated sugar (sucrose) or high-fructose corn syrup (HFCS). Many non-caloric or low-caloric sweeteners, however, are prohibitively expensive and/or contain unpleasant off-flavors and/or have unexpected and less-than-desirable sweetness profiles.

Furthermore, it is of interest to enhance desired flavor sensations, e.g., sweet taste, of non-caloric or low-caloric or standard (caloric) sweeteners, conventional or otherwise.

Compounds that can enhance certain flavor sensations are of great interest and may allow not only improvement and/or intensification of the perceived flavor but also the ability to reach a certain flavor intensity using reduced concentrations of flavor ingredients. As one example, by employing an enhancer, less sweetener may be necessary to achieve a desired sweetness level, which may result in less calories and/or associated undesirable flavor notes/off-notes. Thus, a need exists for sweetness enhancers.

Non-caloric, high intensity sweeteners are known to have sweetness levels or "sweetnesses" that are significantly higher than those of conventional sweeteners. These non-caloric sweeteners often are added to the consumable products to replace at least a portion of the conventional caloric sweeteners, which results in a consumable product having a sweet flavor and a reduced amount of caloric sweetener. Acesulfame potassium is an exemplary non-caloric, high intensity sweetener.

The patent WO2008147726 discloses for example some amido compounds as sweetness enhancers.

Typically, the caloric sweeteners and/or the non-caloric sweeteners are combined with other components, e.g., surfactants, emulsifiers, gums, or other sweeteners, to form sweetener compositions. These other components often improve the physical or chemical properties of the caloric or non-caloric sweetener. The sweetener compositions then may be incorporated into the respective consumable product. In most cases, the sweetener or the sweetness associated therewith is released from the consumable product quickly upon consumption of the consumable product. This release accounts for the sweetness realized by the consumer.

For some consumable products, e.g., chewing gum and other confections, once the consumable product begins to be consumed, it is desirable to have a prolonged release rate of sweetener from the consumable product. As a result, the sensation of sweetness is enjoyed by the consumer over a prolonged period of time. Conventional sweeteners, however, have been known to have more rapid release rates from the respective consumable product. As such, a majority of the sweetener is quickly released from the consumable product immediately upon consumption and very little sweetener then is left to be released during the remainder of the lifetime of the consumable product. As one example, a chewing gum comprising a conventional sweetener composition is often considered to be very sweet as the gum is initially chewed, but after some minutes of chewing, the gum is considered to be significantly less sweet.

Thus, a need exists for sweetener compositions that employ sweeteners, e.g., non-caloric, high intensity sweeteners, and that provide for prolonged release rates of these sweeteners from a consumable product to the palate of the consumer. As such, the sweetness associated with the sweetener composition may be recognized by the consumer over a longer period of time and result a prolonged sweetening sensation is realized by the consumer.

### Brief Description of the Figures

**Fig. 1** is a chart showing the results of the recombinant human taste receptor T1R2/T1R3 dependent cell based assay with the compound of formula (Ia).

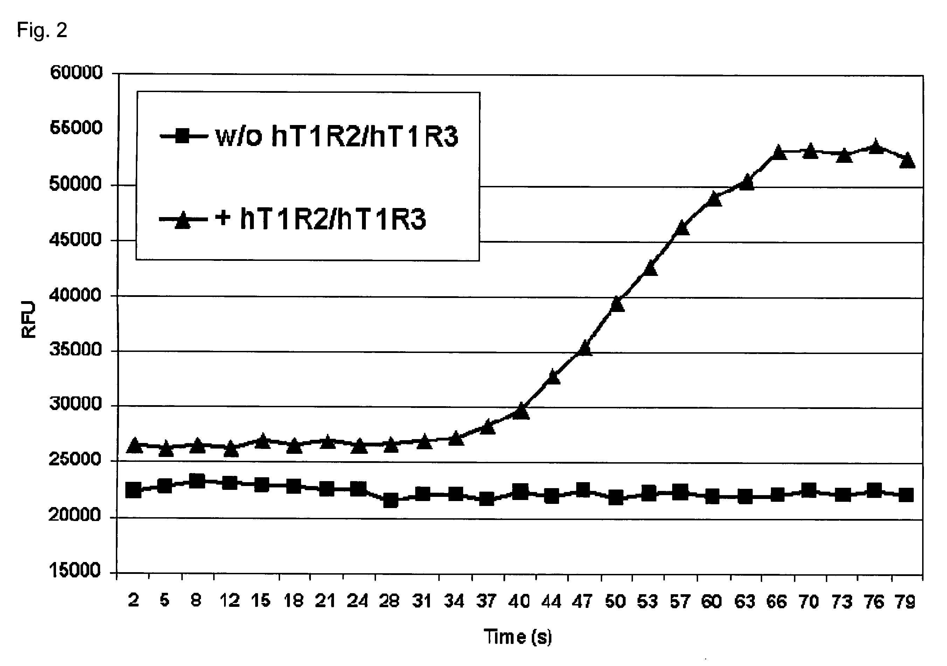

**Fig. 2** is a chart showing the selective stimulation of receptor carrying cells by compound of formula (Ia) is verified by measuring the time response in the cell assay over a period of 78 seconds.
**Fig. 3** is a table summarizing the results of the taste and spit assay with the compound of formula (Ia).
**Fig. 4** is a chart showing the results of the stability test with the compound of formula (Ia) at pH = 3.0.
**Fig. 5** is a chart showing the results of the stability test with the compound of formula (Ia) at pH = 4.5.
**Fig. 6** is a chart showing the results of the stability test with the compound of formula (Ia) at pH = 6.5.

### Summary of the Invention

The present invention, in one aspect, relates to a compound of formula (I), 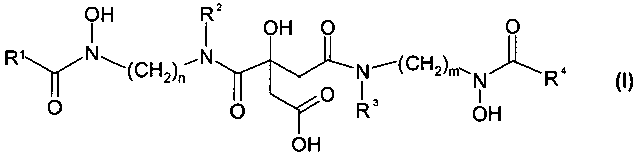 wherein
R¹ and R⁴ are identical or different and are
   C₁-C₈-alkyl, C₁-C₈-alkoxy, C₂-C₈-alkenyl, C₂-C₈-alkynyl, C₃-C₈-cycloalkyl, C₃-C₈-cycloalkenyl, wherein each of the above mentioned radicals is unsubstituted or substituted by one or more radicals selected from the group consisting of halogen or hydroxyl,
   aryl, preferably phenyl or naphthyl, wherein the aryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy, or
   heteroaryl, wherein the heteroaryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy,
R² and R³ are identical or different and are hydrogen or C₁-C₈-alkyl and,
n and m are identical or different and are an integer from 1 to 5,
or a stereoisomer or a salt or a hydrate thereof.

In one embodiment, the invention relates to a compound of formula (I), wherein
R¹ and R⁴ are identical or different and are
   C₁-C₄-alkyl, C₁-C₄-alkoxy, C₂-C₄-alkenyl, C₂-C₄-alkynyl, C₃-C₆-cycloalkyl, C₃-C₆-cycloalkenyl, wherein each of the above mentioned radicals is unsubstituted or substituted by one or more radicals selected from the group consisting of halogen or hydroxyl,
   phenyl or naphthyl, wherein the phenyl or naphthyl is unsubstituted or substituted by one or more radicals selected from the group consisting of hydroxy, cyano, nitro, halogen, halogen-C₁-C₄-alkyl, halogen-C₁-C₄-alkoxy, C₁-C₄-alkyl and C₁-C₄-alkoxy, or
   heteroaryl, wherein the heteroaryl is unsubstituted or substituted by one or more radicals selected from the group consisting of hydroxy, cyano, nitro, halogen, halogen-C₁-C₄-alkyl, halogen-C₁-C₄-alkoxy, C₁-C₄-alkyl and C₁-C₄-alkoxy,
R² and R³ are identical or different and are hydrogen or C₁-C₄-alkyl and,
n and m are identical or different and are an integer from 1 to 5.

In another embodiment, the invention relates to a compound of formula (I), wherein
R¹ and R⁴ are identical or different and are
   C₁-C₄-alkyl, C₁-C₄-alkoxy, C₃-C₆-cycloalkyl, wherein each of the above mentioned radicals is unsubstituted or substituted by one or more radicals selected from the group consisting of halogen or hydroxyl, or
   phenyl, wherein the phenyl is unsubstituted or substituted by one or more radicals selected from the group consisting of hydroxy, cyano, nitro, halogen, halogen-C₁-C₄-alkyl, halogen-C₁-C₄-alkoxy, C₁-C₄-alkyl and C₁-C₄-alkoxy,
R² and R³ are identical or different and are hydrogen or methyl and,
n and m are identical or different and are an integer from 3 to 5.

In a preferred embodiment, the invention relates to a compound of formula (I), wherein n and m are different.

In a particularly preferred embodiment n is 5 and m is 3.

In another embodiment, the invention relates to a compound of formula (I), wherein R¹ and R⁴ are identical.

In another embodiment, the invention relates to a compound of formula (I), wherein R¹ and R⁴ are C₁-C₄-alkyl, preferably methyl.

In another embodiment, the invention relates to a compound of formula (I), wherein R² and R³ are identical.

In another embodiment, the invention relates to a compound of formula (I), wherein R² and R³ are hydrogen.

In another embodiment, the invention relates to a compound of formula (I), wherein the compound of formula (I) is the compound of formula (Ia)

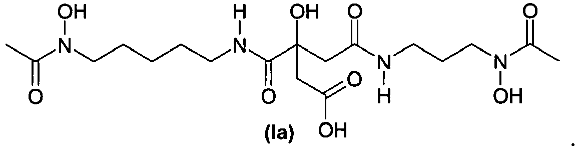

The present invention, in another aspect, relates to the use of a compound of formula (I), 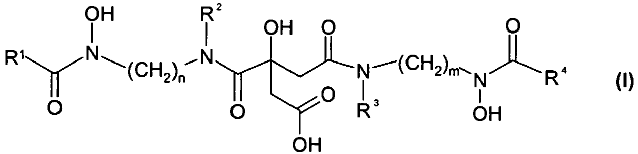 wherein
R¹ and R⁴ are identical or different and are
   C₁-C₈-alkyl, C₁-C₈-alkoxy, C₂-C₈-alkenyl, C₂-C₈-alkynyl, C₃-C₈-cycloalkyl, C₃-C₈-cycloalkenyl, wherein each of the above mentioned radicals is unsubstituted or substituted by one or more radicals selected from the group consisting of halogen or hydroxyl,
   aryl, preferably phenyl or naphthyl, wherein the aryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy, or
   heteroaryl, wherein the heteroaryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy,
R² and R³ are identical or different and are hydrogen or C₁-C₈-alkyl and, n and m are identical or different and are an integer from 1 to 5,
or a stereoisomer or a salt or a hydrate thereof, as a sweetness enhancer.

The preferred embodiments of the compound of the compound of formula (I) as mentioned above apply accordingly to the use as a sweetness enhancer according to the present invention.

In another aspect, the present invention relates to a sweetener composition comprising:
(a) at least one sweetener; and
(b) a compound of formula (I) 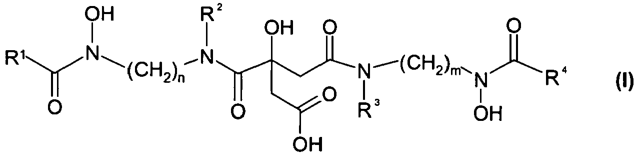 wherein
   R¹ and R⁴ are identical or different and are
      C₁-C₈-alkyl, C₁-C₈-alkoxy, C₂-C₈-alkenyl, C₂-C₈-alkynyl, C₃-C₈-cycloalkyl, C₃-C₈-cycloalkenyl, wherein each of the above mentioned radicals is unsubstituted or substituted by one or more radicals selected from the group consisting of halogen or hydroxyl,
      aryl, preferably phenyl or naphthyl, wherein the aryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy, or
      heteroaryl, wherein the heteroaryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy,
   R² and R³ are identical or different and are hydrogen or C₁-C₈-alkyl and,
   n and m are identical or different and are an integer from 1 to 5,
or a stereoisomer or a salt or a hydrate thereof.

The preferred embodiments of the compound of the compound of formula (I) as mentioned above apply accordingly to the sweetener compositions according to the present invention.

Preferably, in the sweetener composition the compound of formula (I) as defined above or a stereoisomer or a salt thereof is a sweetness enhancer.

In one embodiment, the sweetener composition comprises a at least one sweetener which is selected from the group consisting of abiziasaponin, abrusosides, in particular abrusoside A, abrusoside B, abrusoside C, abrusoside D, acesulfame potassium, advantame, albiziasaponin, alitame, aspartame, superaspartame, bayunosides, in particular bayunoside 1, bayunoside 2, brazzein, bryoside, bryonoside, bryonodulcoside, carnosifloside, carrelame, curculin, cyanin, chlorogenic acid, cyclamates and its salts, cyclocaryoside I, dihydroquercetin-3-acetate, dihydroflavenol, dulcoside, gaudichaudioside, glycyrrhizin, glycyrrhetin acid, gypenoside, hematoxylin, isomogrosides, in particular iso-mogroside V, lugduname, magap, mabinlins, micraculin, mogrosides (lo han guo), in particular mogroside IV and mogroside V, monatin and its derivatives, monellin, mukurozioside, naringin dihydrochalcone (NarDHC), neohesperidin dihydrochalcone (NDHC), neotame, osladin, pentadin, periandrin I-V, perillartine, D-phenylalanine, phlomisosides, in particular phlomisoside 1, phlomisoside 2, phlomisoside 3, phlomisoside 4, phloridzin, phyllodulcin, polpodiosides, polypodoside A, pterocaryosides, rebaudiosides, in particular rebaudioside A, rebaudioside B, rebaudioside C, rebaudioside D, rebaudioside F, rebaudioside G, rebaudioside H), rubusosides, saccharin and its salts and derivatives, scandenoside, selligueanin A, siamenosides, in particular siamenoside I, stevia, steviolbioside, stevioside and other steviol glycosides, strogines, in particular strogin 1, strogin 2, strogin 4, suavioside A, suavioside B, suavioside G, suavioside H, suavioside I, suavioside J, sucralose, sucronate, sucrooctate, talin, telosmoside A₁₅, thaumatin, in particular thaumatin I and II, trans-anethol, trans-cinnamaldehyde, trilobatin, D-tryptophane, erythritol, galactitol, hydrogenated starch syrups including maltitol and sorbitol syrups, inositols, isomalt, lactitol, maltitol, mannitol, xylitol, arabinose, dextrin, dextrose, fructose, high fructose corn syrup, fructooligosaccharides, fructooligosaccharide syrups, galactose, galactooligosaccharides, glucose, glucose and (hydrogenated) starch syrups/hydrolysates, isomaltulose, lactose, hydrolysed lactose, maltose, mannose, rhamnose, ribose, sucrose, tagatose, trehalose and xylose.

Preferably, the at least one sweetener is acesulfame potassium, sucrose or fructose.

Preferably, the sweetener composition comprises a first sweetener and a second sweetener.

Preferably, the at least one sweetener is a natural sweetener.

In other embodiments, the at least one sweetener is an artificial sweetener.

In some embodiments, the sweetener composition comprises
(c) a pregelatinized starch.

In these embodiments (and also in other embodiments), the at least one sweetener is preferably acesulfame potassium or sucrose.

In one embodiment, the sweetener composition comprises from 80 wt% to 95 wt% of pregelatinized starch based on the total weight of the sweetener composition. The pregelatinized starch, when combined with the sweetener, provides for prolonged release of the sweetener from a consumable product and for a prolonged sweetening sensation realized by the consumer.

In one embodiment, the sweetener is absorbed or adsorbed onto the pregelatinized starch.

In one embodiment, the sweetener composition comprises homogeneous particles comprising the sweetener and the pregelatinized starch.

In one embodiment, the pregelatinized starch has a specific surface less than or equal to 0.5 m²/g.

In one embodiment, the pregelatinized starch has a specific surface ranging from 0.05 m²/g to 0.5 m²/g.

In one embodiment, the pregelatinized starch is non-granular.

In one embodiment, the pregelatinized starch is granular.

In one embodiment, the pregelatinized starch comprises particles and at least 50% of the pregelatinized starch particles have a particle size between 50 to 500 micrometers.

In one embodiment, the sweetener composition comprises a compound of formula (I) as defined above or a stereoisomer or a salt or a hydrate thereof in a purity of greater than about 60 % by weight, e.g., greater than about 70 % by weight, greater than about 80 % by weight, greater than about 90 % by weight, greater than about 98 % by weight, or greater than about 99 % by weight.

In one embodiment, a 1 gram portion of the sweetener composition provides sweetness comparable to from one to three teaspoons of granulated sugar, preferably comparable to two teaspoons of granulated sugar.

In one embodiment, 1 gram of the sweetener composition contains less calories and carbohydrates than about 5 grams of granulated sugar, e.g., less than about 3 grams, less than about 2 grams, or less than about 1 gram of granulated sugar.

In one embodiment, the sweetener composition further comprises at least one additional ingredient selected from bubble forming agents, bulking agents, carriers, fibers, sugar alcohols, flavorings, flavor enhancers, flavor stabilizers, acidulants, anti-caking and free-flow agents.

In one aspect, the present invention relates to a method of controlling the release rate of taste sensations, e.g., sweetness, associated with a sweetener from a sweetener composition comprising at least one sweetener, the compound of formula (I) or a stereoisomer or a salt or a hydrate thereof as defined above, comprising the step of admixing at least one sweetener and the compound of formula (I) or a stereoisomer or a salt or a hydrate thereof as defined above with a pregelatinized starch to form a release controlled sweetener composition.

In one aspect, the present invention relates to a method of controlling the release rate of taste sensations associated with at least one sweetener from a consumable comprising the step of combining at least one sweetener, the compound of formula (I) or a stereoisomer or a salt or a hydrate thereof as defined above, a pregelatinized starch, and a consumable product to form a released controlled consumable product.

Preferably, at least one sweetener, the compound of formula (I) or a stereoisomer or a salt or a hydrate thereof as defined above and the pregelatinized starch are combined to form a sweetener composition, which may then be combined with the consumable product.

In one aspect, the present invention relates to a method for decreasing a release rate of the at least one sweetener and a compound of formula (I) or a stereoisomer or a salt or a hydrate thereof as defined above from a consumable comprising at least one sweetener, a compound of formula (I) or a stereoisomer or a salt or a hydrate thereof as defined above and a consumable product, and having an initial release rate of the at least one sweetener and the compound of formula (I) or a stereoisomer or a salt or a hydrate thereof as defined above comprising the step of adding to the consumable a pregelatinized starch in an amount effective to decrease the release rate of at least one sweetener and/or a compound of formula (I) or a stereoisomer or a salt or a hydrate thereof as defined above from the consumable product to final release rate.

The invention, in one aspect, relates to a tabletop sweetener composition comprising
(a) at least one sugar sweetener, which is selected from the group consisting of monosaccharides, disaccharides, oligosaccharides and polysaccharides, preferably the at least one sugar sweetener is selected from the group consisting of arabinose, dextrin, dextrose, fructose, high fructose corn syrup, fructooligosaccharides, fructooligosaccharide syrups, galactose, galactooligosaccharides, glucose, glucose and (hydrogenated) starch syrups/hydrolysates, isomaltulose, lactose, hydrolysed lactose, maltose, mannose, rhamnose, ribose, sucrose, stachyose, tagatose, trehalose, xylose, and combinations thereof, most preferably the at least one sugar sweetener is a disaccharide and/or fructose;
(b) at least one sugar alcohol (or polyol), which is selected from the group consisting of erythritol, galactitol, hydrogenated starch syrups including maltitol and sorbitol syrups, inositols, isomalt, lactitol, maltitol, mannitol, xylitol, and combinations thereof, preferably the at least one sugar alcohol is erythritol;
(c) a compound of formula (I) 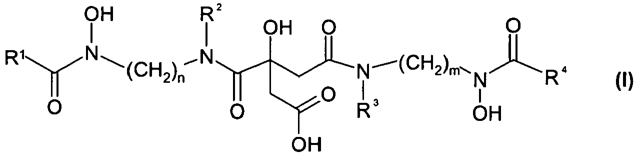 wherein
   R¹ and R⁴ are identical or different and are
      C₁-C₈-alkyl, C₁-C₈-alkoxy, C₂-C₈-alkenyl, C₂-C₈-alkynyl, C₃-C₈-cycloalkyl, C₃-C₈-cycloalkenyl, wherein each of the above mentioned radicals is unsubstituted or substituted by one or more radicals selected from the group consisting of halogen or hydroxyl,
      aryl, preferably phenyl or naphthyl, wherein the aryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy, or
      heteroaryl, wherein the heteroaryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy,
   R² and R³ are identical or different and are hydrogen or C₁-C₈-alkyl and,
   n and m are identical or different and are an integer from 1 to 5,
   or a stereoisomer or a salt or a hydrate thereof, and
(d) a taste-improving amount of cellulose.

The preferred embodiments of the compound of the compound of formula (I) as mentioned above apply accordingly to the tabletop sweetener compositions according to the present invention.

In one embodiment, the invention relates to a tabletop sweetener composition comprising
(a) a disaccharide carbohydrate and/or fructose;
(b) erythritol;
(c) a compound of formula (I) as defined above or a stereoisomer or a salt or a hydrate thereof; and
(d) a taste-improving amount of cellulose.

In preferred embodiments, in the tabletop sweetener composition the disaccharide is selected from the group consisting of isomaltulose lactose, maltose, sucrose, and trehalose.

Preferably, the tabletop sweetener composition comprises between about 40 % by weight and about 70 % by weight sugar alcohol, in particular erythritol, in particular between about 50 % by weight and about 60 % by weight sugar alcohol, in particular erythritol, in particular about 55 % by weight sugar alcohol, in particular erythritol.

Preferably, the tabletop sweetener composition comprises between about 27 % by weight and about 50 % by weight sugar sweetener, in particular disaccharide, in particular between about 35 % by weight and about 45 % by weight sugar sweetener, in particular disaccharide, in particular between about 30 % by weight and about 40 % by weight sugar sweetener, in particular disaccharide.

Preferably, the tabletop sweetener composition comprises between about 0.5 % by weight and about 7.0 % by weight of a compound of formula (I) as defined above or a stereoisomer or a salt or a hydrate thereof, in particular between about 0.7 % by weight and 5.0 % by weight of a compound of formula (I) as defined above or a stereoisomer or a salt or a hydrate thereof, in particular between about 1.0 % by weight and about 2.5 % by weight of a compound of formula (I) as defined above or a stereoisomer or a salt or a hydrate thereof.

Preferably, the tabletop sweetener composition comprises between about 0.4 % by weight and about 3.0 % by weight cellulose, in particular between about 0.7 % by weight and about 2.0 % by weight cellulose, in particular 1.0 % by weight cellulose.

In one embodiment, the tabletop sweetener composition further comprises a sweetness modifier, in particular less than about 2 % by weight of a sweetness modifier. In terms of ranges, the tabletop sweetener composition may, for example, comprise between about 0.01 % by weight and about 2 % by weight sweetness modifier, in particular between about 0.1 % by weight and about 1.5 % by weight sweetness modifier.

In other embodiments, the tabletop sweetener composition further comprises a mouthfeel enhancer, in particular less than about 1 % by weight of a mouthfeel enhancer. In terms of ranges, the tabletop sweetener composition may, for example, comprise between about 0.01 % by weight and about 1 % by weight mouthfeel enhancer, in particular between about 0.1 % by weight and about 0.5 % by weight mouthfeel enhancer.

In other embodiments, the tabletop sweetener composition further comprises a flavoring, in particular less than about 1 % by weight of a flavoring. In terms of ranges, the tabletop sweetener composition may, for example, comprise between about 0.01 % by weight and about 1 % by weight flavoring, in particular between about 0.1 % by weight and about 0.5 % by weight flavoring.

In one embodiment, the tabletop sweetener composition substantially comprises sweetener particles.

Preferably, the sweetener particles have an average particle size of between about 50 microns and about 1250 microns, in particular the sweetener particles have an average particle size of between about 100 microns and about 1000 microns.

In one embodiment, the tabletop sweetener composition has less than about 5 calories per gram, in particular the tabletop sweetener composition has less than about 3 calories per gram, in particular the sweetener composition has less than about 1 calorie per gram.

The present invention, in another aspect, further relates to a tabletop sweetener composition comprising
(a) a plurality of first sweetener particles, wherein the first sweetener particles have (i) a sugar alcohol core, in particular an erythritol core, (ii) a first sugar alcohol core-coating layer, in particular a first erythritol core-coating layer comprising a compound of formula (I) 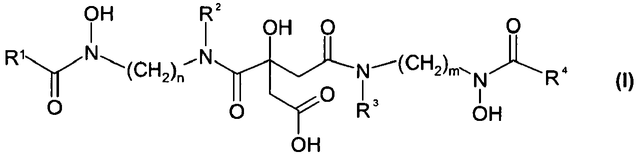 wherein
   R¹ and R⁴ are identical or different and are
      C₁-C₈-alkyl, C₁-C₈-alkoxy, C₂-C₈-alkenyl, C₂-C₈-alkynyl, C₃-C₈-cycloalkyl, C₃-C₈-cycloalkenyl, wherein each of the above mentioned radicals is unsubstituted or substituted by one or more radicals selected from the group consisting of halogen or hydroxyl,
      aryl, preferably phenyl or naphthyl, wherein the aryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy, or
      heteroaryl, wherein the heteroaryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy,
   R² and R³ are identical or different and are hydrogen or C₁-C₈-alkyl and,
   n and m are identical or different and are an integer from 1 to 5,
   or a stereoisomer or a salt or a hydrate thereof, and cellulose, and (iii) a second sugar alcohol core-coating layer, in particular a second erythritol core-coating layer comprising a sugar sweetener, in particular a disaccharide, wherein the second sugar alcohol core-coating layer, in particular the second erythritol core-coating layer lies outside of the first sugar alcohol core-coating layer, in particular outside of the first erythritol core-coating layer; and
(b) a plurality of second sweetener particles, wherein the second sweetener particle has (i) a sugar sweetener core, in particular a disaccharide core, (ii) a first sugar sweetener core-coating layer, in particular a first disaccharide core-coating layer comprising a compound of formula (I) 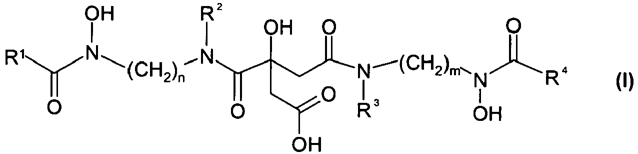 wherein
   R¹ and R⁴ are identical or different and are
      C₁-C₈-alkyl, C₁-C₈-alkoxy, C₂-C₈-alkenyl, C₂-C₈-alkynyl, C₃-C₈-cycloalkyl, C₃-C₈-cycloalkenyl, wherein each of the above mentioned radicals is unsubstituted or substituted by one or more radicals selected from the group consisting of halogen or hydroxyl,
      aryl, preferably phenyl or naphthyl, wherein the aryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy, or
      heteroaryl, wherein the heteroaryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy,
   R² and R³ are identical or different and are hydrogen or C₁-C₈-alkyl and, n and m are identical or different and are an integer from 1 to 5,
or a stereoisomer or a salt or a hydrate thereof, and cellulose, and (iii) a second sugar sweetener core-coating layer, in particular a second disaccharide core-coating layer comprising a sugar sweetener, in particular a disaccharide, wherein the second sugar sweetener core-coating layer, in particular the second disaccharide core-coating layer lies outside of the first sugar sweetener core-coating layer, in particular outside of the first disaccharide core-coating layer.

The preferred embodiments of the compound of the compound of formula (I) as mentioned above apply accordingly to the tabletop sweetener compositions according to the present invention.

In one embodiment, the tabletop sweetener composition comprises a mixture of the plurality of first sweetener particles and the plurality of second sweetener particles.

Preferably, the disaccharide core comprises isomaltulose.

In one embodiment, the second erythritol core-coating layer comprises isomaltulose.

In one embodiment, the second disaccharide core-coating layer comprises isomaltulose.

In one embodiment, at least one of the first sugar alcohol core-coating layers, in particular the first erythritol core-coating layer and the first sugar sweetener core-coating layer, in particular the first disaccharide core-coating layer, further comprise a flavoring.

In one embodiment, at least one of the first sugar alcohol core-coating layers, in particular the first erythritol core-coating layer, and the first sugar sweetener core-coating layer, in particular the first disaccharide core-coating layer, further comprise a mouthfeel enhancer.

In one embodiment, at least one of the first sugar alcohol core-coating layers, in particular the first erythritol core-coating layer, and the first sugar sweetener core-coating layer, in particular the first disaccharide core-coating layer, further comprise a sweetness modifier.

Preferably, the plurality of first sweetener particles and the plurality of second sweetener particles have an average particle size between about 50 microns and about 1250 microns, in particular, the plurality of first sweetener particles and the plurality of second sweetener particles have an average particle size between about 100 microns and about 1000 microns.

In another aspect, the present invention further relates to a consumable comprising
(a) a consumable product; and
(b) a sweetener composition of the invention as defined above.

In one aspect, the present invention further relates to a consumable comprising
(a) a consumable product; and
(b) a tabletop sweetener composition of the invention as defined above.

The preferred embodiments of the compound according to formula (I) as mentioned above apply accordingly to the consumable according to the present invention.

Preferably, the compound of formula (I) as defined above or a stereoisomer or a salt or a hydrate thereof, the sweetener composition of the invention and the tabletop sweetener composition of the invention are present in the consumable in an amount effective to increase a sweetness level of the consumable.

Preferably, in the consumable of the invention the compound of formula (I) as defined above or a stereoisomer or a salt or a hydrate thereof is present in a concentration from 0.1 wppm to 100 wppm, in particular 0.2 wppm to 50 wppm, particularly preferred from 0.5 wppm to 10 wppm.

In one embodiment, the consumable product is selected from water-based consumables, solid dry consumables, dairy products, dairy-derived products and dairy-alternative products.

Preferably, the consumable product is a water-based consumable product selected from the group consisting of beverage, water, aqueous beverage, enhanced/slightly sweetened water drink, flavored carbonated and still mineral and table water, carbonated beverage, non-carbonated beverage, carbonated water, still water, soft drink, non-alcoholic drink, alcoholic drink, beer, wine, liquor, fruit drink, juice, fruit juice, vegetable juice, broth drink, coffee, tea, black tea, green tea, oolong tea, herbal infusion, cacoa (water-based), tea-based drink, coffee-based drinks, cacao-based drink, infusion, syrup, frozen fruit, frozen fruit juice, water-based ice, fruit ice, sorbet, dressing, salad dressing, jams, marmalades, canned fruit, savoury, delicatessen products like delicatessen salads, sauces, ketchup, mustard, pickles and marinated fish, sauce, soup, and beverage botanical materials (e.g. whole or ground), or instant powder for reconstitution (e.g. coffee beans, ground coffee, instant coffee, cacao beans, cacao powder, instant cacao, tea leaves, instant tea powder).

Preferably, the consumable product is a solid dry consumable product selected from the group consisting of cereals, baked food products, biscuits, bread, breakfast cereal, cereal bar, energy bars/nutritional bars, granola, cakes, rice cakes, cookies, crackers, donuts, muffins, pastries, confectionaries, chewing gum, chocolate products, chocolate, fondant, hard candy, marshmallow, pressed tablets, snack foods, botanical materials (whole or ground), and instant powders for reconstitution.

Preferably, the consumable product is a dairy product, dairy-derived product and/or dairy-alternative product selected from the group consisting of milk, fluid milk, cultured milk product, cultured and noncultured dairy-based drink, cultured milk product cultured with lactobacillus, yoghurt, yoghurt-based beverage, smoothie, lassi, milk shake, acidified milk, acidified milk beverage, butter milk, kefir, milk-based beverages, milk/juice blend, fermented milk beverage, ice cream, dessert, sour cream, dip, salad dressing, cottage cheese, frozen yoghurt, soy milk, rice milk, soy drink, and rice milk drink.

In one embodiment, the consumable product is a carbonated drink.

In one embodiment, the consumable product is a non-carbonated drink.

In one embodiment, the consumable product is a cereal.

In one embodiment, the consumable product is a yoghurt.

In one embodiment, the consumable product is a chewing-gum.

Preferably, the consumable product is a dental product selected from the group consisting of toothpaste, dental floss, mouthwash, denture adhesive, enamel whitener, fluoride treatments and oral care gels, toothpaste being particularly preferred.

Preferably, the consumable product is a cosmetic product selected from the group consisting of lipstick, lip balm, lip gloss and petroleum jelly.

Preferably, the consumable product is a pharmaceutical product selected from the group consisting of over-the-counter and prescription drugs, non-tobacco snuff, tobacco substitutes, chewable medications, cough syrups, throat sprays, throat lozenges, cough drops, antibacterial products, pill coatings, gel caplets, soluble fiber preparations, antacids, tablet cores, rapidly absorbed liquid compositions, stable foam compositions, rapidly disintegrating pharmaceutical dosage forms, beverage concentrates for medicinal purposes, aqueous pharmaceutical suspensions, liquid concentrate compositions, and stabilized sorbic acid solutions, phosphate buffers, saline solutions, emulsion, non-aqueous pharmaceutical solvents, aqueous pharmaceutical carriers, solid pharmaceutical carrier, and pharmaceutical preservatives/additives (antimicrobials, antioxidants, chelating agents, inert gases, flavoring agents, coloring agents).

In one embodiment, the consumable product is an animal feed or animal food.

In one embodiment, the consumable product is a chewing gum. As one example, the sweetener composition may comprise pregelatinized starch and the sweetener may comprise acesulfame potassium.

In one embodiment, the consumable product is a chewing gum. As one example, the sweetener composition may comprise pregelatinized starch and the sweetener may comprise sucrose.

Preferably, in embodiments wherein the consumable product is a chewing gum, the consumable product further comprises menthol.

The invention, in another aspect, further relates to a method of providing a consumable of the invention as defined above by admixing a compound of formula (I) as defined above or a stereoisomer or a salt or a hydrate thereof, a sweetener composition of the invention as defined above or a tabletop sweetener composition of the invention as defined above to a consumable product.

The invention, in another aspect, further relates to a method of enhancing the taste sensations associated with flavor ingredients by admixing a compound of formula (I) as defined above or a stereoisomer or a salt or a hydrate thereof, a sweetener composition of the invention as defined above or a tabletop sweetener composition as defined above with one or more flavor ingredients to provide a flavor-enhanced composition or consumable.

The invention, in another aspect, further relates to a method of increasing a sweetness level of a consumable having an initial sweetness level comprising the step of adding to the consumable a compound of formula (I) as defined above or a stereoisomer or a salt or a hydrate thereof in an amount effective to increase the sweetness level of the consumable to a final sweetness level.

The invention, in another aspect, relates to a process for the preparation of a compound of formula (I) 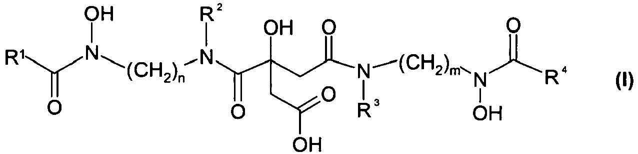 or a stereoisomer or a salt or a hydrate thereof, wherein R¹, R², R³, R⁴, n and m are defined as above comprising at least the following steps:
(a) reaction of a compound of the formula (II), 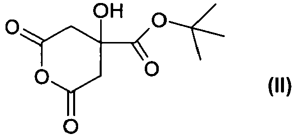 with a compound of the general formula (III), 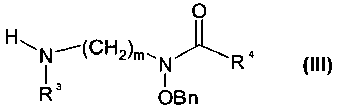 wherein R³, R⁴ and m are defined as above and Bn is benzyl,
   if appropriate in the presence of a solvent to obtain a compound of the general formula (IV), 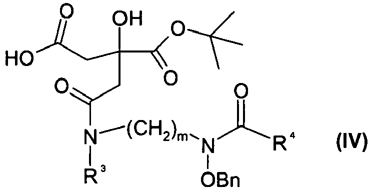 wherein R³, R⁴, m and Bn are defined as above;
(b) reaction of a compound of the general formula (IV) with a protecting agent, preferably with dibenzyl carbonate, if appropriate in the presence of a solvent and a base, preferably DMAP, to obtain a compound of the general formula (V), 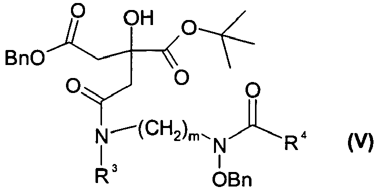 wherein R³, R⁴, m and Bn are defined as above;
(c) reaction of a compound of the general formula (V) with an acid if appropriate in the presence of a solvent to obtain a compound of the general formula (VI), 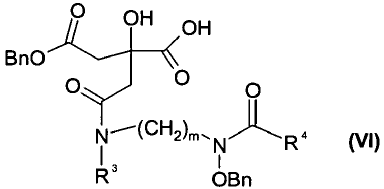 wherein R³, R⁴, m and Bn are defined as above;
(d) reaction of a compound of the general formula (VI) with a compound of the general formula (VII), 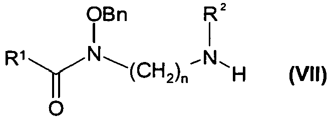 wherein R¹, R², n and Bn are as defined above,
   in the presence of a coupling agent, preferably in the presence of T3P®, and if appropriate in the presence of a solvent to yield a compound of the general formula (VIII), 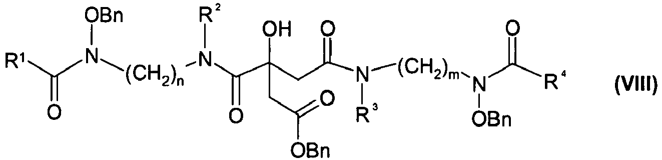 wherein R¹, R², R³, R⁴, n, m and Bn are as defined above; and
(e) reaction of a compound of the general formula (VIII) with a deprotecting agent if appropriate in the presence of a solvent in order to obtain a compound of the general formula (I) as defined above.

The preferred embodiments of the compound of the compound of formula (I) as mentioned above apply accordingly to the process of preparation of a compound of formula (I) according to the present invention.

In one aspect, the invention relates to a compound of the formulae (IV), (V), (VI), (VII) and (VIII) as defined above.

In another aspect, the invention relates to a method of obtaining the compound of formula (I) as defined above, comprising isolating the compound of formula (I) as defined above from a natural organism, in particular isolating the compound of formula (Ia) as defined above from an actinomycetes strain.

### Detailed Description of the Invention

### Novel Sweetness Enhancers

As indicated above, there is a need for alternative sweetness enhancers which are healthy, i.e. non-caloric, non-cariogenic and ideal for diabetics as they would also allow reducing levels of conventional caloric sweeteners and therefore calorie reduction at the same sweetness level. Also, the need exists for sweetness enhancers having an excellent temperature and pH stability, excellent storage and solubility properties as well as a taste-enhancing effects and synergies when combined with other sweetening compounds or food-related ingredients, e.g., pregelatinized starch. In particular, novel sweetness enhancers that are derived from natural products are of great interest. In addition, the need exists for sweetness enhancers that are free from off tastes, e.g. bitter or metallic tastes.

It is therefore an object of the present invention to provide an alternative sweetness enhancer having the above mentioned desired characteristics.

In one aspect, the present invention provides the novel compounds of the general formula (I) 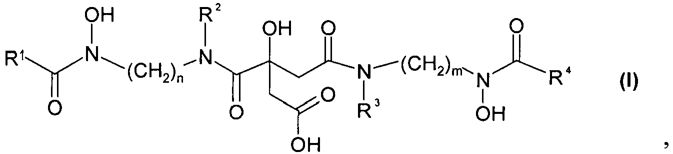 wherein
R¹ and R⁴ are identical or different and are
   C₁-C₈-alkyl, C₁-C₈-alkoxy, C₂-C₈-alkenyl, C₂-C₈-alkynyl, C₃-C₈-cycloalkyl, C₃-C₈-cycloalkenyl, wherein each of the above mentioned radicals is unsubstituted or substituted by one or more radicals selected from the group consisting of halogen or hydroxyl,
   aryl, preferably phenyl or naphthyl, wherein the aryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy, or
   heteroaryl, wherein the heteroaryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy,
R² and R³ are identical or different and are hydrogen or C₁-C₈-alkyl and,
n and m are identical or different and are an integer from 1 to 5,
or a stereoisomer or a salt or a hydrate thereof.

As used herein, including the accompanying claims, the substituents have the following meanings.

As used herein, the term "halogen" means a fluorine, chlorine, bromine or iodine atom, preferably a fluorine or chlorine atom.

As used herein, the term "C₁-C₈-alkyl" alone or in combination means a straight-chain or branched alkyl group with 1 to 8 carbon atoms, preferably a straight or branched-chain alkyl group with 1 to 6 carbon atoms and particularly preferred a straight or branched-chain alkyl group with 1 to 4 carbon atoms. Examples of straight-chain and branched C₁-C₈-alkyl groups are methyl, ethyl, propyl, isopropyl, butyl, isobutyl, tert.-butyl, the isomeric pentyls, the isomeric hexyls, the isomeric heptyls, the isomeric octyls, preferably methyl and ethyl and most preferred methyl.

As used herein, the term "C₁-C₈-alkoxy" means the group R'O-, wherein R' is C₁-C₈-alkyl and has the meanings defined above. Examples of C₁-C₈-alkoxy groups are methoxy, ethoxy, n-propoxy, isopropoxy, n-butoxy, isobutoxy, sec.-butoxy and tert.-butoxy, preferably methoxy and ethoxy.

As used herein, the term "C₂-C₈-alkenyl" alone or in combination means a straight-chain or branched-chain hydrocarbon residue comprising an olefinic bond and 1 to 8, preferably 1 to 6, particularly preferred 1 to 4 carbon atoms. Examples of alkenyl groups are ethenyl, 1-propenyl, 2-propenyl, isopropenyl, 1-butenyl, 2-butenyl, 3-butenyl and isobutenyl. A preferred example is 2-propenyl.

As used herein, the term "C₂-C₈-alkynyl" alone or in combination means a straight-chain or branched chain hydrocarbon residue comprising an alkyne bond and 1 to 8, preferably 1 to 6, particularly preferred 1 to 4 carbon atoms. Examples of alkyne groups are propargyl, 1-methyl-2-propynyl, 2-butynyl or 3-butynyl.

As used herein, the term "C₃-C₈-cycloalkyl" means a carbocyclic saturated ring system having 3 to 8 carbon atoms, preferably 1 to 6, particularly preferred 1 to 4 carbon atoms, for example cyclopropyl, cyclobutyl, cyclopentyl, cyclohexyl, cycloheptyl or cyclooctyl, preferably cyclopentyl and cyclohexyl.

As used herein, the term "aryl" means a mono-, bi- or polycyclic aromatic system, for example phenyl, naphthyl, tetrahydronaphthyl, indenyl, indanyl, pentalenyl, fluorenyl and the like, preferably phenyl or naphthyl, particularly preferred phenyl.

As used herein, the term "heteroaryl" means an aromatic or partly unsaturated 5- or 6-membered ring which comprises one, two or three atoms selected from nitrogen, oxygen and/or sulphur, such as pyridyl, pyrazinyl, pyrimidinyl, pyridazinyl, 2-oxo-1,2-dihydropyridinyl, oxazolyl, oxydiazolyl, isoxazolyl, thiadiazolyl, tetrazolyl, pyrazolyl, imidazolyl, thiazolyl and thienyl. The term "heteroaryl" further refers to bicyclic aromatic or partly unsaturated groups comprising two 5- or 6- membered rings, in which one or both rings can contain one, two or three atoms selected from nitrogen, oxygen or sulphur, such as quinolinyl, isoquinolinyl, cinnolinyl, pyrazolo[1,5-a]pyridyl, imidazo[1,2-a]pyridyl, quinoxalinyl, benzothiazolyl, benzotriazolyl, indolyl, indazolyl. Preferred heteroaryl groups are pyridyl or pyrazinyl.

As used herein, the term "stereoisomer(s)" as it relates to a compound of formula (I) encompasses any possible enantiomers, diastereomers, cis-trans-isomers and/or E-/Z-isomers of a compound of formula (I) and its salts or hydrates. In particular, the term "stereoisomer" means a single compound or a mixture of two or more compounds, wherein at least one chiral center is predominantly present in one definite isomeric form, in particular the S-enantiomer, the R-enantiomer and the racemate of a compound of formula (I). It is also possible that two or more stereogenic centers are predominantly present in one definite isomeric form of a derivative of a compound of formula (I) as defined above. In the sense of the present invention, "predominantly" has the meaning of at least 60%, preferably at least 70%, particularly preferably at least 80%, most preferably at least 90%. According to the present invention, also stereoisomers of a compound of formula (I) may be present as a salt or a hydrate.

As used herein, the term "salt(s)" as it relates to a compound of formula (I) as defined above means the physiologically acceptable acid addition salts and base salts of the compound of formula (I) or its derivatives or its stereoisomers. Suitable acid addition salts are formed from acids which form non-toxic salts. Examples include but are not limited to the acetate, aspartate, benzoate, besylate, bicarbonate, carbonate, bisulphate, sulphate, borate, camsylate, citrate, edisylate, esylate, formate, fumarate, gluceptate, gluconate, glucuronate, hexafluorophosphate, hibenzate, hydrochloride/chloride, hydrobromide, bromide, hydroiodide, iodide, isethionate, lactate, malate, maleate, malonate, mesylate, methylsulphate, naphthylate, nicotinate, nitrate, orotate, oxalate, palmitate, pamoate, phosphate, hydrogen phosphate, dihydrogen phosphate, sacharate, stearate, succinate, tartrate, tosylate and trifluoroacetate salts. Suitable base salts are formed from bases which form non-toxic salts. Examples include but are not limited to the aluminium, arginine, benzathine, calcium, choline, diethylamine, diolamine, glycine, lysine, magnesium, meglumine, olamine, potassium, sodium, tromethamine and zinc salts.

As used herein, the term "hydrate(s)" as it relates to a compound of formula (I) means a compound of formula (I) or a stereoisomer or a salt thereof that includes water. "Hydrate(s)" are formed by the addition of water or its elements. In one embodiment, a compound of formula (I) as defined above or a stereoisomer or a salt thereof may form crystals that incorporate water into the crystalline structure without chemical alteration.

The terms stereoisomer, salt, and hydrate may also be used in conjunction with one another. For example, a stereoisomer of a compound of formula (I) may have a salt and/or a derivative. Combinations of these terms are considered to be within the scope of the invention.

The compounds of formula (I) of the invention as defined above, including stereoisomers and salts and hydrates thereof may also be designated as "the compound(s) of the invention."

In one embodiment, the invention relates to a compound of formula (I), wherein
R¹ and R⁴ are identical or different and are
   C₁-C₄-alkyl, C₁-C₄-alkoxy, C₂-C₄-alkenyl, C₂-C₄-alkynyl, C₃-C₆-cycloalkyl, C₃-C₆-cycloalkenyl, wherein each of the above mentioned radicals is unsubstituted or substituted by one or more radicals selected from the group consisting of halogen or hydroxyl,
   phenyl or naphthyl, wherein the phenyl or naphthyl is unsubstituted or substituted by one or more radicals selected from the group consisting of hydroxy, cyano, nitro, halogen, halogen-C₁-C₄-alkyl, halogen-C₁-C₄-alkoxy, C₁-C₄-alkyl and C₁-C₄-alkoxy, or
   heteroaryl, wherein the heteroaryl is unsubstituted or substituted by one or more radicals selected from the group consisting of hydroxy, cyano, nitro, halogen, halogen-C₁-C₄-alkyl, halogen-C₁-C₄-alkoxy, C₁-C₄-alkyl and C₁-C₄-alkoxy,
R² and R³ are identical or different and are hydrogen or C₁-C₄-alkyl and,
n and m are identical or different and are an integer from 1 to 5.

In another embodiment, the invention relates to a compound of formula (I), wherein
R¹ and R⁴ are identical or different and are
   C₁-C₄-alkyl, C₁-C₄-alkoxy, C₃-C₆-cycloalkyl, wherein each of the above mentioned radicals is unsubstituted or substituted by one or more radicals selected from the group consisting of halogen or hydroxyl, or
   phenyl, wherein the phenyl is unsubstituted or substituted by one or more radicals selected from the group consisting of hydroxy, cyano, nitro, halogen, halogen-C₁-C₄-alkyl, halogen-C₁-C₄-alkoxy, C₁-C₄-alkyl and C₁-C₄-alkoxy,
R² and R³ are identical or different and are hydrogen or methyl and,
n and m are identical or different and are an integer from 3 to 5.

In a preferred embodiment, the invention relates to a compound of formula (I), wherein n and m are different.

In a particularly preferred embodiment, n is preferably 5 and m is preferably 3.

In another embodiment, the invention relates to a compound of formula (I), wherein R¹ and R⁴ are identical.

In another embodiment, the invention relates to a compound of formula (I), wherein R¹ and R⁴ are C₁-C₄-alkyl.

In another embodiment, the invention relates to a compound of formula (I), wherein R² and R³ are identical.

In another embodiment, the invention relates to a compound of formula (I), wherein R² and R³ are hydrogen.

In a particularly preferred embodiment, the compound of formula (I) is the compound of formula (Ia)

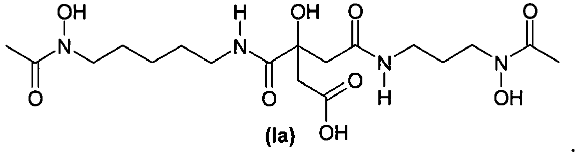

It has now been found that the compounds of formula (I) 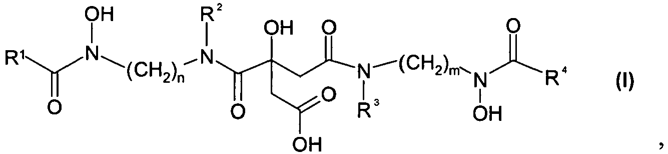 wherein
R¹ and R⁴ are identical or different and are
   C₁-C₈-alkyl, C₁-C₈-alkoxy, C₂-C₈-alkenyl, C₂-C₈-alkynyl, C₃-C₈-cycloalkyl, C₃-C₈-cycloalkenyl, wherein each of the above mentioned radicals is unsubstituted or substituted by one or more radicals selected from the group consisting of halogen or hydroxyl,
   aryl, preferably phenyl or naphthyl, wherein the aryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy, or
   heteroaryl, wherein the heteroaryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy,
R² and R³ are identical or different and are hydrogen or C₁-C₈-alkyl and, n and m are identical or different and are an integer from 1 to 5,
or a stereoisomer or a salt or a hydrate thereof are useful as sweetness enhancers.

Thus, in another aspect, the invention relates to the use of a compound of formula (I) as defined above or a stereoisomer of a salt or a hydrate thereof as a sweetness enhancer.

As used herein, the term "enhance" means to have an effect on a particular flavor sensation in consumables or other products placed in the oral cavity which is found more pronounced (stronger, enhanced) in its taste intensity and/or which is found to have an earlier onset of the flavor sensation.

As used herein, the term "sweetness enhancer(s)" means any compound, which is capable of enhancing or intensifying the perception of sweet taste of sweetener compositions or sweetened compositions, e.g. a compound of formula (I) or a stereoisomer or a salt or a hydrate thereof. The phrase "sweetness enhancer" is synonymous to the terms "sweet taste potentiator", "sweetness potentiator", and "sweetness intensifier".

As used herein, the phrase "high intensity sweetener (s)" means any sweetener, which may in raw, extracted, purified, or any other from, singularly or in combination thereof have a sweetness potency greater than sucrose (common table sugar) yet have comparatively less calories.

As used herein, the term "sweetener(s)" includes all artificial and natural sweeteners, sugar alcohols (or polyols) and sugar sweeteners (or carbohydrates). Artificial and natural sweeteners include but are not limited to abiziasaponin, abrusosides, in particular abrusoside A, abrusoside B, abrusoside C, abrusoside D, acesulfame potassium, advantame, albiziasaponin, alitame, aspartame, superaspartame, bayunosides, in particular bayunoside 1, bayunoside 2, brazzein, bryoside, bryonoside, bryonodulcoside, carnosifloside, carrelame, curculin, cyanin, chlorogenic acid, cyclamates and its salts, cyclocaryoside I, dihydroquercetin-3-acetate, dihydroflavenol, dulcoside, gaudichaudioside, glycyrrhizin, glycyrrhetin acid, gypenoside, hematoxylin, isomogrosides, in particular iso-mogroside V, lugduname, magap, mabinlins, micraculin, mogrosides (lo han guo), in particular mogroside IV and mogroside V, monatin and its derivatives, monellin, mukurozioside, naringin dihydrochalcone (NarDHC), neohesperidin dihydrochalcone (NDHC), neotame, osladin, pentadin, periandrin I-V, perillartine, D-phenylalanine, phlomisosides, in particular phlomisoside 1, phlomisoside 2, phlomisoside 3, phlomisoside 4, phloridzin, phyllodulcin, polpodiosides, polypodoside A, pterocaryosides, rebaudiosides, in particular rebaudioside A, rebaudioside B, rebaudioside C, rebaudioside D, rebaudioside F, rebaudioside G, rebaudioside H), rubusosides, saccharin and its salts and derivatives, scandenoside, selligueanin A, siamenosides, in particular siamenoside I, stevia, steviolbioside, stevioside and other steviol glycosides, strogines, in particular strogin 1, strogin 2, strogin 4, suavioside A, suavioside B, suavioside G, suavioside H, suavioside I, suavioside J, sucralose, sucronate, sucrooctate, talin, telosmoside A₁₅, thaumatin, in particular thaumatin I and II, trans-anethol, trans-cinnamaldehyde, trilobatin, D-tryptophane, and combinations thereof. Sugar alcohols (or polyols) include but are not limited to erythritol, galactitol, hydrogenated starch syrups including maltitol and sorbitol syrups, inositols, isomalt, lactitol, maltitol, mannitol, xylitol, and combinations thereof. Sugar sweeteners (or carbohydrates) include monosaccharides, disaccharides, oligosaccharides and polysaccharides such as but not limited to arabinose, dextrin, dextrose, fructose, high fructose corn syrup, fructooligosaccharides, fructooligosaccharide syrups, galactose, galactooligosaccharides, glucose, glucose and (hydrogenated) starch syrups/hydrolysates, isomaltulose, lactose, hydrolysed lactose, maltose, mannose, rhamnose, ribose, sucrose, stachyose, tagatose, trehalose, xylose, and combinations thereof. The sweeteners are known substances and are for example described by H. Mitchell (H. Mitchell, "Sweeteners and Sugar Alternatives in Food Technology", Backwell Publishing Ltd, 2006) and in WO 2009/023975 A2.

Suitable hydrogenated starch hydrolysates include, but are not limited to, those disclosed in U.S. patent no. 4,279,931 and various hydrogenated glucose syrups and/or powders which contain sorbitol, maltitol, hydrogenated disaccharides, hydrogenated higher polysaccharides, or combination thereof. Hydrogenated starch hydrosylates are primarily prepared by the controlled catalytic hydrogenation of con syrups. The resulting hydrogenated starch hydrosylates are mixtures of monomeric, dimeric, and polymeric saccharides.

As shown in the Examples, the inventors have now surprisingly and unexpectedly found that the compounds of formula (I) or a stereoisomer or a salt or a hydrate thereof are useful as a sweetness enhancer and have no characteristic intrinsic taste at the indicated concentration. The compounds of formula (I) or a stereoisomer or a salt or a hydrate thereof do not have the bitter aftertaste associated with saccharin, (for example, the bitter aftertaste associated with saccharin), or a metallic, acidic, astringent or throat-burning aftertaste (for example, those aftertastes often found in high-intensity sweeteners) at the indicated concentration. In addition, the compounds of formula (I) or a stereoisomer or a salt or a hydrate thereof do not exhibit a liquorice aftertaste.

Further, as shown in the Examples, it has been found that the compounds of formula (I) or a stereoisomer or a salt or a hydrate thereof have an excellent temperature and pH stability as well as very good solubility properties.

This excellent taste profile and the excellent temperature and pH stability as well as the very good solubility properties make the compounds of formula (I) or a stereoisomer or a salt or a hydrate thereof desirable for use in consumables or other products placed in the oral cavity.

### Process for the Preparation of a Compound of Formula (I)

In another aspect, the present invention relates to a process for the preparation of a compound of formula (I) 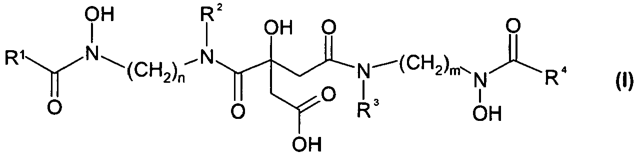 or a stereoisomer or a salt or a hydrate thereof, wherein R¹, R², R³, R⁴, n and m are defined as above comprising at least the following steps:
(a) reaction of a compound of the formula (II), 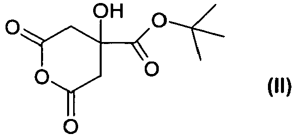 with a compound of the general formula (III), 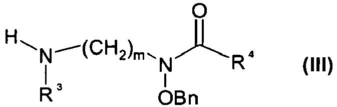 wherein R³, R⁴ and m are defined as above and Bn is benzyl,
   if appropriate in the presence of a solvent to obtain a compound of the general formula (IV), 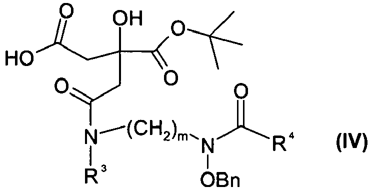 wherein R³, R⁴, m and Bn are defined as above;
(b) reaction of a compound of the general formula (IV) with a protecting agent, preferably with dibenzyl carbonate, if appropriate in the presence of a solvent and a base, preferably DMAP, to obtain a compound of the general formula (V), 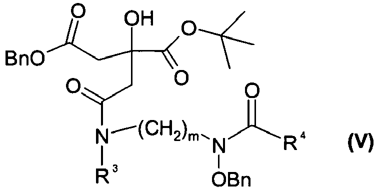 wherein R³, R⁴, m and Bn are defined as above;
(c) reaction of a compound of the general formula (V) with an acid if appropriate in the presence of a solvent to obtain a compound of the general formula (VI), 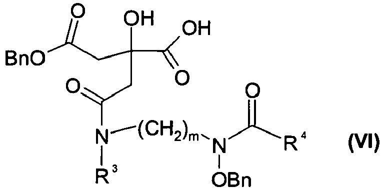 wherein R³, R⁴, m and Bn are defined as above;
(d) reaction of a compound of the general formula (VI) with a compound of the general formula (VII), 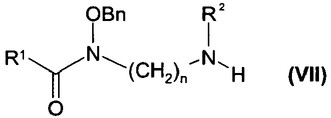 wherein R¹, R², n and Bn are as defined above,
   in the presence of a coupling agent, preferably in the presence of T3P®, and if appropriate in the presence of a solvent to yield a compound of the general formula (VIII), 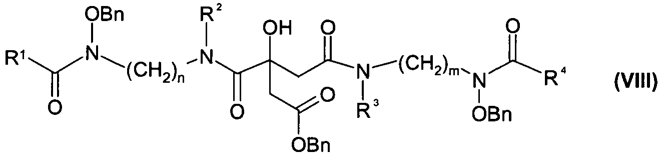 wherein R¹, R², R³, R⁴, n, m and Bn are as defined above; and
(e) reaction of a compound of the general formula (VIII) with a deprotecting agent if appropriate in the presence of a solvent in order to obtain a compound of the general formula (I) as defined above.

The preparation of the compound of the formula (II) used as a starting material in process step (a) is described in J. Prakt. Chem. 1987, 329, 447. Alternatively, the compound of the formula (II) may be obtained in a two-step synthesis starting from citric acid. Process step (a) is carried out preferably in the present of a solvent, preferably in the presence of methylene chloride.

Process step (b) may be carried out as described in Synthesis 1994, 1063.

Process step (c) is preferably carried out in the presence of gaseous hydrogen chloride in the present of a solvent, preferably in the presence of ethyl acetate, at an appropriate temperature, preferably at about 0° C.

Process step (d) is carried out under standard coupling conditions.

Process step (e) is carried out under standard reductive deprotection conditions preferably in the presence of Pd/C in the presence of a solvent, preferably in ethanol.

The compounds of the general formula (III) can be prepared from commercially available amines. The preparation of the compound of the general formula (III), wherein R³ is hydrogen, R⁴ is methyl and m is 3, is described in J. Org. Chem. 1983, 48, 24. Other compounds of the general formula (III) can be easily obtained in analogy to this synthesis.

The compounds of the general formula (VII) (cf. cpd. 6 of the scheme below), wherein R¹ is methyl, R² is hydrogen and n is 5 can be obtained as shown in the scheme below.

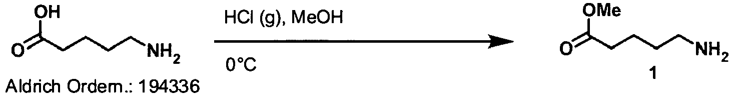

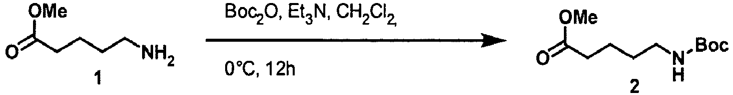

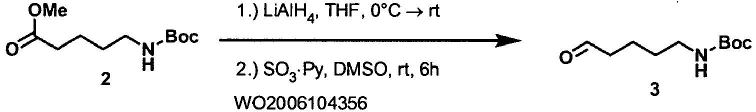

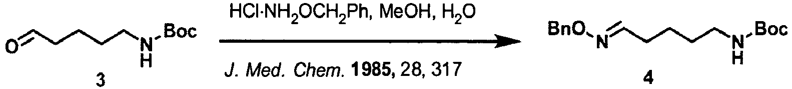

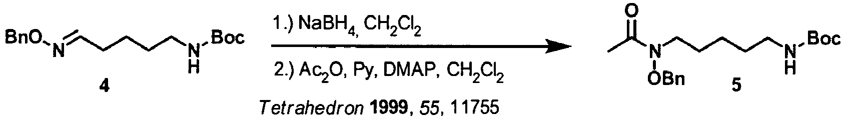

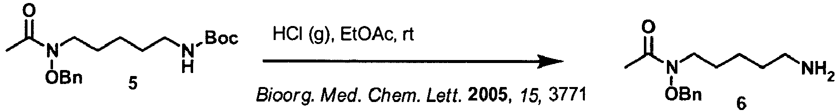

In a particular preferred embodiment of the process of the invention, R¹ and R⁴ are methyl, R² and R³ are hydrogen, n is 5 and m is 3.

In one aspect, the invention relates to a compound of the formula (IV) as defined above.

In one aspect, the invention relates to a compound of the formula (V) as defined above.

In one aspect, the invention relates to a compound of the formula (VI) as defined above.

In one aspect, the invention relates to a compound of the formula (VII) as defined above.

In one aspect, the invention relates to a compound of the formula (VIII) as defined above.

### Method of obtaining the compound of formula (I) by isolation from a natural organism

In another aspect, the invention relates to a method of obtaining a compound of formula (I) as defined above comprising isolating the compound of formula (I) as defined above from a natural organism, in particular isolating the compound of formula (Ia) as defined above from an actinomycetes strain.

As shown in the Examples, the compound of formula (Ia) can be isolated from the actinomycetes strain with the identification reference 01496axxx000004 and the accession number DSM 25420, which has been deposited in accordance with the terms as those laid down in the Budapest Treaty on the International Recognition of the Deposit of Microorganisms for the Purposes of Patent Procedure of April 28, 1977 on November 30, 2011, at the DSMZ - Deutsche Sammlung von Mikroorganismen und Zellkulturen GmbH, Inhoffenstr. 7 B, 38124 Braunschweig, Germany, by the AnalytiCon Discovery GmbH, Hermannswerder Haus 17, 14473 Potsdam, Germany.

In one embodiment, the method comprises the following steps:
a) preparing a fermentation broth of an actinomycetes strain, preferably of the actinomycetes strain with the accession number DSM 25420;
b) extracting the compound of formula (Ia) from the so obtained fermentation broth,
c) isolating the compound of formula (Ia) of the invention, and
d) if appropriate purifying the so obtained fractions in order to obtain the compound of formula (Ia).

### Sweetener Compositions and Properties Thereof

In another aspect, the invention relates to a sweetener composition comprising
(a) at least one sweetener; and
(b) a compound of formula (I) 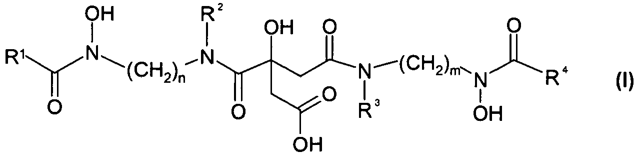
wherein
R¹ and R⁴ are identical or different and are
   C₁-C₈-alkyl, C₁-C₈-alkoxy, C₂-C₈-alkenyl, C₂-C₈-alkynyl, C₃-C₈-cycloalkyl, C₃-C₈-cycloalkenyl, wherein each of the above mentioned radicals is unsubstituted or substituted by one or more radicals selected from the group consisting of halogen or hydroxyl,
   aryl, preferably phenyl or naphthyl, wherein the aryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy, or
   heteroaryl, wherein the heteroaryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy,
R² and R³ are identical or different and are hydrogen or C₁-C₈-alkyl and,
n and m are identical or different and are an integer from 1 to 5,
or a stereoisomer or a salt or a hydrate thereof.

The preferred embodiments of the compound of the compound of formula (I) as mentioned above apply accordingly to the sweetener compositions according to the present invention.

Preferably, the compounds of formula (I) as defined above or a stereoisomer or a salt or a hydrate thereof are a sweetness enhancer.

In one embodiment, the sweetener composition comprises at least one sweetener which is selected from the group consisting of abiziasaponin, abrusosides, in particular abrusoside A, abrusoside B, abrusoside C, abrusoside D, acesulfame potassium, advantame, albiziasaponin, alitame, aspartame, superaspartame, bayunosides, in particular bayunoside 1, bayunoside 2, brazzein, bryoside, bryonoside, bryonodulcoside, carnosifloside, carrelame, curculin, cyanin, chlorogenic acid, cyclamates and its salts, cyclocaryoside I, dihydroquercetin-3-acetate, dihydroflavenol, dulcoside, gaudichaudioside, glycyrrhizin, glycyrrhetin acid, gypenoside, hematoxylin, isomogrosides, in particular iso-mogroside V, lugduname, magap, mabinlins, micraculin, mogrosides (lo han guo), in particular mogroside IV and mogroside V, monatin and its derivatives, monellin, mukurozioside, naringin dihydrochalcone (NarDHC), neohesperidin dihydrochalcone (NDHC), neotame, osladin, pentadin, periandrin I-V, perillartine, D-phenylalanine, phlomisosides, in particular phlomisoside 1, phlomisoside 2, phlomisoside 3, phlomisoside 4, phloridzin, phyllodulcin, polpodiosides, polypodoside A, pterocaryosides, rebaudiosides, in particular rebaudioside A, rebaudioside B, rebaudioside C, rebaudioside D, rebaudioside F, rebaudioside G, rebaudioside H), rubusosides, saccharin and its salts and derivatives, scandenoside, selligueanin A, siamenosides, in particular siamenoside I, stevia, steviolbioside, stevioside and other steviol glycosides, strogines, in particular strogin 1, strogin 2, strogin 4, suavioside A, suavioside B, suavioside G, suavioside H, suavioside I, suavioside J, sucralose, sucronate, sucrooctate, talin, telosmoside A₁₅, thaumatin, in particular thaumatin I and II, trans-anethol, trans-cinnamaldehyde, trilobatin, D-tryptophane, erythritol, galactitol, hydrogenated starch syrups including maltitol and sorbitol syrups, inositols, isomalt, lactitol, maltitol, mannitol, xylitol, arabinose, dextrin, dextrose, fructose, high fructose corn syrup, fructooligosaccharides, fructooligosaccharide syrups, galactose, galactooligosaccharides, glucose, glucose and (hydrogenated) starch syrups/hydrolysates, isomaltulose, lactose, hydrolysed lactose, maltose, mannose, rhamnose, ribose, sucrose, tagatose, trehalose and xylose.

Preferably, the at least one sweetener is acesulfame potassium, sucrose or fructose.

In another embodiment, the sweetener composition comprises a first sweetener and a second sweetener.

Preferably, the first sweetener is fructose.

Preferably, the at least one sweetener is a natural sweetener.

In another embodiment, the at least one sweetener is an artificial sweetener.

In another embodiment, the sweetener composition comprises a compound of formula (I) as defined above or a stereoisomer or a salt or a hydrate thereof in a purity of greater than about 60 % by weight, e.g., greater than about 70 % by weight, greater than about 80 % by weight, greater than about 90 % by weight, greater than about 98 % by weight, or greater than about 99 % by weight.

Preferably, a 1 gram portion of the sweetener composition provides sweetness comparable to one to three teaspoons of granulated sugar, preferably comparable to two teaspoons of granulated sugar.

For example, the compositions may contain sweetness comparable to that of granulated sugar (sucrose), and therefore can be used "spoon-for-spoon" or "cup-for-cup" in place of sugar.

As used herein, the phrase "sweetness comparable" means that an experienced sensory evaluator, on average, will determine that the sweetness presented in a first composition is within a range of 80% to 120% of the sweetness presented in a second composition. The phrase "a sweetness comparable" relates to a determination ascertained by four or more experienced sensor evaluators in a sweetness matching test (designated hereinafter as "taste and spit assay"), as discussed below. Thus, for instance, 100 mg/ml of a sweetener composition comprising the compound of formula (I) provides "sweetness comparable" to 100 mg/ml of sucrose if the sweetener composition of the invention has a sweetness falling within the range of sweetness presented in 80-120 mg/ml of sucrose.

The sweetness and/or sweetness enhancing properties of a compound, in some embodiments, can be identified by *an in vitro* in cell based assay as described in the Examples, in EP 1 865 316 B1 or by field effector transistor technology of e.g. Alpha MOS.

The taste of a sample of a compound, e.g. of the compound of formula (I), with regard to sweetness and/or sweetness enhancing properties, in other embodiments, may be assessed *in vivo* by using a panel of trained sensory evaluators experienced in the sweet taste estimation procedure, e.g. in the taste and spit assay as described e.g. in Example 2.

In these cases, panelists are asked to take a sample of the liquid to be assessed (test substance, e.g. a compound of formula (I) as defined above) into the mouth and after some time allowed for taste perception to spit the sample out completely. Subsequently, the panelists are asked to rinse their mouth well with water or black tea to reduce any potential carry over effects. The tasting of a sample can be repeated if required.

In a first descriptive test (qualitative assessment for sweetness) the panelists are asked to taste the quality of single samples (maximum 3 subsequent samples). The individuals of the taste panel are asked to answer the following questions with regard to the quality of taste: 1) does the sample taste sweet?, 2) is there another taste detectable (bitter, sour, salty, umami)?, 3) is there an off- or aftertaste?, 4) is there anything else remarkable about the perception of the sample?

In the next step (assessment of sweetness enhancing, e.g. fructose enhancing, features) the panelists are asked to answer questions in a pairwise comparison test to determine the enhancement of sweet taste of the test substance with fructose relative to fructose only. Again the panelists are given samples. Two samples are prepared for direct comparison regarding sweetness. One sample contains fructose in a solvent and the other sample additionally contains the test substance. Designation of the samples with A and B is randomized and is decoded after the taste procedure. The questions to be answered are: 1) does one sample taste sweeter than the other?, 2) if so, which one?, 3) are there any other differences in the taste between the two samples? The result of the taste and spit assay is a qualitative evaluation of the differences between the two samples.

In another embodiment, the sweetness and/or sweetness enhancing properties of the inventive sweetener composition, when dissolved in water, correspond to a particular degrees Brix, a well-known measurement of sugar content in an aqueous solution. In some embodiments, for example, when 5 grams of sweetener composition are dissolved in 95 grams of water, the resultant solution has a sweetness that corresponds to a degrees Brix value ranging from 1 to 1000, e.g., from 5 to 500 or from 5 to 100.

Preferably, one gram of the sweetener composition contains less calories and carbohydrates than about 1 gram of granulated sugar, e.g., less than about 0.5 grams of granulated sugar.

In another embodiment of the invention, the sweetener composition of the invention is substantially free of off-taste.

In one embodiment of the invention, the sweetener composition of the invention is liquid at ambient conditions.

In another embodiment of the invention, the sweetener composition of the invention is solid at ambient conditions.

In one embodiment of the invention, the sweetener composition of the invention comprises homogeneous particles comprising the sweetener and a compound of formula (I) as defined above or a stereoisomer or a salt or a hydrate thereof. Preferably, the homogeneous sweetener particles have an average particle size of between about 50 microns and about 1250 microns, e.g., between about 100 microns and about 1000 microns.

In another embodiment of the invention, the sweetener composition comprises a mixture of first particles comprising the sweetener and second particles comprising a compound of formula (I) as defined above or a stereoisomer or a salt or a hydrate thereof.

Preferably, the sweetener composition of the invention comprises from 0.0005 to 1.0 wt% of the sweetness enhancer compound of formula (I) as defined above or a stereoisomer or a salt or a hydrate thereof, based on the total weight of the sweetener composition, preferably from 0.0001 to 0.1 wt%, particularly preferred from 0.001 to 0.05 wt%.

In one embodiment, the sweetener compositions further comprise at least one additional sweetness enhancer, e.g., at least two or at least three. Suitable additional sweetness enhancers are well known in the art. In one embodiment, the at least one additional sweetness enhancer may be selected from the group consisting of terpenes (such as sesquiterpenes, diterpenes, and triterpenes), flavonoids, amino acids, proteins, polyols, other known natural sweeteners (such as cinnamaldehydes, selligueains and hematoxylins), secodammarane glycosides, and analogues thereof

Exemplary sweetness enhancers include stevioside, steviolbioside, rebaudioside A, rebaudioside B, rebaudioside C, rebaudioside D, rebaudioside F, dulcoside A, rubusoside; hernandulcin; pine rosin diperpenoid; mukurozioside; baiyunosdie; phlomisoside, such as phlomisoside I and phlomisodie II; glycyrrhizic acid; periandrins, such as periandrin I, periandrin II, periandrin III, and periandrin IV; osladin; polypodosides, such as polypodoside A and polypodoside B; mogrosides, such as mogroside IV and mogroside V; abrusoside A and abrusosdie B; cyclocariosdies, such as cyclocarioside A and cyclocarioside B; pterocaryoside A and pterocaryoside B; flavonoids, such as phyllodulcin, phloridzin, neoastilbin, and dihydroquercetin acetate; amino acids, such as glycine and monatin; proteins, such as thaumatins (thaumatin I, thaumatin II, thaumatin iii, and thaumatin IV), monellin, mabinlins (mabinlin I and mabinlin II), brazzein, miraculin, and curculin; polyols such as erythritol; cinnamaldehyde; selligueains, such as selligueain A and selligueain B; hematoxylin; and mixtures thereof.

Additional exemplary sweetness enhancers include pine rosin diterpenoids; phloridizin; neoastilbin; dihydroquercetin acetate; glycine; erythritol; cinnamaldehyde; selligueain A; selligueain B; hematoxylin; rebaudioside A; rebaudioside B; rebaudioside C; rebaudioside D; rebaudioside E; dulcoside A; steviolbioside; rubusoside; stevia; stevioside; steviol 13 O-β-D-glycoside; mogroside V; Luo Han Guo; siamenoside; siamenoside I; monatin and salts thereof (monatin SS, RR, RS, SR); curculin; glycyrrhizic acid and its salts; thaumatin I; thaumatin II; thaumatin III; thaumatin IV; monellin; mabinlin I; mabinlin II; brazzein; hernandulcin; phyllodulcin; glycyphyllin; phloridzin; trilobatin; baiyunoside; osladin; polypodoside A; polypodoside B; pterocaryoside A; pterocaryoside B; mukurozioside; mukurozioside lib; phlomisoside I; phlomisoside II; periandrin I; periandrin II; periandrin III; periandrin VI; periandrin V; cyclocarioside A; cyclocarioside B; suavioside A; suavioside B; suavioside G; suavioside H; suavioside I; suavioside J; labdane glycosides; baiyunoside; gaudichaudioside A; mogroside IV; iso-mogroside; bryodulcoside; bryobioside; bryoside; bryonoside; carnosifloside V; carnosifloside VI; scandenoside R6; 1 1 -oxomogroside V; abrusoside A; abrusoside B; abrusoside C; abrusoside D; abrusoside E; gypenoside XX; glycyrrhizin; apioglycyrrhizin; araboglycyrrhizin; pentadin; perillaldehyde; rebaudioside F; steviol; 13-[(2-O-(3-O-a-D-glucopyranosyl)-β-D-glucopyranosyl-3-O-β-D-glucopyranosyl-β-D-glucopyranosyl)oxy]kaur-16-en-18-oic acid β-D-glucopyranosyl ester; 13-[(2-O-β-D-glucopyranosyl-3-O-(4-O-α-D-glucopyranosyl)-β-D-glucopyranosyl-β-D-glucopyranosyl)oxy] kaur-16-en-18- oic acid β-D-glucopyranosyl ester; 13-[(3-O-β-D-glucopyranosyl-β-D-glucopyranosyl)oxy]kaur-16-en-18-oic acid β-D-glucopyranosyl ester; 13- hydroxy-kaur-16-en-18-oic acid β-D-glucopyranosyl ester; 13-methyl-16-oxo- 17-norkauran-18-oic acid β-D-glucopyranosyl ester; 13-[(2-0-β-D- glucopyranosyl-3-O-β-D-giucopyranosyl-β-D-glucopyranosyl)oxy] kaur-15-en-18-oic acid β-D-glucopyranosyl ester; 13-[(2-O-β-D-glucopyranosyl-3-O-β-D-glucopyranosyl-β-D-glucopyranosyl)oxy] kaur-15-en-18-oic acid; 13-[(2-0-β- D-glucopyranosyl-3-O-β-D-glucopyranosyl]-β-D-glucopyranosyl)oxy]-17- hydroxy-kaur-15-en-18-oic acid β-D-glucopyranosyl ester; 13-[(2-O-(3-D- glucopyranosyl-3-O-β-D-glucopyranosyl-β-D-glucopyranosyl)oxy]-16-hydroxy kauran-18-oic acid β-D-glucopyranosyl ester; 13-[(2-O-β-D-glucopyranosyl-3-O-β-D-glucopyranosyl-β-D-glucopyranosyl)oxy]-16-hydroxy kauran-18-oic acid; isosteviol; mogroside IA; mogroside IE; mogroside 11-A; mogroside 11-E; mogroside III; mogroside V; isomogroside V; 1 1-Oxomogroside; mogrol; 1 1- oxomogrol; 1 1 -oxomogroside IA; 1-[13-hydroxykaur-16-en-18-oate] β-D- glucopyranuronic acid; 13-[(2-O-β-D-glucopyranosyl β-D-glucopyranosyl)oxy]- 17-hydroxy-kaur-15-en-18-oic acid β-D-glucopyranosyl ester; 13-[(2-0-β-D- glucopyranosyl-β-D-glucopyranosyl)oxy] kaur-16-en-18-oic acid-(2-O-β -D-glucopyranosyl-β-D-glucopyranosyl)ester (rebaudioside E); 13-[(2-O-α-L-rhamnopyranosyl-3-O-β-D-glucopyranosyl-β-D-glucopyranosyl)oxy] kaur-16- en-18-oic acid-(2-0-β-D-glucopyranosyl-β-D-glucopyranosyl) ester; 13-[(2-O- β-D-glucopyranosyl-3-O-β-D-glucopyranosyl-β-D-glucopyranosyl)oxy]- kaur-16-en-18-oic acid-(2-O-α-L-rhamnopyranosyl-β-D-glucopyranosyl) ester; 13-[(2-O-β-D-glucopyranosyl β-D-glucopyranosyl)oxy]-17-oxo-kaur-15-en-oic acid β-D-glucopyranosl ester; 13-[(2-O-(6-O-β-D-glucopyranosyl)-β-D-glucopyranosyl-β-D-glucopyranosyl)oxy] kaur-16-en-18-oic acid β-D-glucopyranosyl ester; 13-[(2-O-β-D-glucopyranosyl-3-O-β-D-fructofuranosyl-β-D-glucopyranosyl)oxy] kaur- 16-en-18-oic acid β-D-glucopyranosyl ester; 13-[(2O-β-D-glucopyranosyl-β-D-glucopyranosyl)oxy] kaur-16-en-18-oic acid-(6-O-β-D-xylopyranosyl-β-D- glucopyranosyl) ester; 13-[(2-O-β-D-glucopyranosyl-β-D-glucopyranosyl)oxy] kaur-16-en-18-oic acid-(4-O-(2-O-α-D-glucopyranosyl)-α-D-glucopyranosyl- D-glucopyranosyl) ester; 13-[(2-O-β-D-glucopyranosyl-3-O-β-D-glucopyranosyl-β-D-glucopyranosyl)oxy] kaur-16-en-18-oic acid-(2-O-6- deoxy-β-D-glucopyranosyl-β-D-glucopyranosyl) ester; 13-[(2-0-β-D- glucopyranosyl-β-D-glucopyranosyl)oxy] kaur-15-en-18-oic acid β-D- glucopyranosyl ester; 13-[(2-O-β-D-glucopyranosyl-3-O-β-D-xylopyranosyl-β-D-glucopyranosyl)oxy] kaur-16-en-18-oic acid β-D-glucopyranosyl ester; 13- [(2-O-β-D-xylopyranosyl-β-D-glucopyranosyl)oxy] kaur-16-en-18-oic acid β-D- glucopyranosyl ester; 13-[(3-O-β-D-glucopyranosyl-β-D-glucopyranosyl)oxy] kaur-16-en-18-oic acid β-D-glucopyranosyl ester;13-[(2-O-6-deoxy-β-D- glucopyranosyl-3-O-β-D-glucopyranosyl-β-D-glucopyranosyl)oxy] kaur-16-en- 18-oic acid β-D-glucopyranosyl ester; 13-[(2-O-6-deoxy β-D-glucopyranosyl- β-D-glucopyranosyl)oxy] kaur-16-en-18-oic acid β-D-glucopyranosyl ester; and mixtures thereof.

Additional exemplary sweetness enhancers include rebaudioside C, rebaudioside F, rebaudioside D, 13- [(2-O-β-D-glucopyranosyl-3-O-β-D-glucopyranosyl]-β-D-glucopyranosyl)oxy]- 17-hydroxy-kaur-15-en-18-oic acid β-D-glucopyranosyl ester, 13-[(2-O-(3-O-β-D-glucopyranosyl)-β-D-glucopyranosyl-3-O-β-D-glucopyranosyl-[3-D-glucopyranosyl)oxy] kaur-16-en-18-oic acid β-D-glucopyranosyl ester, and Rubusoside. Further for example, the at least one sweetness enhancer is chosen from rebaudioside A, stevioside, rebaudioside D, rebaudioside E, mogroside V, mogroside IV, brazzein, and monatin.

In one embodiment, the at least one sweetness enhancer is present in an amount at or below the sweetness detection threshold level of the at least one sweetness enhancer. In some embodiments, the at least one sweetness enhancer is present in an amount below the sweetness detection threshold level of the at least one sweetness enhancer. The sweetness detection threshold level can be specific for a particular compound. However, generally, in some embodiments, the at least one sweetness enhancer is present in an amount ranging from 0.5 ppm to 1000 ppm. For example, the at least one sweetness enhancer may be present in an amount ranging from 1 ppm to 300 ppm; and at least one sweetness enhancer may be present in an amount ranging from 0.1 ppm to 75 ppm; and at least one sweetness enhancer may be present in an amount ranging from 500 ppm to 3000 ppm.

As used herein, the terms "sweetness threshold," "sweetness recognition threshold," and "sweetness detection threshold" are understood to mean the level at which the lowest known concentration of a certain sweet compound that is perceivable by the human sense of taste and it can vary from person to person. For example, a typical sweetness threshold level for sucrose in water can be 0.5%. Further for example, the at least one sweetness enhancer to be used can be assayed in water at least 25% lower and at least 25% higher than the sucrose detection level of 0.5% in water to determine the sweetness threshold level. A person of skill in the art will be able to select the concentration of the at least one sweetness enhancer so that it may impart an enhanced sweetness to a composition comprising at least one sweetener. For example, a skilled artisan may select a concentration for the at least one sweetness enhancer so that the at least one sweetness enhancer does not impart any perceptible sweetness to a composition that does not comprise at least one sweetener.

In some embodiments, the compounds listed above as sweeteners may also function as sweetness enhancers. Generally speaking, some sweeteners may also function as sweetness enhancers and *vice versa.*

In one embodiment, the sweetener composition as defined above comprises
(c) a pregelatinized starch.

It has now been found that the inventive combination of a sweetener, a compound of formula (I) or a stereoisomer or a salt or a hydrate thereof, and pregelatinized starch provides for a sweetener composition that, when utilized in combination with a consumable product, demonstrates a prolonged release rate of the sweetener and the compound of formula (I) or a stereoisomer or a salt or a hydrate thereof from the consumable product.

Preferably, the sweetener is acesulfame potassium. Acesulfame potassium is a commercially available high intensity sweetener. One suitable commercial acesulfame potassium product is Sunett® from Nutrinova Nutrition Specialties & Food Ingredients.

In one preferred embodiment, the at least one additional sweetener comprises sucrose, which is commercially available.

Without being bound by theory, it is believed that a sweetener and a compound of formula (I) or a stereoisomer or a salt or a hydrate thereof possess characteristics that provide for prolonged release rates when a sweetener and a compound of formula (I) or a stereoisomer or a salt or a hydrate thereof are employed in combination with the pregelatinized starch. As one particular example, acesulfame potassium has a particular solubility that, unexpectedly, allows the acesulfame potassium to effectively load onto the pregelatinized starch. In one embodiment, paraffin may be employed to introduce other types of sweeteners/flavors onto the pregelatinized starch. Paraffin, surprisingly, may provide for improvements in loading and, subsequently, prolonged release rates.

Pregelatinized starches are known commodities. As one example, WO 89/04842 discloses amylase treated granular starches that provide a microporous matrix material adapted for absorption and releasable containment of functional compositions. The microporous starch granules are chemically derivatized to enhance absorption and structural properties. Absorbed functional substances are released from the microporous starch matrix under the influence of mechanical compression, by diffusion into a surrounding fluid or as a result of degradation of the granular starch matrix. Also, WO 2009/103514 discloses a liquid loaded starch material comprising a solid carrier material consisting of pregelatinized, non-granular starch material, which consists of flake-shaped starch particles, wherein the size distribution of the starch particles is such that at least 50% by weight of the starch particles have a particle size of between 100 and 375 µm, and wherein the BET specific surface area is less than or equal to 0.5 m²/g and one or more liquid components. This reference also provides for the use of same in food and animal feed products, pharmaceuticals, nutraceuticals, agrochemicals, and cosmetic or personal care products. The reference also provides a process for preparing said powdered liquid-loaded starch material. In addition, US 5,919,486 discloses a "liquid oil and fat ingredient or others" that are carried by pores of a porous carrier composed of porous starch grain obtained by reacting an enzyme having raw starch digestive activity "onto the starch." These references, however, do not disclose the combination of pregelatinized starches with sweeteners, e.g., non-caloric, high intensity sweeteners, such as acesulfame potassium.

In one embodiment, the sweetener composition demonstrates a prolonged release rate from the consumable. In one embodiment, the consumable comprises the sweetener composition and the sweetener composition provides for an initial sweetness level. Sweetness levels, e.g., "sweetnesses," may be determined by tasting panels, as discussed above. Over time, as the consumable product continues to be consumed, the initial sweetness level decreases to a reduced sweetness level. In preferred embodiments, the initial sweetness level is essentially maintained over time. In one embodiment, the inventive sweetener composition releases from the consumable product over a prolonged time period. Preferably, the inventive sweetener releases from the consumer product over a time period that is at least 5% longer than the time period for a conventional sweetener composition (employed in similar amounts and in a similar consumable product) that does not comprise pregelatinized starch, e.g., at least 10% longer, at least 20% longer, at least 30% longer, or at least 50% longer.

In one embodiment, a level of mouthfeel of the sweetener composition is prolonged by the addition the pregelatinized starch. In one embodiment, when employed in a chewing gum, the inventive sweetener composition will provide a bulkier and/or a heavier final chewing gum.

In preferred embodiments, the sweetener composition comprises from 80 wt% to 95 wt% pregelatinized starch based on the total weight of the sweetener composition, e.g., from 82 wt% to 93 wt% or from 85 wt% to 90 wt%. In terms of limits, the sweetener composition may comprise at least 80 wt% pregelatinized starch, e.g., at least 82 wt% or at least 85 wt%. In terms of upper limits, the sweetener composition may comprise less than 95 wt% pregelatinized starch, e.g., less than 93 wt% or less than 90 wt%.

In one embodiment, a sweetener and a compound of formula (I) or a stereoisomer or a salt or a hydrate thereof are absorbed into the pregelatinized starch. In another embodiment, a sweetener and a compound of formula (I) or a stereoisomer or a salt or a hydrate thereof are adsorbed onto the pregelatinized starch. The pregelatinized starch comprises a plurality of pores and capillaries. In a preferred embodiment, the sweetener is disposed in these pores and/or capillaries. Exemplary disposition methods are discussed below.

In another aspect, the present invention relates to a method of controlling the release rate of taste sensations associated with a sweetener and a compound of formula (I) or a stereoisomer or a salt or a hydrate thereof. The taste sensations may be for example the sweetness provided by a sweetener and a compound of formula (I) or a stereoisomer or a salt or a hydrate thereof. In these embodiments, a sweetener and a compound of formula (I) or a stereoisomer or a salt or a hydrate thereof are a component of a sweetener composition. The method comprises the step of contacting, e.g., admixing, a sweetener and a compound of formula (I) or a stereoisomer or a salt or a hydrate thereof, with a pregelatinized starch to form a release controlled sweetener composition. The release controlled sweetener composition has a prolonged release rate, as compared to a similar sweetener composition that does not include the pregelatinized starch.

### Pregelatinized Starch

The pregelatinized starch may vary widely and many pregelatinized starches are known in the art. Examples include the pregelatinized starches disclosed in US 5,919,486, WO 89/04842, WO 2007/110645, and WO 2009/103514. The shape of the pregelatinized starch particles may vary widely. For example, the pregelatinized starch may be granular. In some cases, small particles sizes and/or irregular particle shapes may correlate with a high specific surface area, which in turn correlates with a high loading capacity. Thus, in some embodiments, small particles with a high specific surface area may be used for the purpose of supporting a sweetener and a compound of formula (I) or a stereoisomer or a salt or a hydrate thereof.

In another embodiment, the starch may be non-granular. Preferably, the starch may be in the form of flakes. In one of these embodiments, the flake-shaped starch particles have a particle size ranging from 50 µm to 500 µm, e.g., from 100 µm to 375 µm, as determined by sieve analysis. In one embodiment, the flake-shaped starch particles are relatively large particles, and the BET specific surface area is as low as 0.5 m²/g or less, e.g., 0.4 m²/g or less, less than 0.3 m²/g. In terms of ranges the flake-shaped particles may have a specific surface area ranging from 0.05 m²/g to 0.5 m²/g, e.g., from 0.05 m²/g to 0.4 m²/g. In one embodiment, the inventive sweetener compositions comprise both granular pregelatinized starch and flake pregelatinized starch.

In one embodiment, the pregelatinized starch, whether it is in granular or flake form, may have a high porosity. In one embodiment, the pregelatinized starch, whether it is in granular or flake form, has a loading capacity greater than 10%, e.g., greater than 20%. In terms of upper limits, the pregelatinized starch, whether it is in granular or flake form, has a loading capacity less than 90%, e.g., less than 60%. The loading capacity may range from 10% to 90%, e.g., from 10% to 60%.

Without being bound by theory, the pregelatinized starches utilized in the present invention possess features and properties that are distinct from typical starches and/or bulking agents. Because conventional bulking agents lack the features and properties of the pregelatinized starches, these conventional bulking agents, when combined with sweeteners, would not be expected to provide the surprising and unexpected release rates demonstrated by the sweetener compositions of the present invention.

The term "pregelatinized starch", as used herein, relates to a starch that has been chemically and/or mechanically and/or thermally treated in the presence of water to decrease the number and size of crystalline regions and increase the randomness in the general structure, and has been subsequently dried. Typically, the structural changes induced by gelatinization are manifested in the loss of birefringence and/or Maltese crosses in polarized light. The pregelatinized starches may or may not have lost their granular structure and are substantially soluble in cold water without cooking. In accordance with the present invention, "pregelatinized starches" may also be chemically modified to impart desirable properties, such as flowability, hydrophobicity and the like. Preferably, the pregelatinized starch used in the present invention is not chemically modified. Furthermore, the term "pregelatinized starch" may also include partially pregelatinized starch (PPS), which contains soluble (gelatinized) and insoluble fractions. Preferably, the pregelatinized starch used in the present invention is completely or predominantly pregelatinized, i.e. with less than 10%, preferably less than 5%, in particular less than 2% or 1% by weight, of crystalline regions.

In accordance with the present invention, the term "chemically modified starches" or "chemical modification" of starches includes, but is not limited to, crosslinked starches, starches modified with blocking groups to inhibit retrogradation, starches modified by the addition of lipophilic groups, acetylated starches, hydroxyethylated and hydroxypropylated starches, inorganically esterified starches, cationic, anionic and oxidized starches, zwitterionic starches, starches modified by enzymes, and combinations thereof.

Suitable pregelatinized starches for use herein can be derived from any native source, wherein native relates to the fact that said starch is found in nature. Typical sources for the starches are cereals, tubers, roots, legumes, fruit starches and hybrid starches. Suitable sources include, but are not limited to, corn, pea, potato, sweet potato, sorghum, banana, barley, wheat, rice, sago, amaranth, tapioca, arrowroot, canna, and low amylose (containing no more than about 10% by weight amylose, preferably no more than 5%) or high amylose (containing at least about 40% by weight amylose) varieties thereof. Also suitable are starches derived from a genetically modified starch crop. A preferred starch for use herein has an amylose content below 40%, including waxy corn starch with less than 1% amylose content. Particularly preferred starches include rice, wheat, tapioca, corn, and potato starches, in particular corn (maize) starch. A "granular shape" is intended to mean a roughly spheroid or ellipsoid shape and includes spherical particles that have indentations in one or more portions thereof, such as the spherical starch particles produced by a conventional spray-drying process. A "non-granular starch material", as used herein, refers to a starch material consisting of particles that do not have a granular shape. A "flake- shaped" or a "flaked" starch particle, when used herein, is a particle that does not have a granular structure and has a heterogeneous shape in the form of irregular flat or thick plates or sheets. Typically, roll-drying or drum-drying processes generate such flake-shaped starch particles. Other processes, however, may be employed to provide the flake-shaped particles.

In preferred embodiments, at least 80 wt%, e.g., at least 90 wt%, at least 95 wt%, or 100 wt% by weight of the starch particles have a particle size of between 50 µm and 500 µm, e.g., from 125 µm and 350 µm, between 125 µm and 325 µm, or between 125 µm and 300 µm.

A particularly preferred pregelatinized, non-granular starch material has a particle size of 100 µm to 375 µm for at least 50% by weight, preferably 80% by weight, of the starch particles, and a BET specific surface area of less than or equal to 0.5 m²/g, preferably less than or equal to 0.4 m²/g.

In one embodiment, the pregelatinized starch has a calorie content similar to that of sucrose.

A preferred commercial pregelatinized starch is Starrier R™ and similar products from Cargill.

In one embodiment, the pregelatinized, starch, whether in granular or flake form, may include minor amounts of one or more additives, preferably in a total amount of no more than 10% by weight, more preferably no more than 5% by weight, most preferably 0% to 1% by weight, based on the total weight of the pregelatinized starch. These optionally present additives may be added to the starch slurry or paste used for preparing the pregelatinized starch material of the present invention. Examples of additive include, but are not limited to, processing aids, such as agents for enhancing the formation of bubbles, surfactants and emulsifiers, and other ingredient, such as salts, sugars, fat, gums and hydrocolloids. In some embodiments, the additives included in the pregelatinized starch material may also be substances that have been added to the formed pregelatinized starch material to provide it with desirable properties. An example thereof is a surface modifying agent, which changes the absorption properties of the starch to improve, for example, the absorption of hydrophobic ingredients like oils and fats.

Preferably, the pregelatinized starch material is produced by a roll-drying or drum-drying process. Roll-drying as well as drum-drying involve the heating of an aqueous starch slurry or paste to gelatinize the starch and to instantaneously remove the moisture. The aqueous starch slurry or paste may be first heated and subsequently dried or, more preferably, the starch may be simultaneously gelatinized by heating and dried using a commercially available drum-dryer or roll-dryer apparatus. As used herein, the term "roll-drying" refers to a process where an aqueous starch slurry or paste is cooked or partially cooked and passed on heated rolls (sometimes also referred to as "drums") for drying or, preferably, a process where the aqueous starch slurry or paste is simultaneously cooked and dried on heated rolls. The term "drum-drying", when used herein, refers to a process very similar to the roll-drying process, except that a thicker coating of the starch slurry or paste is applied to heated drums.

In one embodiment, a process for preparing the pregelatinized starch material described hereinabove starts with mixing starch (generally in the form of a starch powder) and water to prepare an aqueous starch slurry or paste having a certain solids content. A starch "slurry or paste" may also include high-viscosity starch preparations, such as a moist filter cake. Suitable starches are as defined above. The starch content typically ranges from 20 wt% to 45 wt% by weight, e.g., from 25 wt% to 40 wt%% by weight, or from 32 wt% to 40 wt%.

The prepared aqueous starch slurry or cake may then be applied onto heated, rotating rolls or drums of a roll-dryer or drum-dryer, conveniently by means of application drums or feed rolls, to simultaneously gelatinize and dry the aqueous starch slurry or paste. After one rotation, the obtained dried starch film is removed from the rolls or drums by a scrapping mechanism, such as a knife blade, to obtain a starch material, which is then subjected to grinding or milling, for example in a rotor beater mill or cutting mill. Finally, the ground (milled) starch material is sieved using one or several sieves of different mesh sizes, as known in the art, to obtain the desired sieve fraction of the pregelatinized, non-granular starch material.

Suitable roll-dryers and drum-driers for preparing the pregelatinized, non-granular starch material of the present invention are commercially available, for example from GMF-Gouda (The Netherlands). Typically, they are designed as indirect dryers, where heat is transferred by pressurized stream to the inside (metal) drum wall, which in turn transfers the heat to the aqueous starch slurry or paste on the other side of the wall. While the basic construction is relatively simple, there are numerous configurations commercially available, which differ in the arrangement and number of drums and feed rolls, the type of scrapping mechanism, etc. Factors, such as the composition of the aqueous slurry or paste, the roll or drum temperature, and the drum or roll speed (which determines the residence time), will have an effect on the physical and chemical properties of the final pregelatinized, non-granular starch material. It is within the scope of the invention to or adjust process parameters to obtain a pregelatinized starch material having desirable properties. For example, different types of starches are known to have varying gelatinization temperatures and thus one or more of the above parameters may be adjusted and optimized to achieve a satisfactory result. Such optimizations are well within the normal capabilities of a person skilled in the art of drum-dried or roll-dried pregelatinized starches.

The rolls or drums are typically heated to have a surface temperature in the range from 120 °C to 200 °C, e.g., from 140 °C to 190 °C, or from 150 °C to 180 °C. The rolls or drums are normally operated at a speed or rotation rate of 5 to 18 rpm, e.g., 5 to 15 rpm, or 8 to 13 rpm.

One or more additional constituents (additives) may be admixed to the aqueous starch slurry or paste including, but not limited to, processing aids, such as bubble-forming agents, surfactants and emulsifiers, and other substances, such as salts, sugars, fat, gums, and hydrocolloids to improve certain properties. For example, the starch slurry or paste applied to the heated rolls or drums gets transformed into a continuous phase of melted starch that includes variable amounts of air bubbles. In order to obtain a pregelatinized starch material with an increased absorption capacity, conditions might be chosen to result in a relatively low bulk density, for example, by adding specific processing aids to the aqueous starch slurry or paste to increase formation of bubbles.

Furthermore, it is also within the scope of the present invention, that the obtained roll-dried or drum-dried, pregelatinized, non- granular starch material is additionally treated with a surface modifying agent to change the absorption properties of the starch. A hydrophobic agent, for example, will further improve the absorption capacity for hydrophobic liquid components, like oils and fats.

Exemplary methods to assess the physical characteristics of the pregelatinized starch provided in the examples follow.

### (1) Particle size distribution

The particle size distribution of starch samples was determined by a sieve analysis using sieves with different openings. The respective sieve fractions on the sieves were weighted and divided by the total weight of the starch sample to give a percentage retained on each sieve.

### (2) Particle shape

The particle shape of starch samples was observed by scanning electronic microscopy at magnifications of 100 to 750 x, as known in the art.

### (3) BET specific surface area

The specific surface area of starch samples was measured by nitrogen absorption in a Gemini II 2370 Surface Area Analyzer (Micromeritrics NV/SA, Brussels, Belgium). The multi-point (11 points by convention) BET-method (Bruauner, Emmett and Teller, J. Am. Chem. Soc. 60:309-319 (1938)) was used to determine the total available surface area.

### Methods of Making a Sweetener Composition of the Invention and Enhancing the Sweetness of a Sweetener Composition

In another aspect, the present invention relates to a method of making a sweetener composition comprising the step of admixing a sweetener with a compound of formula (I) 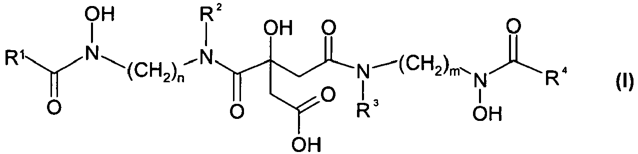 wherein
R¹ and R⁴ are identical or different and are
   C₁-C₈-alkyl, C₁-C₈-alkoxy, C₂-C₈-alkenyl, C₂-C₈-alkynyl, C₃-C₈-cycloalkyl, C₃-C₈-cycloalkenyl, wherein each of the above mentioned radicals is unsubstituted or substituted by one or more radicals selected from the group consisting of halogen or hydroxyl,
   aryl, preferably phenyl or naphthyl, wherein the aryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy, or
   heteroaryl, wherein the heteroaryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy,
R² and R³ are identical or different and are hydrogen or C₁-C₈-alkyl and,
n and m are identical or different and are an integer from 1 to 5,
or a stereoisomer or a salt or a hydrate thereof.

The preferred embodiments of the compound of the compound of formula (I) as mentioned above apply accordingly to the method of making a sweetener composition according to the present invention.

In one embodiment, the method yields a sweetener composition comprising a compound of formula (I) as defined above in the form of an extract or in isolated or purified form.

In one embodiment, the sweetener composition comprises a compound of formula (I) as defined above or a stereoisomer or a salt or a hydrate thereof in a purity of greater than about 60 % by weight, e.g., greater than about 70 % by weight, greater than about 80 % by weight, greater than about 90 % by weight, greater than about 98 % by weight, or greater than about 99 % by weight.

In one embodiment, the inventive method further comprises the step of combining a compound of formula (I) as defined above or a stereoisomer or a salt or a hydrate thereof with at least one other additional ingredient chosen from bubble forming agents, bulking agents, carriers, fibers, sugar alcohols, oligosaccharides, sugars, high intensity sweeteners, nutritive sweeteners, flavoring, flavor enhancers, flavor stabilizers, acidulants, anti-caking, free-flow agents, and any combination thereof. Preferably the resultant composition comprises about 3 to about 200 mg of a compound of formula (I) as defined above or a stereoisomer or a salt or a hydrate thereof per 1 gram of the composition, e.g., about 3 to about 100 mg of a compound of formula (I) as defined above or a stereoisomer or a salt or a hydrate thereof per 1 gram of the composition, or about 5 to about 10 mg of a compound of formula (I) as defined above or a stereoisomer or a salt or a hydrate thereof per 1 gram of the composition.

In one embodiment, the invention relates to a method for enhancing the sweetness of a sweetener composition comprising a sweetener, comprising the step of adding to the sweetener a compound of formula (I) as defined above or a stereoisomer or a salt or a hydrate thereof to form an enhanced sweetener composition. Preferably, a compound of formula (I) as defined above is added in amount effective to increase the sweetness of the sweetener composition to an increased sweetness level. In preferred embodiments, the increase sweetness level is greater than an initial sweetness level of a comparative sweetener compositionally the same as the aforementioned sweetener composition of the invention but without the compound of formula (I) or a stereoisomer or a salt or a hydrate thereof.

In one aspect, the present invention relates to a method for preparing a sweetener composition comprising pregelatinized starch. The inventive method comprises the step of applying a sweetener and a compound of formula (I) or a stereoisomer or a salt or a hydrate thereof to the pregelatinized starch. In one embodiment, the inventive method comprises the step of combining, e.g., admixing, a sweetener and a compound of formula (I) or a stereoisomer or a salt or a hydrate thereof with a pregelatinized starch to form a release controlled sweetener composition.

For loading the pregelatinized starch material with a sweetener and a compound of formula (I) or a stereoisomer or a salt or a hydrate thereof the starch material may be placed in a vessel supporting mechanical mixing and preferable capable of being sealed. Suitable mixing devices are, for example, a paddle mixer, a ribbon blender, a V-blender, or a plough blade mixer. The sweetener and a compound of formula (I) or a stereoisomer or a salt or a hydrate thereof may then be supplied, for example poured, pumped or, preferably, sprayed via a nozzle, into the vessel and applied onto the agitated pregelatinized starch material. In some embodiments, the sweetener and a compound of formula (I) or a stereoisomer or a salt or a hydrate thereof are supplied in the form of a sweetener mixture comprising a sweetener and a compound of formula (I) or a stereoisomer or a salt or a hydrate thereof and a carrier. Spraying via a nozzle is advantageously used because the nozzle leads to the formation of small droplets that are more easily absorbed by the pregelatinized starch. Loading from the gas phase or under supercritical conditions is also possible. The mixing may be continued until an even distribution of the sweetener into and/or onto the pregelatinized starch is obtained. The time required for spraying or pumping is dependent upon the addition level of the sweetener and a compound of formula (I) or a stereoisomer or a salt or a hydrate thereof onto the pregelatinized starch and the time required to ensure complete absorption to form a free flowing powder.

In one embodiment, the sweetener compositions of the present invention are formed by contacting the pregelatinized starch with a sweetener mixture comprising the sweetener and a compound of formula (I) or a stereoisomer or a salt or a hydrate thereof and a carrier. Preferably, the carrier is glycerol, however ever other suitable carriers may be employed. Without being bound by theory, the pregelatinized starch has a plurality of pores and capillaries. Upon contacting the sweetener mixture with these pores and/or capillaries, the sweetener and a compound of formula (I) or a stereoisomer or a salt or a hydrate thereof are distributed therein. The sweetener and a compound of formula (I) or a stereoisomer or a salt or a hydrate thereof that are disposed in the pores and/or capillaries may then be released, e.g., released at a prolonged rate, as pressure is applied to the sweetener composition or to the consumable product that comprises the composition, e.g., via chewing.

Another suitable method for loading the sweetener and a compound of formula (I) or a stereoisomer or a salt or a hydrate thereof onto the pregelatinized starch material may be a fluidized-bed loading process. In such a process, the pregelatinized starch material is fluidized by forcing air or another gas upward through a bed of starch particles. The sweetener and a compound of formula (I) or a stereoisomer or a salt or a hydrate thereof are then sprayed via a nozzle onto the fluidized starch particles to yield a sweetener-loaded starch material of evenly loaded starch particles. Again, the sweetener and a compound of formula (I) or a stereoisomer or a salt or a hydrate thereof may be supplied in the form of a sweetener mixture comprising the sweetener and a carrier.

A further suitable loading method for use herein comprises the steps of suspending the pregelatinized starch carrier material in the sweetener and the compound of formula (I) or a stereoisomer or a salt or a hydrate thereof followed by separating the powdered sweetener-loaded starch material from the liquid components by conventional separation methods, such as filtration or centrifugation.

Depending on the type of sweetener(s) to be loaded, the sweetener(s) may be heated or cooled. In case of high viscous liquid components, for example, it might be favourable to heat the sweetener(s) to decrease the viscosity and facilitate the loading process. In case of temperature-sensitive sweetener(s), cooling might be desired or required. Means for cooling or heating, such as a cooled or heated blender, are well-known to a person skilled in the art.

In accordance with the present invention, the pregelatinized starch may be pre-treated before loading with an inert gas to remove, for instance, oxygen. The pregelatinized starch may also be vacuum-treated before loading to increase the absorption capacity. Further, when sensitive liquids are to be loaded, the loading operation might be carried out under an inert gas atmosphere, for example under a nitrogen atmosphere to protect against loss of quality by oxidation.

After having loaded the pregelatinized starch material with one or more sweetener(s), further processing steps may optionally follow. For example, flowing or anti-caking agents may be added to the sweetener-loaded starch material, such as tricalcium phosphate, silica, silicates and/or stearates, to increase flowability. The powdered sweetener-loaded starch material of the present invention may also be provided with a coat and/or further encapsulated by any suitable encapsulating or coating materials, such as maltodextrins, starches, modified starches, dextrins, oils, fats, waxes, hydrocolloids, proteins, as known in the art.

The inventive sweetener compositions may further comprise celluloses. As used herein, the term "cellulose" refers to any cellulosic material (other than the pregelatinized starches described herein) known to the skilled person. As indicated above, the pregelatinized starches of utilized in the present invention are different from conventional starches or celluloses and possess particular characteristics that provide for some of the features of the inventive sweetener composition. These characteristics are not present in all starches or cellulosic materials. Thus, it is not expected that conventional starches and/or celluloses would provide for the inventive features demonstrated by the present invention.

In typical embodiments, the cellulose includes polysaccharides having linear chains of at least several hundred beta-linked D-glucose units. When obtained from commercial sources, for example, the cellulose may exist as a powder. Further, in some embodiments, the cellulose is insoluble or substantially insoluble in water. In other embodiments, when incorporated into a consumable product, the cellulose preferably will not detract substantially from the overall product dissolution. Chemically modified celluloses can be employed in the compositions of the invention provided the modifications do not result in water soluble material. The cellulose may have any particle size (or particle size distribution) that is suitable for use in a sweetener composition. For example, in some embodiments, the size of the cellulose particles may range from about 1 micron to about 400 microns, e.g., from about 3 microns to about 300 microns, from about 5 microns to about 200 microns, or from about 6 microns to about 100 microns. In some embodiments, the insoluble cellulose is a cellulose that if used in amounts exceeding 1% in an aqueous medium can lead to significant viscosity change.

### Formulations

In another aspect, the present invention relates to formulations of the sweetener composition.

In these formulations, the sweetener composition of the invention may take any suitable form including, but not limited to, an amorphous solid, a crystal, a powder, a tablet, a liquid, a cube, a glace or coating, a granulated product, an encapsulated form abound to or coated on to carriers/particles, wet or dried, or combinations thereof.

For example, in one embodiment, the sweetener composition formulations can be provided in pre-portioned packets or ready-to-use formulations, which include a compound of formula (I) as defined above. For example, in one embodiment, a single serving packet formulation (usually about a 1 gram portion) can provide sweetness comparable to that contained in two teaspoons of granulated sugar (sucrose). It is known in the art that a "teaspoon" of sucrose contains approximately 4 grams of sucrose.

In another embodiment, a volume of a ready-to-use formulation can provide sweetness comparable to the same volume of granulated sugar. Preferably, a single serving packet of the composition comprising a compound of formula (I) as defined above (e.g. 1 gram) can provide sweetness comparable to about 0.9 to about 9.0 grams of granulated sugar (sucrose). In another embodiment, 1 gram of the sweetener composition contains less calories and carbohydrates than about 1 gram of granulated sugar.

As used herein, the term "about" encompasses the range of experimental error that occurs in any measurement. Unless otherwise stated, all measurement numbers are presumed to have the word "about" in front of them if the word "about" is not expressly used.

The formulation of the invention may contain further additives known to those skilled in the art. These additives include but are not limited to bubble forming agents, bulking agents, carriers, fibers, sugar alcohols, oligosaccharides, sugars, high intensity sweeteners, nutritive sweeteners, flavorings, flavor enhancers, flavor stabilizers, acidulants, anti-caking and free-flow agents. Such additives are for example described by H. Mitchell (H. Mitchell, "Sweeteners and Sugar Alternatives in Food Technology", Backwell Publishing Ltd, 2006).

As used herein, the term "flavorings" may include those flavors known to the skilled person, such as natural and artificial flavors. These flavorings may be chosen from synthetic flavor oils and flavoring aromatics and/or oils, oleoresins and extracts derived from plants, leaves, flowers, fruits, and so forth, and combinations thereof. Nonlimiting representative flavor oils include spearmint oil, cinnamon oil, oil of wintergreen (methyl salicylate), peppermint oil, Japanese mint oil, clove oil, bay oil, anise oil, eucalyptus oil, thyme oil, cedar leaf oil, oil of nutmeg, allspice, oil of sage, mace, oil of bitter almonds, and cassia oil. Also useful flavorings are artificial, natural and synthetic fruit flavors such as vanilla, and citrus oils including lemon, orange, lime, grapefruit, yazu, sudachi, and fruit essences including apple, pear, peach, grape, blueberry, strawberry, raspberry, cherry, plum, pineapple, watermelon, apricot, banana, melon, apricot, ume, cherry, raspberry, blackberry, tropical fruit, mango, mangosteen, pomegranate, papaya and so forth. Other potential flavors include a milk flavor, a butter flavor, a cheese flavor, a cream flavor, and a yogurt flavor; a vanilla flavor; tea or coffee flavors, such as a green tea flavor, a oolong tea flavor, a tea flavor, a cocoa flavor, a chocolate flavor, and a coffee flavor; mint flavors, such as a peppermint flavor, a spearmint flavor, and a Japanese mint flavor; spicy flavors, such as an asafetida flavor, an ajowan flavor, an anise flavor, an angelica flavor, a fennel flavor, an allspice flavor, a cinnamon flavor, a camomile flavor, a mustard flavor, a cardamom flavor, a caraway flavor, a cumin flavor, a clove flavor, a pepper flavor, a coriander flavor, a sassafras flavor, a savory flavor, a Zanthoxyli Fructus flavor, a perilla flavor, a juniper berry flavor, a ginger flavor, a star anise flavor, a horseradish flavor, a thyme flavor, a tarragon flavor, a dill flavor, a capsicum flavor, a nutmeg flavor, a basil flavor, a marjoram flavor, a rosemary flavor, a bayleaf flavor, and a wasabi (Japanese horseradish) flavor; alcoholic flavors, such as a wine flavor, a whisky flavor, a brandy flavor, a rum flavor, a gin flavor, and a liqueur flavor; floral flavors; and vegetable flavors, such as an onion flavor, a garlic flavor, a cabbage flavor, a carrot flavor, a celery flavor, mushroom flavor, and a tomato flavor. These flavoring agents may be used in liquid or solid form and may be used individually or in admixture. Commonly used flavors include mints such as peppermint, menthol, spearmint, artificial vanilla, cinnamon derivatives, and various fruit flavors, whether employed individually or in admixture. Flavors may also provide breath freshening properties, particularly the mint flavors when used in combination with cooling agents. A preferred flavoring is menthol and, in one embodiment, the inventive sweetener composition comprises acesulfame potassium, menthol, and pregelatinized starch. Flavors may also provide breath freshening properties, particularly the mint flavors when used in combination with cooling agents. These flavorings may be used in liquid or solid form and may be used individually or in admixture.

Other useful flavorings include aldehydes and esters such as cinnamyl acetate, cinnamaldehyde, citral diethylacetal, dihydrocarvyl acetate, eugenyl formate, p-methylamisol, and so forth may be used. Generally any flavoring or food additive such as those described in Chemicals Used in Food Processing, publication 1274, pages 63-258, by the National Academy of Sciences, may be used.

Further examples of aldehyde flavorings include but are not limited to acetaldehyde (apple), benzaldehyde (cherry, almond), anisic aldehyde (licorice, anise), cinnamic aldehyde (cinnamon), citral, i.e., alpha-citral (lemon, lime), neral, i.e., beta-citral (lemon, lime), decanal (orange, lemon), ethyl vanillin (vanilla, cream), heliotrope, i.e., piperonal (vanilla, cream), vanillin (vanilla, cream), alpha-amyl cinnamaldehyde (spicy fruity flavors), butyraldehyde (butter, cheese), valeraldehyde (butter, cheese), citronellal (modifies, many types), decanal (citrus fruits), aldehyde C-8 (citrus fruits), aldehyde C-9 (citrus fruits), aldehyde C-12 (citrus fruits), 2-ethyl butyraldehyde (berry fruits), hexenal, i.e., trans-2 (berry fruits), tolyl aldehyde (cherry, almond), veratraldehyde (vanilla), 2,6-dimethyl-5-heptenal, i.e., melonal (melon), 2,6-dimethyloctanal (green fruit), and 2-dodecenal (citrus, mandarin), cherry, grape, strawberry shortcake, and mixtures thereof. These listings of flavorings are merely exemplary and are not meant to limit either the term "flavoring" or the scope of the invention generally.

In some embodiments, the flavoring may be employed in either liquid form and/or dried form. When employed in the latter form, suitable drying means such as spray drying the oil may be used. Alternatively, the flavoring may be absorbed onto water soluble materials, such as cellulose, starch, sugar, maltodextrin, gum arabic and so forth or may be encapsulated. The actual techniques for preparing such dried forms are well-known.

In some embodiments, the flavorings may be used in many distinct physical forms well-known in the art to provide an initial burst of flavor and/or a prolonged sensation of flavor. Without being limited thereto, such physical forms include free forms, such as spray dried, powdered, beaded forms, encapsulated forms, and mixtures thereof.

### Tabletop Sweetener Compositions

In another aspect, the present invention relates to tabletop sweetener compositions comprising a compound of formula (I) 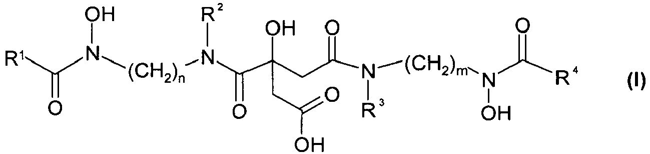 wherein
R¹ and R⁴ are identical or different and are
   C₁-C₈-alkyl, C₁-C₈-alkoxy, C₂-C₈-alkenyl, C₂-C₈-alkynyl, C₃-C₈-cycloalkyl, C₃-C₈-cycloalkenyl, wherein each of the above mentioned radicals is unsubstituted or substituted by one or more radicals selected from the group consisting of halogen or hydroxyl, aryl, preferably phenyl or naphthyl, wherein the aryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy, or
   heteroaryl, wherein the heteroaryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy,
R² and R³ are identical or different and are hydrogen or C₁-C₈-alkyl and,
n and m are identical or different and are an integer from 1 to 5,
or a stereoisomer or a salt or a hydrate thereof, and to methods of manufacturing such tabletop sweetener compositions.

The preferred embodiments of the compound of the compound of formula (I) as mentioned above apply accordingly to the tabletop sweetener compositions according to the present invention.

"Tabletop sweetener," as used herein, refers to sweetener compositions that comprise at least one sweetener, and optionally, at least one sweetness enhancer, which can be used in the preparation of various food items and/or as an additive to food items. As one example, the tabletop sweetener may be used in the preparation of baked goods or other sweetened foods. As another example, the tabletop sweetener may be used to season, sweeten, or otherwise customize a prepared food item, e.g., beverages, fruit, or yoghurt. In a preferred aspect, the tabletop sweetener is in a crystalline, granulated, or powder form. In various aspects, the tabletop sweetener will comprise one or more sweeteners and/or one or more sweetness enhancers. In one embodiment, the tabletop sweetener may comprise the sweetness enhancer in combination with either or both a caloric sweetener and/or substantially non-caloric sweeteners. Typical examples of caloric sweeteners that may be used in tabletop sweeteners include sucrose, fructose, and glucose. Common tabletop forms of these caloric sweeteners include cane sugar, bee sugar, and the like. In recent decades, substantially non-caloric sweeteners have gained popularity. In many instances, these sweeteners can be used as substitutes for caloric sweeteners and are often referred to as "sugar substitutes."

In many instances, sugar substitutes provide a greater sweetening effect than comparable amounts of caloric sweeteners, such as sucrose or fructose. Therefore, smaller amounts of sugar substitutes are required to achieve sweetness comparable to that of an amount of sugar. Sugar substitutes, however, typically have a taste profile that differs from sucrose or fructose. Such differences include, but are not limited to, increased astringency, bitterness, various aftertastes, delayed onset of sweetness, and different mouthfeel. Therefore, sugar substitutes are often formulated with other materials that can provide bulk and can enhance the taste profile to be more similar to that of sucrose or fructose. Thus, sugar substitutes have been formulated to create a tabletop sweetener formulation that has a bulk and a taste profile that is comparable to sucrose or fructose. Nevertheless, consumers can still distinguish the low-calorie sweetener formulations from caloric tabletop sweeteners. Therefore, if low-calorie tabletop sweeteners are to replace caloric tabletop sweeteners, formulations of low-calorie sweeteners must be continuously improved to meet consumer demand.

There is an increasing interest in such sweeteners containing natural ingredients. This interest stems partially from increasing consumer interest in such products, but also from the rise of retail and internet stores selling natural products, and requiring suppliers of such products to certify that natural ingredients are used in any products being supplied.

Therefore, there is a need for new tabletop sweetener formulations which are low in calories (or have no calories) and that can reasonably approximate the taste profile, mouthfeel, and texture of caloric sweeteners.

The invention, in another aspect, relates to a tabletop sweetener composition comprising
(a) at least one sugar sweetener, which is selected from the group consisting of monosaccharides, disaccharides, oligosaccharides and polysaccharides, preferably the at least one sugar sweetener is selected from the group consisting of arabinose, dextrin, dextrose, fructose, high fructose corn syrup, fructooligosaccharides, fructooligosaccharide syrups, galactose, galactooligosaccharides, glucose, glucose and (hydrogenated) starch syrups/hydrolysates, isomaltulose, lactose, hydrolysed lactose, maltose, mannose, rhamnose, ribose, sucrose, stachyose, tagatose, trehalose, xylose, and combinations thereof, most preferably the at least one sugar sweetener is a disaccharide and/or fructose;
(b) at least one sugar alcohol (or polyol), which is selected from the group consisting of erythritol, galactitol, hydrogenated starch syrups including maltitol and sorbitol syrups, inositols, isomalt, lactitol, maltitol, mannitol, xylitol, and combinations thereof, preferably the at least one sugar alcohol is erythritol;
(c) a compound of formula (I) 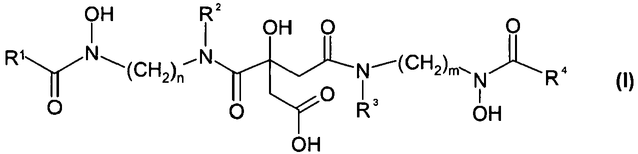 wherein
   R¹ and R⁴ are identical or different and are
      C₁-C₈-alkyl, C₁-C₈-alkoxy, C₂-C₈-alkenyl, C₂-C₈-alkynyl, C₃-C₈-cycloalkyl, C₃-C₈-cycloalkenyl, wherein each of the above mentioned radicals is unsubstituted or substituted by one or more radicals selected from the group consisting of halogen or hydroxyl,
      aryl, preferably phenyl or naphthyl, wherein the aryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy, or
      heteroaryl, wherein the heteroaryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy,
   R² and R³ are identical or different and are hydrogen or C₁-C₈-alkyl and,
   n and m are identical or different and are an integer from 1 to 5,
   or a stereoisomer or a salt or a hydrate thereof and
(d) a taste-improving amount of cellulose.

In one embodiment, the invention relates to a tabletop sweetener composition comprising
(a) a disaccharide carbohydrate and/or fructose;
(b) erythritol;
(c) a compound of formula (I) as defined above or a stereoisomer or a salt or a hydrate thereof; and
(d) a taste-improving amount of cellulose.

In some embodiments of the invention, the tabletop sweetener composition comprises a disaccharide and contains no fructose. In other embodiments, the tabletop sweetener composition comprises fructose and does not contain disaccharide. In other embodiments, the tabletop sweetener compositions comprise both a disaccharide and fructose.

As used herein, the term "disaccharide" refers to any sugar having two monosaccharide units. The monosaccharide units may exist as either ketones or aldehydes, and may have either a cyclic or acyclic structure. When a monosaccharide exists as a cyclic structure, the monosaccharide may exist as a hemiacetal or hemiketal, among other forms. Moreover, when a monosaccharide exists as a cyclic structure, either anomer is included within this definition. Illustrative monosaccharides include trioses, tetroses, pentoses, hexoses, heptoses, octoses, and nonoses. In forming a disaccharide, the monosaccharide units may bond to form either reducing disaccharides or non-reducing disaccharides.

As used herein, the terms "sugar alcohol(s)" or "polyol(s)" refer to sugar alcohols such as but not limited to erythritol, galactitol, hydrogenated starch syrups including maltitol and sorbitol syrups, inositols, isomalt, lactitol, maltitol, mannitol, xylitol, and combinations thereof.

As used herein, the term "erythritol" refers to a sugar alcohol well known to the skilled person. Erythritol, in either food grade or reagent grade is readily available through commercial sources.

As used herein, the term "cellulose" refers to any cellulosic material known to the skilled person. In typical embodiments, the cellulose includes polysaccharides having linear chains of at least several hundred beta-linked D-glucose units. When obtained from commercial sources, for example, the cellulose may exist as a powder. Further, in typical embodiments, the cellulose is insoluble or substantially insoluble in water; yet, in an application like tabletop sweeteners, when incorporated in such an application, it preferably will not detract substantially from the overall product dissolution. Chemically modified celluloses can be employed in the compositions of the invention provided the modifications do not result in water soluble material. The cellulose may have any particle size (or particle size distribution) that is suitable for use in a sweetener composition. For example, in some embodiments, the size of the cellulose particles may range from about 1 micron to about 400 microns, e.g., from about 3 microns to about 300 microns, from about 5 microns to about 200 microns, or from about 6 microns to about 100 microns. In some embodiments, the insoluble cellulose is a cellulose that if used in amounts exceeding 1% in an aqueous medium can lead to significant viscosity change.

In some embodiments of the invention, a "taste-improving amount" of cellulose is used. This "taste-improving amount" refers to an amount of cellulose that imparts an unexpected improvement in the taste profile of sweetener compositions of the invention. In some instances, for example, the taste improvement may be perceived as an enhancement in the sweetness of the sweetener composition or of the beverage or foodstuff containing the sweetener composition. In other instances, for example, the taste improvement may be perceived as a reduction or masking of the bitterness of the sweetener composition or of the beverage or foodstuff containing the sweetener composition. The taste improvement may also be a combination of both sweetness enhancement and bitterness reduction. In some embodiments of the sweetener compositions, the taste-improving amount of cellulose ranges from about 0.4 % by weight to about 3.0 % by weight, e.g. from about 0.7 % by weight to about 2.0 % by weight, of cellulose, based on the total weight of the sweetener composition. In some embodiments, the sweetener composition contains about 1 % by weight cellulose, based on the total weight of the sweetener composition.

In one embodiment, the disaccharide includes, but is not limited to, disaccharides containing glucose, fructose, and galactose. In another embodiment, the disaccharide carbohydrate includes, but is not limited isomaltulose, lactose, maltose, sucrose, and trehalose. In another embodiment, the disaccharide is isomaltulose.

In a preferred embodiment of the invention, the disaccharide is selected from the group consisting of isomaltulose, lactose, maltose, sucrose, and trehalose.

Sweetener compositions of the invention may contain varying amounts of at least one sugar sweetener, in particular of disaccharide and/or fructose, of at least one sugar alcohol, in particular of erythritol, of a compound of formula (I) as defined above or a stereoisomer or a salt or a hydrate thereof, and of cellulose. The desired amount of a compound of formula (I) as defined above or a stereoisomer or a salt or a hydrate thereof may vary depending on, among other factors, the desired use of the tabletop sweetener composition, the presence or absence of other components in the tabletop sweetener composition, the identity of any sugar sweetener, in particular of any disaccharide, if present, and the presence or absence of fructose.

In some embodiments, the tabletop sweetener composition contains from about 40 % by weight to about 70 % by weight sugar alcohol, in particular erythritol, based on the total weight of the sweetener composition, e.g., from about 50 % by weight to about 60 % by weight, from about 55 % by weight to about 65 % by weight, from about 57 % by weight to about 63 % by weight, or from about 60 % by weight to about 62 % by weight. In some embodiments, the sweetener composition contains about 55 % by weight sugar alcohol, in particular erythritol, based on the total weight of the sweetener composition. In still other embodiments, the sweetener composition contains about 61-62 % by weight sugar alcohol, in particular erythritol, based on the total weight of the sweetener composition.

In some embodiments, the tabletop sweetener composition contains from about 27 % by weight to about 50 % by weight sugar sweetener, in particular disaccharide, based on the total weight of the tabletop sweetener composition, e.g., from about 35 % by weight to about 45 % by weight from about 30 % by weight to about 40 % by weight, from about 30 % by weight to about 38 % by weight, from about 32 % by weight to about 36 % by weight, or from about 33 % by weight to about 35 % by weight. In some such embodiments, the tabletop sweetener composition contains about 41 % by weight sugar sweetener, in particular disaccharide, based on the total weight of the sweetener composition. In still other embodiments, the tabletop sweetener composition contains about 33-34 % by weight sugar sweetener, in particular disaccharide, based on the total weight of the sweetener composition. In a preferred embodiment, the sugar sweetener is isomaltulose.

In some embodiments, the sweetener composition contains from about 0.5 % by weight to about 7.0 % by weight a compound of formula (I) as defined above or a stereoisomer or a salt or a hydrate thereof, based on the total weight of the sweetener composition, e.g., from about 0.7 % by weight to about 5.0 % by weight, or from about 1.0 % by weight to about 2.5 % by weight. The amount of a compound of formula (I) as defined above or a stereoisomer or a salt or a hydrate thereof used may in certain situations depend on the purity of the material.

In another embodiment, tabletop sweetener compositions of the invention contain (a) from about 38 % by weight to about 43 % by weight of isomaltulose; (b) from about 50 % by weight to about 60 % by weight erythritol; (c) from about 0.75 % by weight to about 1.75 % by weight compound of formula (I) as defined above or a stereoisomer or a salt or a hydrate thereof; and (d) from about 0.5 % by weight to about 1.5 % by weight cellulose; based on the total weight of the tabletop sweetener composition.

In another embodiment, tabletop sweetener compositions of the invention contain (a) from about 30 % by weight to about 38 % by weight of isomaltulose; (b) from about 55 % by weight to about 65 % by weight erythritol; (c) from about 0.75 % by weight to about 1.75 % by weight compound of formula (I) as defined above or a stereoisomer or a salt or a hydrate thereof; and (d) from about 0.5 % by weight to about 1.5 % by weight cellulose; based on the total weight of the tabletop sweetener composition.

Tabletop sweetener compositions of the invention may also contain amounts of other ingredients in addition to sugar sweetener, in particular in addition to disaccharide and/or fructose, the sugar alcohol such as erythritol, a compound of formula (I) as defined above or a stereoisomer or a salt or a hydrate thereof with the sugar alcohol, in particular in place of or in combination and cellulose. Such additional ingredients include, but are not limited to, sweetness modifiers, mouthfeel enhancers, flavorings (e.g., vanilla flavoring), and the like. Honey and/or evaporated cane juice may be used in place of or in combination with erythritol. Natural flavors and other ingredients are preferred when the product is to be labeled as "all-natural."

In another embodiment, the tabletop sweetener composition comprises less than about 2 % by weight of a sweetness modifier, e.g., less than about 0.5% by weight. In terms of ranges, the tabletop sweetener composition may, for example, comprise between about 0.01 % by weight and about 2 % by weight sweetness modifier, in particular between about 0.1 % by weight and about 1.5 % by weight sweetness modifier.

In another embodiment, the tabletop sweetener composition comprises less than about 1 % by weight of a mouthfeel enhancer, e.g., less than about 0.5% by weight. In terms of ranges, the tabletop sweetener composition may, for example, comprise between about 0.01 % by weight and about 1 % by weight mouthfeel enhancer, in particular between about 0.1 % by weight and about 0.5 % by weight mouthfeel enhancer.

In another embodiment, the tabletop sweetener composition comprises less than about 1 % by weight of a flavoring, e.g., less than about 0.5% by weight. In terms of ranges, the tabletop sweetener composition may, for example, comprise between about 0.01 % by weight and about 1 % by weight flavoring, in particular between about 0.1 % by weight and about 0.5 % by weight flavoring.

In some embodiments, sweetener compositions of the invention provide at least one, if not more than one, of the following desirable characteristics: (a) fewer calories per gram than standard table sugar; (b) fewer calories than an amount of standard table sugar perceived as providing comparable sweetness; and (c) lower glycemic index than that of standard table sugar. In some embodiments, the sweetener composition has less than about 5 calories/gram, or less than about 3 calories/gram, or less than about 1 calorie/gram. As used herein, the term "calorie" refers to the unit of energy commonly appearing on the packaging of food and/or beverage items sold in the United States. The term, as such, does not refer to 1 cal. of energy, but rather corresponds to approximately 1 kcal. of energy. In a typical tabletop sweetener application, for example, the sweetener composition can be packaged in a form where it provides a similar sweetness to about 7 grams of sucrose, preferably 5 g of sucrose, while providing less than about 5 calories.

In another embodiment, tabletop sweetener compositions of the invention contain a plurality of sweetener particles, wherein such particles contain one or more of the ingredients present in the tabletop sweetener composition. In some embodiments, the tabletop sweetener composition substantially comprises sweetener particles. In such embodiments, the tabletop sweetener composition contains at least about 80 % by weight sweetener particles, or at least about 85 % by weight sweetener particles, or at least about 90 % by weight sweetener particles, based on the total weight of the tabletop sweetener composition.

Sweetener particles, when present in the tabletop sweetener composition, can have any size suitable for use of the composition as a sweetener. In some embodiments, the average size of the sweetener particles is between about 50 microns and about 1250 microns, e.g., between about 100 microns and about 1000 microns. Screening to eliminate particles of undesired sizes can be carried out during the manufacturing process. Thus, in some embodiments, the particle sizes, after screening to eliminate undesired large particles which may be as large as 1500 µm, may vary up to about 16 mesh, e.g. up to about 14 mesh, or up to about 12 mesh, based on the standard United States sieve scale. Further, smaller particle sizes, e.g., about 50 mesh, 100 mesh, or 150 mesh, or particles having sizes less than about 1 µm, e.g., less than about 0.5 µm, may be present with the larger particles. Screening to eliminate particles having sizes less than, for example, about 100 mesh or 150 mesh can be carried out if desired.

Sweetener particles in the tabletop sweetener composition may or may not have uniform composition. Preferably, the tabletop sweetener compositions of the invention comprise a compound of formula (I) as defined above or a stereoisomer or a salt or a hydrate thereof and an effective amount of cellulose where the composition is a mixture of particles. More specifically, the mixture comprises (a) particles having an erythritol core and (b) particles having a disaccharide core and a compound of formula (I) as defined above and the cellulose, as well as other components, are predominantly coated on the particles. These coatings on the cores can be either a continuous phase or a discontinuous phase, i.e., where the different coating components form discrete regions in the core coatings.

Thus, in another aspect, the invention relates to a tabletop sweetener composition comprising:
(a) a plurality of first sweetener particles, wherein the first sweetener particles have (i) a sugar alcohol core, in particular an erythritol core, (ii) a first sugar alcohol core-coating layer, in particular a first erythritol core-coating layer comprising a compound of formula (I) as defined above or a salt or a hydrate thereof and cellulose, and (iii) a second sugar alcohol core-coating layer, in particular a second erythritol core-coating layer comprising a sugar sweetener, in particular a disaccharide, wherein the second sugar alcohol core-coating layer, in particular the second erythritol core-coating layer lies outside of the first sugar alcohol core-coating layer, in particular outside of the first erythritol core-coating layer; and
(b) a plurality of second sweetener particles, wherein the second sweetener particle has (i) a sugar sweetener core, in particular a disaccharide core, (ii) a first sugar sweetener core-coating layer, in particular a first disaccharide core-coating layer comprising a compound of formula (I) or a stereoisomer or a salt or a hydrate thereof and cellulose, and (iii) a second sugar sweetener core-coating layer, in particular a second disaccharide core-coating layer comprising a sugar sweetener, in particular a disaccharide, wherein the second sugar sweetener core-coating layer, in particular the second disaccharide core-coating layer lies outside of the first sugar sweetener core-coating layer, in particular outside of the first disaccharide core-coating layer.

In such embodiments, the core-coating layers may or may not have uniform compositions, and may or may not substantially coat the underlying core or layer. In some embodiments, the first sugar alcohol core-coating layer, in particular the first erythritol core-coating layer and/or the first sugar sweetener core-coating layer, in particular the first disaccharide core-coating layer have discrete regions of a compound of formula (I) as defined above or a stereoisomer or a salt or a hydrate thereof and cellulose.

In another embodiment, the tabletop sweetener composition comprises a mixture of the plurality of first sweetener particles and the plurality of second sweetener particles.

In another embodiment of the tabletop sweetener composition, the sugar sweetener core, in particular the disaccharide core contains isomaltulose. Further, in some embodiments, the second sugar alcohol core-coating layer, in particular the second erythritol core-coating layer and/or the second sugar sweetener core-coating layer, in particular the second disaccharide core-coating layer contain isomaltulose.

These tabletop sweetener compositions may also contain flavorings (e.g., vanilla flavor), mouthfeel enhancers, and/or sweetness modifiers. When one or more of these are present, the first sugar alcohol core-coating layer, in particular the first erythritol core-coating layer and/or the sugar sweetener core-coating layer, in particular the disaccharide core-coating layer may contain one or more of flavorings (e.g., vanilla flavor), mouthfeel enhancers, and/or sweetness modifiers. Moreover, as used herein, the term "layer" may or may not refer to a material that entirely surrounds the underlying material. Thus, a "layer" may be non-uniform in composition and may provide only discontinuous coverage of the underlying material. Moreover, when one layer covers another, the boundary between the layers may or may not be discrete; thus, the boundary between layers may be continuous or semi-continuous.

In the sweetener compositions described herein, the tabletop sweetener compositions may or may not contain other particles in addition to the plurality of first sweetener particles and the plurality of second sweetener particles. The first sweetener particles and the second sweetener particles may have any particle size that is suitable for use of the composition as a sweetener.

In some embodiments, the average size of the first sweetener particles and second sweetener particles is between about 50 microns and about 1250 microns, e.g., between about 100 microns and about 1000 microns. In some embodiments, the particle sizes of the first sweetener particles and the second sweetener particles, after screening to eliminate undesired large particles which may be as large as 1500 µm, will vary up to about 16 mesh, e.g., up to about 14 mesh, or up to about 12 mesh, based on the standard United States sieve scale. Further, smaller particle sizes, e.g., about 50 mesh, 100 mesh, or 150 mesh, or particles having sizes less than about 1 µm, e.g., less than about 0.5 µm, will be present with the larger particles. In some embodiments, the tabletop sweetener composition comprises a mixture of the plurality of first sweetener particles and the second sweetener particles. Such a mixture may or may not contain other types of particles.

The layers in the sweetener composition particles are generally not distinct, i.e., there is no clear demarcation between the first layer and the second layer. For example, in one embodiment, the first layer contains a compound of formula (I) as defined above or a stereoisomer or a salt or a hydrate thereof, optional flavoring components, etc., all encased in sugar sweetener, in particular encased in disaccharide; and the second layer will be predominantly sugar sweetener, in particular disaccharide with some of the other components. The relative quantities of the various components in the layers, and whether there are layers in the particles, can be modified as necessary by adjusting when during the manufacturing process the components are added.

In some embodiments of the invention, the tabletop sweetener composition comprises a compound of formula (I) as defined above or a stereoisomer or a salt or a hydrate thereof and a taste-improving amount of cellulose as a mixture, where the mixture comprises (a) particles a sugar alcohol core, in particular having an erythritol core and (b) particles a sugar sweetener core, in particular having a disaccharide core. In some such embodiments, the disaccharide core comprises isomaltulose. Further, in some such embodiments, the sugar alcohol core, in particular the erythritol core and/or the sugar sweetener core, in particular the disaccharide core further comprise coating layers having discrete regions of a compound of formula (I) as defined above or a stereoisomer or a salt or a hydrate thereofand cellulose. When such coating layers are present, the coating layers may or may not substantially coat the underlying core material. These particles may have any particle size that is suitable for use of the composition as a sweetener. In some embodiments, the average size of the particles is between about 50 microns and about 1250 microns, e.g., between about 100 microns and about 1000 microns. In some embodiments, the particle sizes of the particles range from about 16 mesh, or from about 14 mesh, or from about 12 mesh to about 100 mesh, based on the standard United States sieve scale.

Sweetener compositions of the invention may have any dissolution rate in water that is suitable for their use as sweeteners. In some embodiments, the sweetener composition can have a dissolution rate in water at 10°C of between about 100 seconds and about 200 seconds, e.g., between about 125 seconds and about 175 seconds, or between about 140 seconds and 160 seconds, based on the dissolution of about 2 grams of the sweetener composition in 240 ml of water. In some embodiments, the sweetener composition can have a dissolution rate in water at 45°C of between about 50 seconds and about 150 seconds, e.g., between about 75 seconds and about 125 seconds, or between about 85 seconds and 110 seconds, based on the dissolution of about 2 grams of the sweetener composition in 240 ml of water. In some embodiments, the dissolution rate of the sweetener composition is about 150 seconds at 10°C and about 96 seconds at 45°C, based on the dissolution of about 2 grams of the sweetener composition in 240 mL of stirred water.

In another embodiment, the invention relates to single-serving packets.

In another embodiment, the invention relates to tabletop sweeteners comprising a compound of formula (I) or a stereoisomer or a salt or a hydrate thereof. Preferably, the tabletop sweetener is a tabletop tablet sweetener, a tabletop "spoon to spoon" sweetener, a tabletop "sachet" sweetener, a tabletop liquid sweetener. The tabletop sweeteners, in addition to the compound of formula (I) or a stereoisomer or a salt or a hydrate thereof component, may contain further substances including but not limited to aspartame, acesulfame potassium, binding agents, citric acid, cyclamate, lactose, carboxymethylcellulose, leucin, maltodextrin, isomalt, NHDC, potassium hydroxide (in aqueous solution), dextrose, other bulking agents, sucralose, sodium cyclamate, sodium hydrogen carbonate, sodium saccharin and tartric acid.

In another embodiment, the invention relates to a package containing a predetermined amount, e.g., from about 0.8 grams to about 3.5 grams, of a solid tabletop sweetener composition, where the predetermined amount of the solid tabletop sweetener composition has a sweetness equivalent to about four times (by weight) the predetermined amount of sucrose, and where the solid sweetener composition comprises:
(a) from about 38 % by weight to about 43 % by weight of isomaltulose;
(b) from about 50 % by weight to about 60 % by weight erythritol;
(c) from about 0.75 % by weight to about 1.75 % by weight of a compound of formula (I) as defined above or a stereoisomer or a salt or a hydrate thereof; and
(d) from about 0.5 % by weight to about 1.5 % by weight cellulose.

In another embodiment, the invention relates to a package containing a predetermined amount, e.g., from about 0.8 grams to about 3.5 grams, of a solid sweetener composition, where the predetermined amount of the solid sweetener composition has a sweetness equivalent to about four times (by weight) the predetermined amount of sucrose, and where the solid sweetener composition comprises:
(a) from about 30 % by weight to about 38 % by weight of isomaltulose;
(b) from about 55 % by weight to about 65 % by weight erythritol;
(c) from about 0.75 % by weight to about 1.75 % by weight of a compound of formula (I) as defined above or a stereoisomer or a salt or a hydrate thereof; and
(d) from about 0.5 % by weight to about 1.5 % by weight cellulose.

In the tabletop sweetener packages containing a predetermined amount of the solid tabletop sweetener composition, the predetermined amount may be about 1 gram and may have a sweetness equivalent to about 4 grams of sucrose, or the predetermined amount may be about 2 grams and may have a sweetness equivalent to about 8 grams of sucrose.

The tabletop sweetener packages may contain a formulation for a ready-to-use sweetener or tabletop sweetener compositions in the form of cubes for use, for example, in restaurants. The cubes weigh approximately 8 grams and are of equivalent size to a standard cube of granulate sugar, which is 2.2cm x 2.2 cm x 1 cm.

Tabletop sweetener compositions of the invention may have any bulk density that is suitable for their use as sweeteners. In some embodiments, the bulk density of the sweetener composition ranges from about 0.5 g/cm³ to about 1.0 g/cm³, or from about 0.7 g/cm³ to about 0.8 g/cm³. In some embodiments, the bulk density of the sweetener composition is about 0.76 g/cm³.

In another aspect, the invention relates to a method of making a tabletop sweetener composition, comprising the steps of:
a) providing a fluid-bed coating apparatus;
b) introducingdry sugar sweetener, in particular dry disaccharide carbohydrate and/or fructose, dry sugar alcohol, in particular dry erythritol, dry compound of formula (I) or a stereoisomer or a salt or a hydrate thereof and dry cellulose powder to the fluid-bed coating apparatus;
c) charging a substantially all of the dry ingredients in the fluid-bed coating apparatus;
d) spraying a coating solution into the fluid-bed coating apparatus to form coated sweetener particles; and
e) drying the coated sweetener particles.

In another aspect, the invention relates to a method of making a tabletop sweetener composition, comprising the steps of:
a) providing a fluid-bed coating apparatus;
b) introducing dry sugar sweetener, in particular dry disaccharide and/or fructose, dry sugar alcohol, in particular dry erythritol and dry compound of formula (I) or a stereoisomer or a salt or a hydrate thereof to the fluid-bed coating apparatus;
c) charging a substantially all of the dry ingredients in the fluid-bed coating apparatus;
d) spraying a coating solution into the fluid-bed coating apparatus to form coated sweetener particles;
e) during the spraying step, introducing dry cellulose powder to the fluid-bed coating apparatus; and
f) drying the coated sweetener particles.

The methods of the invention described above may be carried out as described in WO 2010/025158 A1.

### Consumables containing a Sweetener Composition or a Tabletop Sweetener Composition of the Invention

The inventive sweetener compositions comprising the compound of formula (I) as defined above or a stereoisomer or a salt or a hydrate thereof described above can be added to any consumable products including but not limited to beverages, dental products, cosmetic products, pharmaceutical products and animal feed or animal food. The inventive tabletop sweetener compositions as described above can be added to any consumable products, which are produced in a household or on a small scale.

Thus, in one aspect, the invention relates to a consumable comprising
(a) a consumable product; and
(b) a sweetener composition of the invention as defined above.

Thus, in one aspect, the invention relates to a consumable comprising
(a) a consumable product; and
(b) a tabletop sweetener composition of the invention as defined above.

In one embodiment, the consumable comprises at least one, e.g., at least two sweeteners, in addition to the compound of formula (I) or a stereoisomer or a salt or a hydrate thereof. In these embodiments, the compound of formula (I) or a stereoisomer or a salt or a hydrate thereof may function as an enhancer to enhance the sweetness of the at least one sweetener.

Preferably, the sweetener composition of the invention and the tabletop sweetener composition of the invention are present in the consumable in an amount effective to increase a sweetness level of the consumable.

As used herein, the unit "wppm" refers to weight parts per million and means 1 milligram per kilogram.

Preferably, in the consumable of the invention the compound of formula (I) as defined above or a stereoisomer or a salt or a hydrate thereof is present in a concentration from 0.1 wppm to 100 wppm, in particular 0.2 wppm to 50 wppm, particularly preferred from 0.5 wppm to 10 wppm.

In embodiments wherein pregelatinized starch is used, the concentration of the at least one sweetener and the pregelatinized starch in the consumable product may vary widely. In one embodiment of the invention, the consumable product composition comprises the at least one sweetener in a concentration from 0.1 wt% to 5 wt%, e.g., from 0.5 wt% to 2 wt%, or from 0.5 wt% to 1.5 wt%.

In one embodiment of the invention, the consumable comprises the pregelatinized starch in an amount ranging from 0.1 wt% to 9.5 wt%, e.g., from 5 wt% to 9.5 wt%, e.g., from 8 wt% to 9.5 wt%

The following consumable products and their ingredients are suitable for use in embodiments of the present invention.

Consumable products include all food products, including but not limited to cereal products, rice products, tapioca products, sago products, baker's products, biscuit products, pastry products, bread products, confectionary products, desert products, gums, chewing gums, chocolates, ices, honey products, treacle products, yeast products, baking-powder, salt and spice products, savory products, mustard products, vinegar products, sauces (condiments), tobacco products, cigars, cigarettes, processed foods, cooked fruits and vegetable products, meat and meat products, jellies, jams, fruit sauces, egg products, milk and dairy products, yoghurts, cheese products, butter and butter substitute products, milk substitute products, soy products, edible oils and fat products, medicaments, beverages, carbonated beverages, alcoholic drinks, beers, soft drinks, mineral and aerated waters and other non-alcoholic drinks, fruit drinks, fruit juices, coffee, artificial coffee, tea, cacoa, including forms requiring reconstitution, food extracts, plant extracts, meat extracts, condiments, sweeteners, nutraceuticals, gelatins, pharmaceutical and non-pharmaceutical gums, tablets, lozenges, drops, emulsions, elixirs, syrups and other preparations for making beverages, and combinations thereof.

As used herein, the term "non-alcoholic drinks" includes, but is not limited to all non-alcoholic drinks mentioned in the Directive 2003/115/EC of 22 December 2003 and in the Directive 94/35/EC of 30 June 2004 on sweeteners for use in foodstuffs. Examples include, but are not limited to water-based, flavored drinks, energy-reduced or with no added sugar, milk- and milk-derivative-based or fruit-juice-based drinks, energy-reduced or with no added sugar, "Gaseosa": non-alcoholic water-based drink with added carbon dioxide, sweeteners and flavorings.

Consumable products include without limitation, water-based consumables, solid dry consumables, dairy products, dairy-derived products and dairy-alternative products.

In one embodiment, the consumable product is a water-based consumable product including but not limited to beverage, water, aqueous beverage, enhanced/slightly sweetened water drink, flavored carbonated and still mineral and table water, carbonated beverage, non-carbonated beverage, carbonated water, still water, soft drink, non-alcoholic drink, alcoholic drink, beer, wine, liquor, fruit drink, juice, fruit juice, vegetable juice, broth drink, coffee, tea, black tea, green tea, oolong tea, herbal infusion, cacoa (e.g. water-based), tea-based drink, coffee-based drinks, cacao-based drink, infusion, syrup, frozen fruit, frozen fruit juice, water-based ice, fruit ice, sorbet, dressing, salad dressing, jams, marmalades, canned fruit, savoury, delicatessen products like delicatessen salads, sauces, ketchup, mustard, pickles and marinated fish, sauce, soup, and beverage botanical materials (e.g. whole or ground), or instant powder for reconstitution (e.g. coffee beans, ground coffee, instant coffee, cacao beans, cacao powder, instant cacao, tea leaves, instant tea powder).

In another embodiment, the consumable product is a solid dry consumable product including but not limited to cereals, baked food products, biscuits, bread, breakfast cereal, cereal bar, energy bars/nutritional bars, granola, cakes, rice cakes, cookies, crackers, donuts, muffins, pastries, confectionaries, chewing gum, chocolate products, chocolate, fondant, hard candy, marshmallow, pressed tablets, snack foods, botanical materials (whole or ground), and instant powders for reconstitution.

In another embodiment, the consumable product is selected from the group of a dairy product, dairy-derived product and dairy-alternative product, including but not limited to milk, fluid milk, cultured milk product, cultured and noncultured dairy-based drink, cultured milk product cultured with lactobacillus, yoghurt, yoghurt-based beverage, smoothie, lassi, milk shake, acidified milk, acidified milk beverage, butter milk, kefir, milk-based beverages, milk/juice blend, fermented milk beverage, ice cream, dessert, sour cream, dip, salad dressing, cottage cheese, frozen yoghurt, soy milk, rice milk, soy drink, and rice milk drink.

Preferably, the consumable product is a carbonated drink and the invention relates to a carbonated drink comprising a sweetener composition of the invention or a tabletop sweetener composition of the invention.

Preferably, the consumable product is a non-carbonated drink and the invention relates to a non-carbonated drink comprising a sweetener composition of the invention or a tabletop sweetener composition of the invention.

In another embodiment, the consumable products are alcoholic beverages and the invention relates to alcoholic beverages comprising a sweetener composition of the invention or a tabletop sweetener composition of the invention, preferably to shandy beer, wine cooler, wildberry cooler (e.g. 5% alcohol), strawberry daiquiri cooler (e.g. 5% alcohol), margarita cooler (e.g. 5% alcohol) and raspberry cooler. In addition, the alcoholic beverages may contain further substances including but not limited to acesulfame potassium, aspartame, beer, color, citric acid monohydrate, cyclamate, fruit juice (e.g. peach, pineapple), lemon flavor, margarita flavor, rum flavor, sucrose, vodka, wildberry flavor, wine and water.

In another embodiment, the consumable products are fruit juices and the invention relates to fruit juices comprising a sweetener composition of the invention or a tabletop sweetener composition of the invention, preferably to functional fruit drinks (e.g. 30 % fruit juice content), fruit nectar, fruit juice drinks, no sugar added fruit beverages (e.g. 5 % juice, kiwi-strawberry flavored) and ruby red grapefruit and tangerine juice drinks (e.g. from concentrate). In addition, the fruit juices may contain further substances including but not limited to aspartame, anthocyane, ascorbic acid, carotinoids, citric acid (e.g. anhydrous), cyclamate, luteine, fruit concentrate, fruit juice concentrate, flavor, fruit, grapefruit pulp cells, grapefruit flavor, kiwi juice concentrate, kiwi-strawberry flavor, malic acid, pectin, ruby red grapefruit concentrate, strawberry juice concentrate, tangerine juice concentrate, tangerine flavor, vegetable extract (e.g. grape, pumpkin, carrot, aronia, blackcurrant, hibiscus etc.) and water.

In another embodiment, the consumable product is ice tea and the invention relates to ice tea comprising a sweetener composition of the invention or a tabletop sweetener composition of the invention, preferably to ice tea and sugar free ice tea mix. In addition, the ice tea may contain further substances including but not limited to base with lemon flavor, base with tea component, citric acid, cyclamate, flavor, instant tea, lemon juice, maltodextrin, malic acid (e.g. powdered), saccharin, sucralose, sucrose, tea and tea extract.

In another embodiment, the consumable products are soft drinks without sugar and the invention relates to soft drinks without sugar comprising a sweetener composition of the invention or a tabletop sweetener composition of the invention, preferably to soft drinks Cola flavored, fruit nectars, fruit juice drinks, soft drinks, soft drinks lemon lime flavored, diet sparkling waters (e.g. peach flavored) and sugar free liquid beverages. In addition, the soft drinks without sugar may contain further substances including but not limited to acesulfame potassium, alitame, aspartame, bilberry flavor, citric acid monohydrate, caffeine, cola flavor, cyclamate, peach flavor, potassium citrate, sodium-cyclamate, grape color, grape flavor, sodium benzoate, sodium citrate, sodium-saccharin, ethylmaltol, flavor, lemon-lime flavor, maltol, neotame, NHDC, passion fruit flavor, pectin, phosphoric acid (e.g. 85%), saccharin, sucralose and water.

In another embodiment, the consumable products are soft drinks with sugar and the invention relates to soft drinks with sugar comprising a sweetener composition of the invention or a tabletop sweetener composition of the invention. In addition, the soft drinks with sugar may contain further substances including but not limited to aspartame, citric acid monohydrate, concentrate, caffeine, flavor, fructose, glucose, glucose syrup, high fructose con syrup (HFCS, e.g. HFCS having total solids: approx. 77 %, fructose: 55 % and glucose: 41 %), neotame, orangeade base, phosphoric acid (e.g. 85%), sodium-cyclamate, sucrose and water.

In another embodiment, the consumable products are sports drinks and the invention relates to sports drinks comprising a sweetener composition of the invention or a tabletop sweetener composition of the invention, preferably to isotonic energy drinks and whey drinks. In addition, the sports drinks may contain further substances including but not limited to acesulfame potassium, aspartame, ascorbic acid, concentrate, caffeine, citric acid, flavor, glucose (anhydrous), herbs, minerals, neohesperidine-DC, natural extracts, sucralose, taurine, vitamins, water and whey powder.

In another embodiment, the consumable products are dry powder beverages and the invention relates to dry powder beverages comprising a sweetener composition of the invention or a tabletop sweetener composition of the invention. In addition, the dry powder beverages may contain further substances including but not limited to acesulfame potassium, aspartame, apple flavor, ascorbic acid, citric acid, cherry flavor, malic acid, orange flavor, raspberry flavor, sodium chloride, trisodium citrate, tricalcium phosphate, titanium dioxide and xantham gum.

In another embodiment, the consumable product is ice coffee and the invention relates to ice coffee comprising a sweetener composition of the invention or a tabletop sweetener composition of the invention. In addition, the ice coffee may contain further substances including but not limited to acesulfame potassium, aspartame, coffee extract, ethylmaltol, flavor and neohesperidine-DC.

In another embodiment, the consumable products are instant cake fillings and the invention relates to instant cake fillings comprising a sweetener composition of the invention or a tabletop sweetener composition of the invention. In addition, the cake fillings may contain further substances including but not limited to milk, isomalt, oligofructose, modified starch, flavors and colors. In another embodiment, the cake fillings may contain further substances including but not limited to raspberries, strawberry puree, polydextrose, isomalt, sorbitol, glycerin, fructose, pectin, locust bean gum, calcium chloride, sodium bicarbonate, citric acid and water.

In another embodiment, the consumable products are biscuits and the invention relates to biscuits comprising a sweetener composition of the invention or a tabletop sweetener composition of the invention. In addition, the biscuits may contain further substances including but not limited to isomalt, powdered isomalt, granulated isomalt, polydextrose, shortening, water, sodium bicarbonate, ammonium bicarbonate, skimmed milk powder, salt, flour, cake flour, flavor, inulin, wheat fiber, shortening, ground raisins, raisin paste, salt, oatrim gel, liquid whole eggs, liquid egg whites, powdered egg whites, egg yolk, vanilla, butter flavor, vanilla flavor, chocolate flavor, cocoa, high fructose corn syrup (HFCS), methocel, baking soda, cinnamon, sodium acid pyrophosphate, margarine spread, margarine, emulsifier, molasses, mono- and diglycerides, powdered cellulose, ground hazelnuts, hazelnuts, sorbitol, oat fiber, vital wheat gluten, chocolate chips, maltitol and fat replacer.

In another embodiment, the consumable products are cakes and the invention relates to cakes comprising a sweetener composition of the invention or a tabletop sweetener composition of the invention. In addition, the cakes may contain further substances including but not limited to baking powder, baking soda, blueberry flavor, all purpose flour, cake flour, diacetyl 4X, dextrose, dried butter flavor, flour, cellulose, crystalline fructose, emulsifier, egg whites solid, eggs, dried egg white, fat replacers such as inulin, isomalt, lecithin, milk, non fat dry milk, modified starch, maltodextrin, oligofructose, potato fiber, polydextrose, salt, shortening, crystalline sorbitol, sodium aluminium phosphate, sucrose, butter flavor, chocolate flavor, (dried) vanilla flavor, water, wheat fiber, xanthan gum and vegetable oil.

In another embodiment, the consumable products are bakery products other than cakes and the invention relates to bakery products other than cakes comprising a sweetener composition of the invention or a tabletop sweetener composition of the invention, preferably to light hot fudge toppings, torteletts with strawberry fillings, sugar free maple flavored syrups, sugar free dark chocolate coatings, sugar free chocolate syrups, reduced-calorie chocolate syrups, no sugar added caramel corn, light chocolate frostings, light caramel toppings and light apple tart. In addition, the bakery products may contain further substances including but not limited to acesulfame potassium, aspartame, baking powder, baking soda, disodium phosphate, maple flavor, caramel flavor, caramel color, flour, carrageenan, cocoa powder, cocoa butter, (microcrystalline) cellulose, citric acid, calcium chloride, crystalline fructose, fructose, chocolate liquor, eggs, dried egg white, fudge flavor, isomalt, lecithin, non fat dry milk, hydrogenated starch hydrolysate, margarine, modified starch, maltisorb, maltodextrin, nonfat dry milk, oligofructose, potassium sorbate, pectin, potato fiber, hydrogenated potato starch, polydextrose, skimmed milk powder, shortening, (crystalline) sorbitol, sodium benzoate, salt, sorbitol, (powdered) sucrose, butter flavor, chocolate flavor, vanillin, (dried) vanilla flavor, water, wheat fiber and xanthan gum.

In another embodiment, the consumable products are confectionary products and the invention relates to confectionary products comprising a sweetener composition of the invention or a tabletop sweetener composition of the invention, preferably to all confectionary products mentioned in the Directive 2003/115/EC of 22 December 2003 and in the Directive 94/35/EC of 30 June 2004 on sweeteners for use in foodstuffs. Examples include, but are not limited to, confectionaries (with or without added sugar), cocoa- or dried-fruit-based confectionaries, energy-reduced or with not added sugar, starch-based confectionaries, energy-reduced or with not added sugar, comets and wafers for ice-cream, with not added sugar, *Essoblaten,* cocoa-, milk-, dried-fruit- or fat-based sandwich spreads, energy-reduced or with not added sugar, breakfast cereals, e.g., with a fiber content of more than 15%, and containing at least 20% bran, energy-reduced or sugar-reduced, breath-freshening micro-sweets with or without added sugar, strongly flavored freshening throat pastilles with or without added sugar, chewing gum with or without added sugar, energy-reduced tablet form confectionaries, cider and perry, drinks consisting of a mixture of a non-alcoholic drink and beer, cider, perry, spirits or wine, spirit drinks containing less than 15% alcohol by volume, alcohol-free beer or beer with an alcohol content not exceeding 1,2% vol., "bière de table/Tafelbier/table beer" (original wort content less than 6%), except for "obergäriges Einfachbier", beers with a minimum acidity of 30 milli-equivalents expressed as NaOH, brown beers of the "oud bruin" type, energy-reduced beer, edible ices, energy-reduced or sugar-reduced canned or bottled fruit, energy-reduced or with or without added sugar, energy-reduced jams, jellies and marmalades, energy-reduced fruit and vegetable preparations, sweet-sour preserves of fruit and vegetables, *Feinkostsalat,* sweet-sour preserves and semi-preserves of fish and marinades of fish, crustaceans and mollusks, energy-reduced soups, sauces, mustard, fine bakery products for special nutritional uses, foods intended for use in energy-restricted diets for weight reduction as referred to in Directive 1996/8/EC, dietary foods for special medical purposes as defined in Directive 1999/21/EC, food supplements as defined in Directive 2002/46/EC supplied in a liquid form, food supplements as defined in Directive 2002/46/EC supplied in a solid form, food supplements as defined in Directive 2002/46/EC, based on vitamins and/or mineral elements and supplied in a syrup-type or chewable form. Particularly preferred confectionary products are sugar free hard candy, reduced calorie no sugar added hard candy, hard candies, sugar free milk chocolate, milk chocolate, sugar free gummy bear, reduced calorie no sugar added gummy bear, sugar free dark chocolate, reduced calorie no sugar added hard candy, reduced calorie no sugar added caramel, reduced calorie caramel, raspberry jellies, jellies, plain bitter chocolate, toffees, sugar-free rice cake, sugar free peppermint breathmint, sugar free orange chewy candy and sugar free jelly beans. In addition, the confectionary products may contain further substances including but not limited to butter fat, (caramel) flavor, citric acid (monohydrate), cherry flavor, chocolate liquor, cocoa butter, cocoa mass, color, corn syrup, (microcrystalline) cellulose, disodium phosphate, egg Albumen-dried, evaporated milk, gelatin, glycerol monostearate, gum Arabic, hydrogenated starch hydrolysate, hydrogenated fat, isomalt, lecithin, lemon oil, maltitol (syrup, powdered and/or granular), medium-grain brown rice, Korean black rice, maltol, mocha paste, neohesperidine-DC, orange flavor, pectin, peppermint flavor, polydextrose, raspberry puree, raspberry puree, salt, sodium caseinate, sorbitol (powder), starch, sucrose, vanillin, vegetable fat, whole milk powder, skimmed milk powder, water and xylitol.

In another embodiment, the consumable products are delicacies sauces and the invention relates to delicacies sauces comprising a sweetener composition of the invention or a tabletop sweetener composition of the invention, preferably to sugar reduced ketchup with sugar, no added sugar Ketchup and tomato ketchup. In addition, the delicacies sauces may contain further substances including but not limited to citric acid, modified starch, mustard, onions, pectin, polydextrose, saccharine sodium, salt, spices, sucralose, sugar, thickener, tomato concentrate and vinegar.

In another embodiment, the consumable products are cereals and the invention relates to cereals comprising a sweetener composition of the invention or a tabletop sweetener composition of the invention.

In another embodiment, the consumable products are dairy products and the invention relates to dairy products comprising a sweetener composition of the invention or a tabletop sweetener composition of the invention, preferably to fruit quarks, whipped creams, (vanilla flavored skim) milk drinks and yoghurt drinks. In addition, the dairy products may contain further substances including but not limited to acesulfame potassium, aspartame, blackcurrant, blackberry, blueberry, cyclamate, flavor, fruit preparation, fruit juice concentrate, fructose, gelatin, inulin, oat, orange juice, pectin, raspberry, redcurrant, stabilizer, wheat fiber, water, quark, yoghurt, whipped cream and whey.

In another embodiment, the consumable products are desserts and the invention relates to desserts comprising a sweetener composition of the invention or a tabletop sweetener composition of the invention, preferably to jellied red fruit cocktails, strawberry sorbet, (fat-free/sugar-free) instant pudding chocolate flavors, instant desserts, vanilla puddings, vanilla pudding - powder mixtures and litchee gelees. In addition, the desserts may contain further substances including but not limited to acesulfame potassium, aspartame, blackberries, brandy, citric acid, caramel color, color, cyclamate, chocolate flavor, cocoa powder, corn starch, disodium phosphate, emulsifier, fructose, granulated sugar, white soft sugar, agar powder, ingestible dextrin, mannan, maltodextrin, mono- and diglycerides, inulin, polydextrose, lemon juice, maltodextrin, milk modified food starch, polydextrose, raspberries, redcurrant juice, salt, soy lecithin, strawberries, strawberry puree, tetrasodium pyrophosphate, litchee flavor, vanilla flavor, wheat starch, water and xanthan gum.

As used herein, the term "desserts" includes, but is not limited to all desserts mentioned in the Directive 2003/115/EC of 22 December 2003 and in the Directive 94/35/EC of 30 June 2004 on sweeteners for use in foodstuffs. Examples include, but are not limited to water-based flavored desserts, energy-reduced or with not added sugar, milk- and milk-derivative-paste preparations, energy-reduced or with no added sugar, fruit-and-vegetable-based desserts, energy-reduced or with no added sugar, egg-based desserts, energy-reduced or with no added sugar, cereal-based desserts, energy-reduced or with no added sugar, breakfast cereals or cereal-based products, energy-reduced or with no added sugar, fat-based desserts, energy-reduced or with no added sugar, edible ices, energy-reduced or with no added sugar, jams, jellies, marmalades and crystallized fruit, energy-reduced or with no added sugar, fruit preparations, energy-reduced or with no added sugar, and "snacks", certain flavors of ready-to-eat, prepacked, dry, savoury starch products and coated nuts.

In another embodiment, the consumable product is water-based ice and the invention relates to water-based ice comprising a sweetener composition of the invention or a tabletop sweetener composition of the invention, preferably to "ice-pops" and no sugar added strawberry sorbet. In addition, the water-based ice may contain further substances including but not limited to acesulfame potassium, aspartame, citric acid, color, fruit concentrate, flavor, isomalt, lemon juice, polydextrose, strawberry puree, sorbitol, thickener and water.

In another embodiment, the consumable product is ice cream and the invention relates to ice cream comprising a sweetener composition of the invention or a tabletop sweetener composition of the invention. In addition, the ice-cream may contain further substances including but not limited to color, emulsifier, flavor, isomalt, milk fat, fat replacer, skim milk powder, palatinit, polydextrose and lactitol.

In another embodiment, the consumable product is yoghurt and the invention relates to yoghurt comprising a sweetener composition of the invention or a tabletop sweetener composition of the invention. In addition, the yoghurt may contain further substances including but not limited to acesulfame potassium, alitame, aspartame, citric aid monohydrate, tri-calcium-dicitrate, cyclamate, Na-cyclamate, fruit preparation, high fructose corn syrup (HFCS), inulin, fructose, fructose syrup, oligofructose syrup, neohesperidine-DC, pectin-solution, saccharin, starch, strawberries, strawberry-flavor, sucralose, water and (low fat, preferably between 0,1 % to 1,5 % fat) yoghurt.

In another embodiment, the consumable products are jams and the invention relates to jams comprising a sweetener composition of the invention or a tabletop sweetener composition of the invention. In addition, the jams may contain further substances including but not limited to gelling agent, isomalt, maltitol, pectin, sorbitol and strawberries.

In another embodiment, the consumable product is chewing-gum and the invention relates to chewing-gum comprising a sweetener composition of the invention or a tabletop sweetener composition of the invention.

In one embodiment, the consumable product is an emulsion product. In such embodiments, the emulsion product comprises the sweetener, the pregelatinized starch, and a carrier. Suitable carriers are available commercially. One preferred carrier is menthol. In a preferred embodiment, the consumable product is a menthol-containing emulsion product comprising the sweetener, the pregelatinized starch, and menthol.

In one embodiment, the emulsion product is formed by preparing a finely ground sweetener, e.g., a sweetener having a low mean particle size, e.g., less than 50 microns, less than 25 microns, less than 10 microns, or less than 5 microns. The finely ground sweetener may be contacted with the carrier to form a suspension. In one embodiment, the carrier is heated, e.g., melted, prior to contact with the finely ground sweetener. In one embodiment, the suspension is then contacted with the pregelatinized starch to form the emulsion product.

Preferably, the finely ground sweetener comprises finely ground acesulfame potassium and the carrier comprises menthol. The finely ground sweetener may be contacted with heated, e.g., melted, menthol to form a suspension and the suspension may be contacted with the pregelatinized starch to form the menthol-containing emulsion product.

In one embodiment, the finely ground sweetener comprises finely ground sucrose.

The amount of a compound of formula (I) as defined above in the consumable of the invention is dependent on the concentration of the natural and or artificial sweeteners contained therein as well as on the presence of further auxiliary substances such as carbon dioxide, flavors (e.g. spices, natural extract or oils), colors, acidulants (e.g. phosphoric acid and citric acid), preservatives, potassium, sodium. The amount of a compound of formula (I) as defined above or a stereoisomer or a salt or a hydrate thereof may generally be between 0.01 mg and 10 g of a compound of formula (I) as defined above or a stereoisomer or a salt or a hydrate thereof per kg of the entire finished consumable, e.g., between 0.01 mg and 1 g of a compound of formula (I) as defined above or a stereoisomer or a salt or a hydrate thereof per kg, between 0.1 mg and 500 mg of a compound of formula (I) as defined above or a stereoisomer or a salt or a hydrate thereof per kg, or between 0.1 mg and 50 mg of a compound of formula (I) as defined above or a stereoisomer or a salt or a hydrate thereof per kg.

In another embodiment, the consumable product is a dental product and the invention relates to a dental product comprising a sweetener composition of the invention. Dental products include, but are not limited to toothpaste, dental floss, mouthwash, denture adhesive, enamel whitener, fluoride treatments and oral care gels. These products are also known in the art.

In a preferred embodiment the consumable product is toothpaste and the invention relates to toothpaste comprising a sweetener composition of the invention. In addition, the toothpaste may contain further substances including but not limited to abrasive silica, dicalcium phosphate dehydrate, hydrated silica (thickener), ethyl alcohol, peppermint flavor, mint flavor, potassium sorbate, sodium lauryl sulphate, sodium carboxymethylcellulose, sodium monofluorophosphate, sodium monofluorophosphate, sorbitol solution, tetrasodium phosphate and titanium dioxide.

In another embodiment, the consumable product is a cosmetic product and the invention relates to a cosmetic product comprising a sweetener composition of the invention. Cosmetic products include but are not limited to lipstick, lip balm, lip gloss, and petroleum jelly. These products are also known in the art.

In another embodiment, the consumable product is a pharmaceutical product and the invention relates to a pharmaceutical product comprising a sweetener composition of the invention or a tabletop sweetener composition of the invention. Pharmaceutical products include but are not limited to over-the-counter and prescription drugs including but not limited to non-tobacco snuff, tobacco substitutes, chewable medications, cough syrups, throat sprays, throat lozenges, cough drops, antibacterial products, pill coatings, gel caplets, soluble fiber preparations, antacids, tablet cores, rapidly absorbed liquid compositions, stable foam compositions, rapidly disintegrating pharmaceutical dosage forms, beverage concentrates for medicinal purposes, aqueous pharmaceutical suspensions, liquid concentrate compositions, and stabilized sorbic acid solutions, phosphate buffers, saline solutions, emulsion, non-aqueous pharmaceutical solvents, aqueous pharmaceutical carriers, solid pharmaceutical carrier, and pharmaceutical preservatives/additives (antimicrobials, antioxidants, chelating agents, inert gases, flavoring agents, coloring agents).

In another embodiment, the consumable product is animal feed or animal food and the invention relates to animal feed or animal food comprising a sweetener composition of the invention.

In one embodiment, the consumable product is a chewing gum. As one example, the sweetener composition may comprise pregelatinized starch and the sweetener may comprise acesulfame potassium.

In one embodiment, the consumable product is a chewing gum. As one example, the sweetener composition may comprise pregelatinized starch and the sweetener may comprise sucrose. Preferably, in embodiments wherein the consumable product is a chewing gum, the consumable product further comprises menthol.

In another aspect, the invention relates to a method of providing a sweetened consumable of the invention as defined above by admixing a sweetener composition of the invention as defined above or a tabletop sweetener composition of the invention as defined above to a consumable product.

In another aspect, the invention relates to a method of enhancing the taste sensations associated with flavor ingredients by admixing a sweetener composition of the invention as defined above or a tabletop sweetener composition as defined above with one or more flavor ingredients to provide a flavor-enhanced composition or consumable.

The invention, in another embodiment, relates to a method of increasing a sweetness level of a consumable having an initial sweetness level comprising the step of adding to the consumable a compound of formula (I) as defined above or a stereoisomer or a salt or a hydrate thereof in an amount effective to increase the sweetness level of the consumable to a final sweetness level.

Preferably, the compound of formula (I) may be used in a comestible composition in a concentration range of from 0.001 wppm to 1 wt.%, more in particular in a range of from 0.01 wppm to 0.1 wt.%, even more in particular in a range of from 0.01 wppm to 0.01 wt.%, even more in particular in a range of from 0.1 wppm to 0.001 wt.%, even more in particular in a range of from 0.01 wppm to 0.001 wt.%, and most particular in a range of from 0.1 wppm to 0.001 wt.%.

In another aspect, the present invention relates to a method of controlling the release rate of the at least one sweetener and the compound of formula (I) or a stereoisomer or a salt or a hydrate thereof and/or the taste sensations associated with the at least one sweetener and the compound of formula (I) or a stereoisomer or a salt or a hydrate thereof from a consumable. The consumable may comprise the at least one sweetener, a compound of formula (I) or a stereoisomer or a salt or a hydrate thereof and a consumable product. The method comprises the step of combining the at least one sweetener and the compound of formula (I) or a stereoisomer or a salt or a hydrate thereof, a pregelatinized starch, and the consumable product to form a released controlled consumable. In some embodiments, the release rate of the at least one sweetener and the compound of formula (I) or a stereoisomer or a salt or a hydrate thereof is controlled such that the above-discussed release rates are achieved. In preferred embodiments, the at least one sweetener and the compound of formula (I) or a stereoisomer or a salt or a hydrate thereof and the pregelatinized starch are combined to form a sweetener composition. This sweetener composition may then be combined with the consumable product to form the consumable product composition.

In another embodiment, the present invention relates to a method for decreasing the release rate of the at least one sweetener and the compound of formula (I) or a stereoisomer or a salt or a hydrate thereof from a consumable. The consumable comprises the at least one sweetener and the compound of formula (I) or a stereoisomer or a salt or a hydrate thereof and a consumable product and has an initial release rate of the at least one sweetener and the compound of formula (I) or a stereoisomer or a salt or a hydrate thereof from the consumable. The method comprises the step of adding to the consumable product composition a pregelatinized starch in an amount effective to decrease the release rate of the at least one sweetener and the compound of formula (I) or a stereoisomer or a salt or a hydrate thereof from the consumable to final release rate. In these embodiments, the addition of the pregelatinized starch to the at least one sweetener and the compound of formula (I) or a stereoisomer or a salt or a hydrate thereof surprisingly and unexpectedly decreases the release rate of the at least one sweetener and the compound of formula (I) or a stereoisomer or a salt or a hydrate thereof. In preferred embodiments, the addition of the pregelatinized starch to the at least one sweetener and the compound of formula (I) or a stereoisomer or a salt or a hydrate thereof provides for a final release rate similar to the release rates discussed above.

### Preferred Combinations of Sweeteners and Sweetness Enhancers

The compound of formula (I) as defined above or a stereoisomer or a salt or a hydrate thereof may also be combined with one or more, e.g., two or more or three or more, sweeteners (e.g. one or more sugar(s)) and/or sweetness enhancers in addition to thecompound of formula (I) as defined above or a stereoisomer or a salt or a hydrate thereof. In one embodiment, these combinations may be utilized in comestible goods, e.g., to sweeten the respective comestible good.

As used herein, the abbreviation "HSH" refers to hydrogenated starch hydrolyzates. The abbreviation "NHDC" refers to neohesperidine dihydrochalcone". A preferred Acesulfame K commercial product is Sunett®, from Nutrinova.

In some exemplary embodiments, the compound of formula (I) as defined above or a stereoisomer or a salt or a hydrate thereof may be combined with one or more sweetener(s), e.g. sugar(s) and/or sweetness enhancer(s), as shown in Table 1 below. The combinations in this listing are merely exemplary and are not meant to limit the scope of the invention.

**Table 1. Preferred Combinations of Sweeteners - General Overview**

| **Additive 1** | **Additive 2 (opt.)** | **Additive 3 (opt.)** | **Additive 4 (opt.)** | **Additive 5 (opt.)** |
|---|---|---|---|---|
| acesulfam K | | | | |
| alitame | | | | |
| aspartame | | | | |
| cyclamate | | | | |
| saccharine | | | | |
| neotame | | | | |
| sucrose | | | | |
| sucralose | | | | |
| fructose | | | | |
| isomalt | | | | |
| polydextrose | | | | |
| oligofructose | | | | |
| sorbitol | | | | |
| HSH | | | | |
| maltitol | | | | |
| alitame | fructose | | | |
| aspartame | fructose | | | |
| aspartame | sucrose | | | |
| aspartame | sucrose | | | |
| aspartame | cyclamate | | | |
| aspartame | NHDC | | | |
| aspartame | saccharine | | | |
| aspartame | alitame | | | |
| aspartame | inulin | | | |
| aspartame | isomalt | | | |
| aspartame | sucralose | | | |
| aspartame | maltol | | | |
| aspartame | maltodextrin | | | |
| cyclamate | sucralose | | | |
| cyclamate | sucrose | | | |
| cyclamate | fructose | | | |
| cyclamate | neotame | | | |
| cyclamate | maltol | | | |
| cyclamate | ethylmaltol | | | |
| saccharine | cyclamate | | | |
| isomalt | oligofructose | | | |
| isomalt | sorbitol | | | |
| isomalt | inulin | | | |
| isomalt | NHDC | | | |
| isomalt | HSH | | | |
| sorbitol | HSH | | | |
| maltitol | HSH | | | |
| sorbitol | polydextrose | | | |
| lactitol | polydextrose | | | |
| sorbitol | xylitol | | | |
| sorbitol | xylitol | | | |
| maltitol | polydextrose | | | |
| maltitol | sorbitol | | | |
| sucrose | fructose | | | |
| sucrose | polydextrin | | | |
| aspartame | sorbitol | polydextrose | | |
| aspartame | saccharin | fructose | | |
| aspartame | saccharin | sucrose | | |
| aspartame | saccharin | cyclamate | | |
| aspartame | cyclamate | fructose | | |
| aspartame | fructose | inulin | | |
| aspartame | sucralose | sucrose | | |
| cyclamate | sucralose | sucrose | | |
| cyclamate | saccharine | sucralose | | |
| cyclamate | neotame | sucrose | | |
| sucrose | maltodextrin | inulin | | |
| isomalt | sorbitol | polydextrose | | |
| isomalt | lactitol | polydextrose | | |
| isomalt | sorbitol | fructose | | |
| maltitol | sorbitol | polydextrose | | |
| isomalt | maltitol | xylitol | | |
| isomalt | maltitol | sorbitol | | |
| aspartame | sucrose | fructose | | |
| polydextrose | sucrose | maltodextrin | | |
| polydextrose | sorbitol | maltodextrin | | |
| dextrose | polydextrose | fructose | | |
| aspartame | cyclamate | saccharine | sucralose | |
| aspartame | sorbitol | polydextrose | sucrose | |
| isomalt | sorbitol | polydextrose | sucrose | |
| isomalt | sorbitol | polydextrose | fructose | |
| acesulfame K | alitame | | | |
| acesulfame K | aspartame | | | |
| acesulfame K | cyclamate | | | |
| acesulfame K | saccharine | | | |
| acesulfame K | neotame | | | |
| acesulfame K | sucrose | | | |
| acesulfame K | sucralose | | | |
| acesulfame K | fructose | | | |
| acesulfame K | isomalt | | | |
| acesulfame K | polydextrose | | | |
| acesulfame K | oligofructose | | | |
| acesulfame K | sorbitol | | | |
| acesulfame K | HSH | | | |
| acesulfame K | maltitol | | | |
| acesulfame K | alitame | fructose | | |
| acesulfame K | aspartame | fructose | | |
| acesulfame K | aspartame | sucrose | | |
| acesulfame K | aspartame | sucrose | | |
| acesulfame K | aspartame | cyclamate | | |
| acesulfame K | aspartame | NHDC | | |
| acesulfame K | aspartame | saccharine | | |
| acesulfame K | aspartame | alitame | | |
| acesulfame K | aspartame | inulin | | |
| acesulfame K | aspartame | isomalt | | |
| acesulfame K | aspartame | sucralose | | |
| acesulfame K | aspartame | maltol | | |
| acesulfame K | aspartame | maltodextrin | | |
| acesulfame K | cyclamate | sucralose | | |
| acesulfame K | cyclamate | sucrose | | |
| acesulfame K | cyclamate | fructose | | |
| acesulfame K | cyclamate | neotame | | |
| acesulfame K | cyclamate | maltol | | |
| acesulfame K | cyclamate | ethylmaltol | | |
| acesulfame K | saccharine | cyclamate | | |
| acesulfame K | isomalt | oligofructose | | |
| acesulfame K | isomalt | sorbitol | | |
| acesulfame K | isomalt | inulin | | |
| acesulfame K | isomalt | NHDC | | |
| acesulfame K | isomalt | HSH | | |
| acesulfame K | sorbitol | HSH | | |
| acesulfame K | maltitol | HSH | | |
| acesulfame K | sorbitol | polydextrose | | |
| acesulfame K | lactitol | polydextrose | | |
| acesulfame K | sorbitol | xylitol | | |
| acesulfame K | maltitol | polydextrose | | |
| acesulfame K | maltitol | sorbitol | | |
| acesulfame K | sucrose | fructose | | |
| acesulfame K | sucrose | polydextrin | | |
| acesulfame K | aspartame | sorbitol | polydextrose | |
| acesulfame K | aspartame | saccharin | fructose | |
| acesulfame K | aspartame | saccharin | sucrose | |
| acesulfame K | aspartame | saccharin | cyclamate | |
| acesulfame K | aspartame | cyclamate | fructose | |
| acesulfame K | aspartame | fructose | inulin | |
| acesulfame K | aspartame | sucralose | sucrose | |
| acesulfame K | cyclamate | sucralose | sucrose | |
| acesulfame K | cyclamate | saccharine | sucralose | |
| acesulfame K | cyclamate | neotame | sucrose | |
| acesulfame K | sucrose | maltodextrin | inulin | |
| acesulfame K | isomalt | sorbitol | polydextrose | |
| acesulfame K | isomalt | lactitol | polydextrose | |
| acesulfame K | isomalt | sorbitol | fructose | |
| acesulfame K | maltitol | sorbitol | polydextrose | |
| acesulfame K | isomalt | maltitol | xylitol | |
| acesulfame K | isomalt | maltitol | sorbitol | |
| acesulfame K | aspartame | sucrose | fructose | |
| acesulfame K | polydextrose | sucrose | maltodextrin | |
| acesulfame K | polydextrose | sorbitol | maltodextrin | |
| acesulfame K | dextrose | polydextrose | fructose | |
| acesulfame K | aspartame | cyclamate | saccharine | sucralose |
| acesulfame K | aspartame | sorbitol | polydextrose | sucrose |
| acesulfame K | isomalt | sorbitol | polydextrose | sucrose |
| acesulfame K | isomalt | sorbitol | polydextrose | fructose |

In other embodiments, the compound of formula (I) as defined above or a stereoisomer or a salt or a hydrate thereof may be combined with one or more, e.g., two or more or three or more, sweetener(s), e.g. sugar(s), and/or sweetness enhancer(s) that are known in the art but are not listed in Table 1. It should be understood that the compound of formula (I) as defined above or a stereoisomer or a salt or a hydrate thereof may, in some embodiments, be combined with at least one of any of the sweetener(s), e.g. sugar(s), and/or sweetness enhancer(s) that are listed in Table 1 and/or with any other sweetener(s), e.g. sugar(s), and/or sweetness enhancer(s) that are known in the art but are not listed in Table 1.

In some embodiments, when the compound of formula (I) as defined above or a stereoisomer or a salt or a hydrate thereof is used with comestible goods such as, e.g., bakery goods, biscuits, cake, other bakery goods, spread, confectionary, delicacies, ice cream, water-based ice, jams, oral hygiene, desserts, or yoghurt, the compound of formula (I) as defined above or a stereoisomer or a salt or a hydrate thereof may be combined with one or more sweetener(s), e.g. sugar(s), and/or sweetness enhancer(s) as follows:
a.) acesulfame K in a concentration range of from 0.01 to 50 g/kg, more preferably from 0.05 to 20 g/kg, even more preferably from 0.1 to 10 g/kg, most preferably of from 0.1 to 8.3 g/kg;
b.) aspartame in a concentration range of from 0.005 to 10 g/kg, more preferably from 0.01 to 7 g/kg, even more preferably from 0.01 to 1 g/kg, most preferably of from 0.06 to 1 g/kg;
c.) cyclamate in a concentration range of from 0.01 to 10.0 g/kg, more preferably from 0.05 to 5 g/kg, even more preferably from 0.1 to 2 g/kg, most preferably of from 0.1 to 1.5 g/kg;
d.) sucralose in a concentration range of from 0.05 to 10 g/kg, more preferably from 0.05 to 5 g/kg, even more preferably from 0.1 to 1 g/kg, most preferably of from 0.1 to 0.36 g/kg;
e.) saccharine in a concentration range of from 0.001 to 20 g/kg, more preferably from 0.001 to 1 g/kg, even more preferably from 0.005 to 0.5 g/kg, most preferably of from 0.03 to 0.15 g/kg;
f.) alitame in a concentration range of from 0.001 to 1 g/kg, more preferably from 0.001 to 0.5 g/kg, even more preferably from 0.005 to 0.1 g/kg, most preferably of from 0.01 to 0.03 g/kg;
g.) NHDC in a concentration range of from 0.0001 to 0.1 g/kg, more preferably from 0.0005 to 0.01 g/kg, even more preferably from 0.001 to 0.01 g/kg, most preferably of from 0.002 to 0.005 g/kg;
h.) sucrose in a concentration range of from 0.1 to 900 g/kg, more preferably from 1 to 500 g/kg, even more preferably from 10 to 500 g/kg, most preferably of from 10 to 300 g/kg;
i.) fructose in a concentration range of from 0.1 to 900 g/kg, more preferably from 1 to 500 g/kg, even more preferably from 5 to 500 g/kg, most preferably of from 5 to 100 g/kg;
j.) isomalt in a concentration range of from 10 to 950 g/kg, more preferably from 20 to 950 g/kg, even more preferably from 40 to 950 g/kg, most preferably of from 50 to 900 g/kg;
k.) maltitol in a concentration range of from 10 to 950 g/kg, more preferably from 15 to 950 g/kg, even more preferably from 25 to 950 g/kg, most preferably of from 50 to 900 g/kg;
l.) sorbitol in a concentration range of from 5 to 750 g/kg, more preferably from 10 to 500 g/kg, even more preferably from 20 to 500 g/kg, most preferably of from 30 to 300 g/kg;
m.) polydextrose in a concentration range of from 5 to 750 g/kg, more preferably from 10 to 750 g/kg, even more preferably from 20 to 500 g/kg, most preferably of from 30 to 300 g/kg;
n.) HSH in a concentration range of from 5 to 950 g/kg, more preferably from 25 to 800 g/kg, even more preferably from 50 to 750 g/kg, most preferably of from 100 to 650 g/kg;
o.) oligofructose in a concentration range of from 5 to 800 g/kg, more preferably from 25 to 750 g/kg, even more preferably from 50 to 500 g/kg, most preferably of from 70 to 200 g/kg;
p.) inulin in a concentration range of from 5 to 500 g/kg, more preferably from 25 to 250 g/kg, even more preferably from 50 to 100 g/kg, most preferably of from 20 to 70 g/kg;
q.) lactitol in a concentration range of from 5 to 500 g/kg, more preferably from 25 to 250 g/kg, even more preferably from 50 to 100 g/kg, most preferably of from 40 to 60 g/kg;
r.) xylitol in a concentration range of from 5 to 500 g/kg, more preferably from 10 to 250 g/kg, even more preferably from 25 to 100 g/kg, most preferably of from 40 to 300 g/kg; and/or
s.) maltodextrin in a concentration range of from 10 to 500 g/kg, more preferably from 25 to 250 g/kg, even more preferably from 50 to 100 g/kg, most preferably of from 75 to 100 g/kg.

The proposed combinations and concentration ranges listed above are merely exemplary and are not meant to limit the scope of the invention.

In other embodiments, in a liquid comestible good such as, e.g., a drink or a beverage, the compound of formula (I) as defined above or a stereoisomer or a salt or a hydrate thereof may be combined with one or more sweetener(s), e.g. sugar(s), and/or sweetness enhancer(s) as follows:
a.) acesulfame K in a concentration range of from 0.005 to 5 g/l, more preferably from 0.005 to 1 g/l, even more preferably from 0.01 to 0.5 g/l, most preferably of from 0.04 to 0.45 g/l;
b.) aspartame in a concentration range of from 0.005 to 5.0 g/l, more preferably from 0.005 to 1 g/l, even more preferably from 0.01 to 0.5 g/l, most preferably of from 0.03 to 0.26 g/l;
c.) cyclamate in a concentration range of from 0.005 to 10 g/l, more preferably from 0.01 to 2 g/l, even more preferably from 0.05 to 1 g/l, most preferably of from 0.2 to 0.7 g/l;
d.) sucralose in a concentration range of from 0.005 to 20 g/l, more preferably from 0.005 to 5 g/l, even more preferably from 0.01 to 2 g/l, most preferably of from 0.01 to 0.77 g/l;
e.) neotame in a concentration range of from 0.0001 to 2 g/l, more preferably from 0.0005 to 0.5 g/l, even more preferably from 0.001 to 0.02 g/l, most preferably of from 0.002 to 0.007 g/l;
f.) alitame in a concentration range of from 0.001 to 20 g/l, more preferably from 0.005 to 0.5 g/l, even more preferably from 0.01 to 0.1 g/l, most preferably of from 0.025 to 0.030 g/l;
g.) sucralose in a concentration range of from 0.001 to 20 g/l, more preferably from 0.001 to 1 g/l even more preferably from 0.005 to 0.1 g/l, most preferably of from 0.01 to 0.1 g/l;
h.) saccharine in a concentration range of from 0.001 to 20 g/l, more preferably from 0.001 to 1 g/l, even more preferably from 0.005 to 0.2 g/l, most preferably of from 0.03 to 0.09 g/l;
i.) NHCD in a concentration range of from 0.0001 to 0.1 g/l, more preferably from 0.0005 to 0.1 g/l, even more preferably from 0.001 to 0.01 g/l, most preferably of approximately 0.005 g/l;
j.) maltol in a concentration range of from 0.001 to 20 g/l, more preferably from 0.001 to 1.0 g/l, even more preferably from 0.005 to 0.2 g/l, most preferably of approximately 0.02 g/l;
k.) ethylmaltol in a concentration range of from 0.0001 to 2 g/l, more preferably from 0.0001 to 0.1 g/1, even more preferably from 0.0005 to 0.002 g/l, most preferably of from 0.007 to 0.020 g/l;
l.) sucrose in a concentration range of from 0.1 to 500 g/l, more preferably from 1 to 100 g/l, even more preferably from 10 to 100 g/l, most preferably of from 15 to 70 g/l; and/or
m.) fructose in a concentration range of from 0.1 to 500 g/l, more preferably from 1 to 100 g/l, even more preferably from 5 to 100 g/l, most preferably of from 10 to 20 g/l.

The proposed combinations and concentration ranges listed above are merely exemplary and are not meant to limit the scope of the invention.

In other embodiments, in a liquid or solid table top sweetener, the compound of formula (I) as defined above or a stereoisomer or a salt or a hydrate thereof may preferably be combined with one or more sweetener(s), e.g. sugar(s), and/or sweetness enhancer(s) as follows:
a.) acesulfame K in a concentration range of from 0.5 to 500 g/kg, more preferably from 1 to 250 g/kg, even more preferably from 5 to 150 g/kg, most preferably of from 5 to 135 g/kg;
b.) aspartame in a concentration range of from 0.5 to 200 g/kg, more preferably from 1 to 100 g/kg, even more preferably from 2 to 50 g/kg, most preferably of from 5 to 30 g/kg;
c.) cyclamate in a concentration range of from 1 to 500 g/kg, more preferably from 5 to 250 g/kg, even more preferably from 10 to 150 g/kg, most preferably of from 30 to 130 g/kg;
d.) sucralose in a concentration range of from 0.1 to 200 g/kg, more preferably from 0.5 to 100 g/kg, even more preferably from 1 to 50 g/kg, most preferably of from 1.5 to 20 g/kg;
e.) saccharin in a concentration range of from 0.1 to 200 g/kg, more preferably from 0.5 to 100 g/kg, even more preferably from 1 to 50 g/kg, most preferably of from 3 to 10 g/kg;
f.) NHCD in a concentration range of from 0.1 to 200 g/kg, more preferably from 0.5 to 100 g/kg, even more preferably from 1 to 50 g/kg, most preferably of from 1 to 5 g/kg;
g.) dextrose in a concentration range above 100 g/kg, more preferably above 250 g/kg, even more preferably above 500 g/kg, most preferably above 900 g/kg;
h.) maltodextrin in a concentration range above 100 g/kg, more preferably above 250 g/kg, even more preferably above 500 g/kg, most preferably above 900 g/kg;
i.) lactose in a concentration range above 50 g/kg, more preferably above 100 g/kg, even more preferably above 500 g/kg, most preferably above 800 g/kg.

The proposed combinations and concentration ranges listed above are merely exemplary and are not meant to limit the scope of the invention.

In other embodiments, when the compound of formula (I) as defined above or a stereoisomer or a salt or a hydrate thereof is used with comestible goods, the compound of formula (I) as defined above or a stereoisomer or a salt or a hydrate thereof may be combined with one or more, e.g., two or more or three or more, sweetener(s), e.g. sugar(s), and/or sweetness enhancer(s) that are known in the art but are not listed above. It should be understood that the compound of formula (I) as defined above or a stereoisomer or a salt or a hydrate thereof may, in some embodiments, be combined with at least one of any of the sweetener(s), e.g. sugar(s), and/or sweetness enhancer(s) that are listed above and/or with any other sweetener(s), e.g. sugar(s), and/or sweetness enhancer(s) that are known in the art.

In other embodiments, the compound of formula (I) as defined above or a stereoisomer or a salt or a hydrate thereof, when used as an additive in a comestible good such as, e.g., bakery goods, biscuits, cake, other bakery goods, spread, confectionary, delicacies, ice cream, water-based ice, jams, oral hygiene, desserts, or yoghurt, may be combined with sweetener(s), e.g. sugar(s), and/or sweetness enhancer(s) as shown in Table 2 below. The combinations in Table 2 are merely exemplary and are not meant to limit the scope of the invention.

**Table 2. Mass ranges - Solid and Semi-Solid Compositions**

| **Additive 1** | **Additive 2 (opt.)** | **Additive 3 (opt.)** | **Additive 4 (opt.)** | **Additive 5 (opt.)** |
|---|---|---|---|---|
| acesulfame K: | | | | |
| 0.8-2 g/kg | | | | |
| alitame: | | | | |
| 0.01-1.0 g/kg | | | | |
| aspartame: | | | | |
| 0.16-6.2 g/kg | | | | |
| cyclamate: | | | | |
| 0.1-10 g/kg | | | | |
| saccharine: | | | | |
| 0.1-10 g/kg | | | | |
| sucrose: | | | | |
| 1-100 g/kg | | | | |
| sucralose: | | | | |
| 0.10-0.36 g/kg | | | | |
| fructose: | | | | |
| 1-100 g/kg | | | | |
| isomalt: | | | | |
| 144.92-881.3 g/kg | | | | |
| isomalt: | | | | |
| 144.92-881.3 g/kg | | | | |
| polydextrose: | | | | |
| 10-500 g/kg | | | | |
| oligofructose: | | | | |
| 10-500 g/kg | | | | |
| sucralose: | | | | |
| 0.1-10 g/kg | | | | |
| sorbitol | | | | |
| 280.7-979.0 g/kg | | | | |
| HSH: | | | | |
| 796.8-981.0 g/kg | | | | |
| maltitol: | | | | |
| 851.0-851.1 g/kg | | | | |
| alitame: | fructose: | | | |
| 0.01-1 g/kg | 1-100 g/kg | | | |
| aspartame: | fructose: | | | |
| 0.10-0.16 g/kg | 8.0-10.0 g/kg | | | |
| aspartame: | sucrose: | | | |
| 0.10-0.3 g/kg | 20.0-225.0 g/kg | | | |
| aspartame: | cyclamate | | | |
| 0.14-0.28 g/kg | 0.12-1.2 g/kg | | | |
| aspartame: | NHDC: | | | |
| 0.15-0.18 g/kg | 0.002-0.005 g/kg | | | |
| aspartame: | saccharine: | | | |
| 0.06-0.14 g/kg | 0.03-0.08 g/kg | | | |
| aspartame: | alitame: | | | |
| 0.01-1 g/kg | 0.001-1 g/kg | | | |
| aspartame: | inulin: | | | |
| 0.01-1 g/kg | 1-100 g/kg | | | |
| aspartame: | isomalt: | | | |
| 0.01-10 g/kg | 10-1000 g/kg | | | |
| aspartame: | sucralose: | | | |
| 0.01-10 g/kg | 0.01-1 g/kg | | | |
| cyclamate: | fructose: | | | |
| 0.01-10 g/kg | 10-20.0 g/kg | | | |
| cyclamate: | sucralose: | | | |
| 0.1-10 g/kg | 0.01-1 g/kg | | | |
| isomalt: | oligofructose: | | | |
| 72.46-93.5 g/kg | 72.46-93.5 g/kg | | | |
| sorbitol: | polydextrose: | | | |
| 1-500 g/kg | 10-1000 g/kg | | | |
| lactitol: | polydextrose: | | | |
| 1-500 g/kg | 10-1000 g/kg | | | |
| maltitol: | polydextrose: | | | |
| 34.0-135.2 g/kg | 33.0-134.4 g/kg | | | |
| maltitol: | sorbitol: | | | |
| 199.8-266.7 g/kg | 176.0-281.3 g/kg | | | |
| isomalt: | polydextrose: | | | |
| 33.8-476.0 g/kg | 33.6-315.7 g/kg | | | |
| isomalt: | inulin: | | | |
| 1-500 g/kg | 10-1000 g/kg | | | |
| isomalt: | sorbitol: | | | |
| 1-500 g/kg | 10-1000 g/kg | | | |
| isomalt: | HSH: | | | |
| 10-1000 g/kg | 10-1000 g/kg | | | |
| sorbitol: | HSH: | | | |
| 56.3-75.6 g/kg | 482.1-639.5 g/kg | | | |
| maltitol: | HSH: | | | |
| 100-5000 g/kg | 10-1000 g/kg | | | |
| sorbitol: | xylitol: | | | |
| 10-1000 g/kg | 10-1000 g/kg | | | |
| isomalt: | xylitol: | | | |
| 345.0-400.7 g/kg | 40.0-41.0 g/kg | | | |
| isomalt: | NHDC: | | | |
| 10-1000 g/kg | 0.001-1 g/kg | | | |
| sucrose: | fructose: | | | |
| 10-1000 g/kg | 10-1000 g/kg | | | |
| sucrose: | maltodextrin: | | | |
| 10-1000 g/kg | 10-1000 g/kg | | | |
| sucrose: | polydextrose: | | | |
| 10-1000 g/kg | 10-1000 g/kg | | | |
| aspartam: | sorbitol: | polydextrose: | | |
| 0.25-0.55 g/kg | 10-1000 g/kg | 159.0-160.0 g/kg | | |
| aspartame: | saccharine: | fructose: | | |
| 0.01-1 g/kg | 0.1-10 g/kg | 1-1000 g/kg | | |
| aspartame: | saccharine: | sucrose: | | |
| 0.06-0.09 g/kg | 0.03-0.08 g/kg | 1-1000 g/kg | | |
| aspartame: | cyclamate: | fructose: | | |
| 0.01-1 g/kg | 0.1-10 g/kg | 1-1000 g/kg | | |
| aspartame: | isomalt: | polydextrose: | | |
| 0.01-1 g/kg | 1-100 g/kg | 1-1000 g/kg | | |
| aspartame: | sucrose: | fructose: | | |
| 0.01-1 g/kg | 10-1000 g/kg | 1-1000 g/kg | | |
| isomalt: | sorbitol: | fructose: | | |
| 1-500 g/kg | 10-1000 g/kg | 1-1000 g/kg | | |
| isomalt: | sorbitol: | polydextrose: | | |
| 63.8-344.6 g/kg | 31.9-51.7 g/kg | 53.9-170.2 g/kg | | |
| isomalt: | lactitol: | polydextrose: | | |
| 1-500 g/kg | 10-1000. g/kg | 1-1000 g/kg | | |
| isomalt: | polydextrose: | inulin: | | |
| 10-1000 g/kg | 10-1000 g/kg | 1-1000 g/kg | | |
| isomalt: | maltitol: | xylitol: | | |
| 10-1000 g/kg | 10-1000 g/kg | 1-1000 g/kg | | |
| dextrose: | polydextrose: | fructose: | | |
| 1-500 g/kg | 10-1000 g/kg | 1-1000 g/kg | | |
| polydextrose: | sucrose: | maltodextrin: | | |
| 1-1000 g/kg | 10-1000 g/kg | 1-1000 g/kg | | |
| polydextrose: | sorbitol: | maltodextrin: | | |
| 10-1000 g/kg | 10-1000 g/kg | 1-1000 g/kg | | |
| polydextrose: | maltitol: | HSH: | | |
| 203.6-296.8 g/kg | 10-1000 g/kg | 226.0-340.0 g/kg | | |
| aspartame: | sorbitol: | polydextrose: | sucrose: | |
| 0.01-1 g/kg | 10-1000 g/kg | 1-1000 g/kg | 10-1000 g/kg | |
| isomalt: | sorbitol: | polydextrose: | sucrose: | |
| 1-500 g/kg | 1-1000 g/kg | 1-1000 g/kg | 10-1000 g/kg | |
| isomalt: | sorbitol: | polydextrose: | fructose: | |
| 1-500 g/kg | 1-100 g/kg | 1-1000 g/kg | 10-1000 g/kg | |
| acesulfame K: | alitame: | | | |
| 0.01-1 g/kg | 0.01-1 g/kg | | | |
| acesulfame K: | aspartame: | | | |
| 0.16-6.2 g/kg | 0.16-6.2 g/kg | | | |
| acesulfame K: | cyclamate: | | | |
| 0.25-0.5 g/kg | 0.01-10 g/kg | | | |
| acesulfame K: | saccharine: | | | |
| 0.01-1 g/kg | 0.1-1 g/kg | | | |
| acesulfame K: | sucrose: | | | |
| 0.01-1 g/kg | 1-100 g/kg | | | |
| acesulfame K: | sucralose: | | | |
| 0.32-0.92 g/kg | 0.10-0.36 g/kg | | | |
| acesulfame K: | fructose: | | | |
| 0.01-1 g/kg | 1-100 g/kg | | | |
| acesulfame K: | isomalt: | | | |
| 0.1-2 g/kg | 144.92-881.3 g/kg | | | |
| acesulfame K: | isomalt: | | | |
| 0.1-2 g/kg | 144.92-881.3 g/kg | | | |
| acesulfame K: | polydextrose: | | | |
| 0.01-1 g/kg | 1-1000 g/kg | | | |
| acesulfame K: | oligofructose: | | | |
| 1-10 g/kg | 1-1000 g/kg | | | |
| acesulfame K: | sucralose: | | | |
| 0.27 g/kg | 0.01-10 g/kg | | | |
| acesulfame K: | sorbitol | | | |
| 0.01-1 g/kg | 280.7-979.0 g/kg | | | |
| acesulfame K: | HSH: | | | |
| 1.5-1.6 g/kg | 796.8-981.0 g/kg | | | |
| acesulfame K: | maltitol: | | | |
| 0.3-0.4 g/kg | 851.0-851.1 g/kg | | | |
| acesulfame K: | alitame: | fructose: | | |
| 0.01-1 g/kg | 0.001-1 g/kg | 0.1-100 g/kg | | |
| acesulfame K: | aspartame: | fructose: | | |
| 0.13-0.20 g/kg | 0.10-0.16 g/kg | 8.0-10.0 g/kg | | |
| acesulfame K: | aspartame: | sucrose: | | |
| 0.12-0.3 g/kg | 0.10-0.3 g/kg | 20.0-225.0 g/kg | | |
| acesulfame K: | aspartame: | cyclamate | | |
| 0.14-0.67 g/kg | 0.14-0.28 g/kg | 0.12-1.2 g/kg | | |
| acesulfame K: | aspartame: | NHDC: | | |
| 0.15-0.18 g/kg | 0.15-0.18 g/kg | 0.002-0.005 g/kg | | |
| acesulfame K: | aspartame: | saccharine: | | |
| 0.06-0.14 g/kg | 0.06-0.14 g/kg | 0.03-0.08 g/kg | | |
| acesulfame K: | aspartame: | alitame: | | |
| 0.01-1 g/kg | 0.01-1 g/kg | 0.001-1 g/kg | | |
| acesulfame K: | aspartame: | inulin: | | |
| 0.01-1 g/kg | 0.01-10 g/kg | 1-1000 g/kg | | |
| acesulfame K: | aspartame: | isomalt: | | |
| 0.01-1 g/kg | 0.01-1 g/kg | 10-1000 g/kg | | |
| acesulfame K: | aspartame: | sucralose: | | |
| 1-10 g/kg | 0.01-1 g/kg | 0.01-1 g/kg | | |
| acesulfame K: | cyclamate: | fructose: | | |
| 0.20-0.35 g/kg | 0.01-1 g/kg | 10-20.0 g/kg | | |
| acesulfame K: | cyclamate: | sucralose: | | |
| 0.1-10 g/kg | 0.1-10 g/kg | 0.01-1 g/kg | | |
| acesulfame K: | isomalt: | oligofructose: | | |
| 0.1-1 g/kg | 72.46-93.5 g/kg | 72.46-93.5 g/kg | | |
| acesulfame K: | sorbitol: | polydextrose: | | |
| 0.1-1 g/kg | 10-1000 g/kg | 10-1000 g/kg | | |
| acesulfame K: | lactitol: | polydextrose: | | |
| 0.1-1 g/kg | 10-1000 g/kg | 10-1000 g/kg | | |
| acesulfame K: | maltitol: | polydextrose: | | |
| 0.1-10 g/kg | 34.0-135.2 g/kg | 33.0-134.4 g/kg | | |
| acesulfame K: | maltitol: | sorbitol: | | |
| 0.1-10 g/kg | 199.8-266.7 g/kg | 176.0-281.3 g/kg | | |
| acesulfame K: | isomalt: | polydextrose: | | |
| 0.65-3.2 g/kg | 33.8-476.0 g/kg | 33.6-315.7 g/kg | | |
| acesulfame K: | isomalt: | inulin: | | |
| 0.1-10 g/kg | 10-1000 g/kg | 10-1000 g/kg | | |
| acesulfame K: | isomalt: | sorbitol: | | |
| 0.1-10 g/kg | 10-1000 g/kg | 10-1000 g/kg | | |
| acesulfame K: | isomalt: | HSH: | | |
| 1-10 g/kg | 10-2000 g/kg | 10-1000 g/kg | | |
| acesulfame K: | sorbitol: | HSH: | | |
| 0.4-1.4 g/kg | 56.3-75.6 g/kg | 10-1000 g/kg | | |
| acesulfame K: | maltitol: | HSH: | | |
| 0.1-1 g/kg | 100-10000 g/kg | 10-1000 g/kg | | |
| acesulfame K: | sorbitol: | xylitol: | | |
| 0.1-1 g/kg | 10-1000 g/kg | 10-1000 g/kg | | |
| acesulfame K: | isomalt: | xylitol: | | |
| 0.1-1 g/kg | 345.0-400.7 g/kg | 40.0-41.0 g/kg | | |
| acesulfame K: | isomalt: | NHDC: | | |
| 0.1-1 g/kg | 100-10000 g/kg | 0.001-1 g/kg | | |
| acesulfame K: | sucrose: | fructose: | | |
| 1-10 g/kg | 10-1000 g/kg | 1-1000 g/kg | | |
| acesulfame K: | sucrose: | maltodextrin: | | |
| 1-10 g/kg | 10-1000 g/kg | 10-1000 g/kg | | |
| acesulfame K: | sucrose: | polydextrose: | | |
| 1-10 g/kg | 10-1000 g/kg | 10-1000 g/kg | | |
| acesulfame K: | aspartam: | sorbitol: | polydextrose: | |
| 0.25-0.55 g/kg | 0.25-0.55 g/kg | 10-1000 g/kg | 159.0-160.0 g/kg | |
| acesulfame K: | aspartame: | saccharine: | fructose: | |
| 0.01-1 g/kg | 0.01-1 g/kg | 0.001-10 g/kg | 1-1000 g/kg | |
| acesulfame K: | aspartame: | saccharine: | sucrose: | |
| 0.06-0.09 g/kg | 0.06-0.09 g/kg | 0.001-10 g/kg | 1-1000 g/kg | |
| acesulfame K: | aspartame: | cyclamate: | fructose: | |
| 0.01-1 g/kg | 0.01-1 g/kg | 0.01-1 g/kg | 1-1000 g/kg | |
| acesulfame K: | aspartame: | isomalt: | polydextrose: | |
| 0.01-1 g/kg | 0.01-1 g/kg | 0.01-1 g/kg | 1-1000 g/kg | |
| acesulfame K: | aspartame: | sucrose: | fructose: | |
| 0.01-1 g/kg | 0.01-1 g/kg | 10-1000 g/kg | 10-1000 g/kg | |
| acesulfame K: | isomalt: | sorbitol: | fructose: | |
| 0.01-1 g/kg | 10-1000 g/kg | 10-1000 g/kg | 10-1000 g/kg | |
| acesulfame K: | isomalt: | sorbitol: | polydextrose: | |
| 0.7-1.2 g/kg | 63.8-344.6 g/kg | 31.9-51.7 g/kg | 53.9-170.2 g/kg | |
| acesulfame K: | isomalt: | lactitol: | polydextrose: | |
| 0.1-1 g/kg | 10-1000 g/kg | 10-1000. g/kg | 1-1000 g/kg | |
| acesulfame K: | isomalt: | polydextrose: | inulin: | |
| 1-10 g/kg | 10-1000 g/kg | 10-1000 g/kg | 1-1000 g/kg | |
| acesulfame K: | isomalt: | maltitol: | xylitol: | |
| 0.01-1 g/kg | 10-1000 g/kg | 10-1000 | 10-1000 g/kg | |
| acesulfame K: | dextrose: | polydextrose: | fructose: | |
| 1-10 g/kg | 10-1000 g/kg | 10-1000 g/kg | 10-1000 g/kg | |
| acesulfame K: | polydextrose: | sucrose: | maltodextrin: | |
| 1-10 g/kg | 10-1000 g/kg | 10-1000 g/kg | 10-1000 g/kg | |
| acesulfame K: | polydextrose: | sorbitol: | maltodextrin: | |
| 1-10 g/kg | 10-1000 g/kg | 10-1000 g/kg | 10-1000 g/kg | |
| acesulfame K: | polydextrose: | maltitol: | HSH: | |
| 1.8-8.3 g/kg | 203.6-296.8 g/kg | 10-1000 g/kg | 226.0-340.0 g/kg | |
| acesulfame K: | aspartame: | sorbitol: | polydextrose: | sucrose: |
| 0.01-1 g/kg | 0.01-1 g/kg | 10-1000 g/kg | 10-1000 g/kg | 10-1000 g/kg |
| acesulfame K: | isomalt: | sorbitol: | polydextrose: | sucrose: |
| 0.1-10 g/kg | 10-1000 g/kg | 10-1000 g/kg | 10-1000 g/kg | 10-1000 g/kg |
| acesulfame K: | isomalt: | sorbitol: | polydextrose: | fructose: |
| 0.1-10 g/kg | 10-1000 g/kg | 10-1000 g/kg | 10-1000 g/kg | 10-1000 g/kg |

In other embodiments, the compound of formula (I) as defined above or a stereoisomer or a salt or a hydrate thereof, when used as an additive in a beverage or a drink, may be combined with sweetener(s), e.g. sugar(s), and/or sweetness enhancer(s) as shown in Table 3 below. The combinations in Table 3 are merely exemplary and are not meant to limit the scope of the invention.

**Table 3. Mass ranges - Beverages and Drinks**

| **Additive 1** | **Additive 2 (opt.)** | **Additive 3 (opt.)** | **Additive 4 (opt.)** | **Additive 5 (opt.)** |
|---|---|---|---|---|
| aspartame: | | | | |
| 0.04-0.26 g/l | | | | |
| cyclamate: | | | | |
| 0.28-0.72 g/l | | | | |
| sucrose: | | | | |
| 40.0-70.0 g/l | | | | |
| sucralose: | | | | |
| 0.073-0.77 g/l | | | | |
| neotame: | | | | |
| 0.006-0.007 g/l | | | | |
| alitame: | | | | |
| 0.025-0.030 g/l | | | | |
| aspartame: | sucralose: | | | |
| 0.09-0.13 g/l | 0.01-0.06 g/l | | | |
| aspartame: | cyclamate: | | | |
| 0.04-0.10 g/l | 0.22-0.40 g/l | | | |
| aspartame: | saccharine: | | | |
| 0.09-0.18 g/l | 0.03-0.08 g/l | | | |
| aspartame: | fructose: | | | |
| 0.04-0.13 g/l | 10.0-20.0 g/l | | | |
| aspartame: | sucrose: | | | |
| 0.06-0.16 g/l | 20.0-65.0 g/l | | | |
| aspartame: | NHDC: | | | |
| 0.06-0.13 g/l | 0.0001-1 g/l | | | |
| aspartame: | maltol: | | | |
| 0.13-0.16 g/l | 0.001-1 g/l | | | |
| cyclamate: | sucralose: | | | |
| 0.2-0.4 g/l | 0.03-0.10 g/l | | | |
| cyclamate: | sucrose: | | | |
| 0.09-0.17 g/l | 10.0-35.0 g/l | | | |
| cyclamate: | saccharine: | | | |
| 0.28-0.40 g/l | 0.04-0.09 g/l | | | |
| cyclamate: | neotame: | | | |
| 0.43-0.44 g/l | 0.003-0.005 g/l | | | |
| cyclamate: | maltol: | | | |
| 0.01-1 g/l | 0.001-1 g/l | | | |
| cyclamate: | ethylmaltol: | | | |
| 0.08-0.40 g/l | 0.007-0.020 g/l | | | |
| sucralose: | sucrose: | | | |
| 0.01-1 g/l | 10-1000 g/l | | | |
| aspartame: | saccharine: | cyclamate: | | |
| 0.01-0.07 g/l | 0.02-0.09 g/l | 0.15-0.41 g/l | | |
| aspartame: | saccharine: | sucrose: | | |
| 0.08-0.13 g/l | 0.03-0.05 g/l | 15.0-30.0 g/l | | |
| aspartame: | sucralose: | sucrose: | | |
| 0.01-1 g/l | 0.001-1 g/l | 10-1000 g/l | | |
| cyclamate: | sucralose: | sucrose: | | |
| 0.01-1 g/l | 0.001-1 g/l | 10-1000 g/l | | |
| cyclamate: | saccharine: | sucralose: | | |
| 0.01-1 g/l | 0.03-0.09 g/l | 0.04-0.08 g/l | | |
| cyclamate: | neotame: | sucrose: | | |
| 0.01-1 g/l | 0.0001-1 g/l | 10-1000 g/l | | |
| aspartame: | cyclamate: | saccharine: | sucralose: | |
| 0.01-1 g/l | 0.01-1 g/l | 0.001-1 g/l | 0.001-1 g/l | |
| acesulfame K: | | | | |
| 0.20-0.45 g/l | | | | |
| acesulfame K: | aspartame: | | | |
| 0.07-0.28 g/l | 0.04-0.26 g/l | | | |
| acesulfame K: | cyclamate: | | | |
| 0.07-0.20 g/l | 0.28-0.72 g/l | | | |
| acesulfame K: | sucrose: | | | |
| 0.15-0.32 g/l | 40.0-70.0 g/l | | | |
| acesulfame K: | sucralose: | | | |
| 0.10-0.25 g/l | 0.073-0.77 g/l | | | |
| acesulfame K: | neotame: | | | |
| 0.15-0.24 g/l | 0.006-0.007 g/l | | | |
| acesulfame K: | alitame: | | | |
| 0.15-0.20 g/l | 0.025-0.030 g/l | | | |
| acesulfame K: | aspartame: | sucralose: | | |
| 0.09-0.19 g/l | 0.09-0.13 g/l | 0.01-0.06 g/l | | |
| acesulfame K: | aspartame: | cyclamate: | | |
| 0.04-0.10 g/l | 0.04-0.10 g/l | 0.22-0.40 g/l | | |
| acesulfame K: | aspartame: | saccharine: | | |
| 0.06-0.14 g/l | 0.09-0.18 g/l | 0.03-0.08 g/l | | |
| acesulfame K: | aspartame: | fructose: | | |
| 0.12-0.13 g/l | 0.04-0.13 g/l | 10.0-20.0 g/l | | |
| acesulfame K: | aspartame: | sucrose: | | |
| 0.06-0.13 g/l | 0.06-0.16 g/l | 20.0-65.0 g/l | | |
| acesulfame K: | aspartame: | NHDC: | | |
| 0.08-0.15 g/l | 0.06-0.13 g/l | 0.0001-1 g/l | | |
| acesulfame K: | aspartame: | maltol: | | |
| 0.13-0.16 g/l | 0.13-0.16 g/l | 0.0001-1 g/l | | |
| acesulfame K: | cyclamate: | sucralose: | | |
| 0.09-0.13 g/l | 0.2-0.4 g/l | 0.03-0.10 g/l | | |
| acesulfame K: | cyclamate: | sucrose: | | |
| 0.07-0.09 g/l | 0.09-0.17 g/l | 10.0-35.0 g/l | | |
| acesulfame K: | cyclamate: | saccharine: | | |
| 0.05-0.26 g/l | 0.28-0.40 g/l | 0.04-0.09 g/l | | |
| acesulfame K: | cyclamate: | neotame: | | |
| 0.21-0.29 g/l | 0.43-0.44 g/l | 0.003-0.005 g/l | | |
| acesulfame K: | cyclamate: | maltol: | | |
| 0.01-1 g/l | 0.01-10 g/l | 0.001-1 g/l | | |
| acesulfame K: | cyclamate: | ethylmaltol: | | |
| 0.08-0.20 g/l | 0.08-0.40 g/l | 0.007-0.020 g/l | | |
| acesulfame K: | sucralose: | sucrose: | | |
| 0.01-1 g/l | 0.01-1 g/l | 10-1000 g/l | | |
| acesulfame K: | aspartame: | saccharine: | cyclamate: | |
| 0.01-0.07 g/l | 0.01-0.07 g/l | 0.02-0.09 g/l | 0.15-0.41 g/l | |
| acesulfame K: | aspartame: | saccharine: | sucrose: | |
| 0.08-0.13 g/l | 0.08-0.13 g/l | 0.03-0.05 g/l | 15.0-30.0 g/ | |
| acesulfame K: | aspartame: | sucralose: | sucrose: | |
| 0.01-1 g/l | 0.01-1 g/l | 0.001-1 g/l | 10-1000 g/l | |
| acesulfame K: | cyclamate: | sucralose: | sucrose: | |
| 0.01-1 g/l | 0.01-1 g/l | 0.001-1 g/l | 10-1000 g/l | |
| acesulfame K: | cyclamate: | saccharine: | sucralose: | |
| 0.015-0.1 g/l | 0.01-1 g/l | 0.03-0.09 g/l | 0.04-0.08 g/l | |
| acesulfame K: | cyclamate: | neotame: | sucrose: | |
| 0.01-1 g/l | 0.01-1 g/l | 0.00001-1 g/l | 10-1000 g/l | |
| acesulfame K: | aspartame: | cyclamate: | saccharine: | sucralose: |
| 0.01-1 g/l | 0.01-1 g/l | 0.01-10 g/l | .0001-1 g/l | 0.001-1 g/l |

In other embodiments, the compound of formula (I) as defined above or a stereoisomer or a salt or a hydrate thereof when used as table top sweetener, may be combined with sweetener(s), e.g. sugar(s) and/or sweetness enhancer(s) as shown in Table 4 below. The combinations in Table 4 are merely exemplary and are not meant to limit the scope of the invention.

**Table 4. Mass ranges - Table Top Sweeteners**

| **Additive 1** | **Additive 2 (opt.)** | **Additive 3 (opt.)** | **Additive 4 (opt.)** |
|---|---|---|---|
| acesulfame K: | | | |
| 6.7-134.0 g/kg | | | |
| aspartame: | | | |
| 5.0-22.0 g/kg | | | |
| cyclamate: | | | |
| 28.0-120.0 g/kg | | | |
| lactose: | | | |
| 100-900.0 g/kg | | | |
| sucralose: | | | |
| 9.6-20.0 g/kg | | | |
| saccharin: | cyclamate: | | |
| 1-100 g/kg | 10-1000 g/kg | | |
| saccharin: | maltodextrin: | | |
| 13.0-20.0 g/kg | 100-10000 g/kg | | |
| aspartame: | dextrose | | |
| 1-100 g/kg | 100-10000 g/kg | | |
| sucralose: | dextrose | | |
| 3.4-5.0 g/kg | 984.1-982.5 g/kg | | |
| aspartame: | NHDC: | lactose: | |
| 1-100 g/kg | 1-100 g/kg | 10-10000 g/kg | |
| aspartame: | cyclamate: | lactose: | |
| 1-100 g/kg | 10-1000 g/kg | 10-10000 g/kg | |
| aspartame: | sucralose: | dextrose: | |
| 1-100 g/kg | 1.7-2.5 g/kg | 979.6-978.8 g/kg | |
| aspartame: | cyclamate: | maltodextrin: | |
| 1-100 g/kg | 1-1000 g/kg | 10-10000 g/kg | |
| saccharin: | cyclamate: | maltodextrin: | |
| 3.3-5.0 g/kg | 28.0-57.0 g/kg | 932.2-974.0 g/kg | |
| acesulfame K: | aspartame: | | |
| 24.0-110.0 g/kg | 5.0-22.0 g/kg | | |
| acesulfame K: | cyclamate: | | |
| 1-100 g/kg | 10-1000 g/kg | | |
| acesulfame K: | lactose: | | |
| 10-1000 g/kg | 100-10000 g/kg | | |
| acesulfame K: | sucralose: | | |
| 12.0-24.0 g/kg | 9.6-20.0 g/kg | | |
| acesulfame K: | saccharin: | cyclamate: | |
| 1-100 g/kg | 1-100 g/kg | 10-1000 g/kg | |
| acesulfame K: | saccharin: | maltodextrin: | |
| 10.0-13.0 g/kg | 13.0-20.0 g/kg | 970-974 g/kg | |
| acesulfame K: | aspartame: | dextrose | |
| 1-100 g/kg | 1-100 g/kg | 10-10000 g/kg | |
| acesulfame K: | sucralose: | dextrose | |
| 1-100 g/kg | 3.4-5.0 g/kg | 984.1-982.5 g/kg | |
| acesulfame K: | aspartame: | NHDC: | lactose: |
| 1-100 g/kg | 1-100 g/kg | 0.1-100 g/kg | 10-10000 g/kg |
| acesulfame K: | aspartame: | cyclamate: | lactose: |
| 1-100 g/kg | 1-100 g/kg | 10-1000 g/kg | 10-10000 g/kg |
| acesulfame K: | aspartame: | sucralose: | dextrose: |
| 1-100 g/kg | 1-100 g/kg | 1.7-2.5 g/kg | 100-10000 g/kg |
| acesulfame K: | aspartame: | 10-1000 g/kg | maltodextrin: |
| 1-100 g/kg | 1-100 g/kg | | 10-10000 g/kg |
| acesulfame K: | saccharin: | cyclamate: | maltodextrin: |
| 7.2-10.0 g/kg | 3.3-5.0 g/kg | 28.0-57.0 g/kg | 10-10000 g/kg |

In other embodiments, when the compound of formula (I) as defined above or a stereoisomer or a salt or a hydrate thereof is used with comestible goods, the compound of formula (I) as defined above or a stereoisomer or a salt or a hydrate thereof may be combined with one or more, e.g., two or more or three or more, sweetener(s), e.g. sugar(s), and/or sweetness enhancer(s) that are known in the art but are not listed above in Tables 2-4. It should be understood that the compound of formula (I) as defined above or a stereoisomer or a salt or a hydrate thereof may, in some embodiments, be combined with at least one of any of the sweetener(s), e.g. sugar(s), and/or sweetness enhancer(s) that are listed above in Tables 2-4 and/or with any other sweetener(s), e.g. sugar(s), and/or sweetness enhancer(s) that are known in the art but are not listed in Tables 2-4.

### Examples

The following examples as well as the accompanying figures provide illustrative embodiments of the inventions described and claimed herein. These examples are not intended to provide any limitation on the scope of the invented subject-matter.

### Example 1: In vitro identification of the compound of formula (I) as a sweet taste modulating substance - Detection of sweetness enhancer activity of the compound of formula (Ia) in a recombinant human taste receptor T1R2/T1R3 dependent cell based assay

The compound of formula (Ia) has been identified in the screening phase as a sweet taste modulating substance by cell based assay analysis, acting as a fructose enhancer *in vitro*.

### 1.1 Description of the general method

In wild type taste cells, e.g. in human taste buds, signal transduction is accomplished by the G-proteins gustducin and/or by G-Proteins of the G alpha-i type. Encountering sweet ligands, the heterodimeric human taste receptor T1R2/T1R3 reacts with induction of second messenger molecules, namely either the increase of the cAMP level in response to most sugars or the increase of the calcium level in response to most artificial sweeteners (Margolskee, 2002; J. Biol Chem. 277, 1-4).

To analyze the function and activity of a test compound, e.g. of compound of formula (Ia), the heterodimeric hT1R2/hT1R3 sweet taste receptor is utilized in a calcium dependent cell based assay. T1R type taste receptors are transfected with the multicistronic plasmid vector pTrix-Eb-R2R3 in a HEK293 cell line stably expressing the promiscuous mouse G-alpha-15 G-protein.

For the generation of stable cell lines a multicistronic expression unit using human taste receptor sequences is used. In a tricistronic expression unit of the expression vector pTrix-Eb-R2R3 is under the control of the human elongation factor 1 alpha promoter. Using standard cloning techniques the cDNA for the receptors ht1R2 and ht1R3 and the cDNA for the blasticidin S deaminase gene is cloned. To enable the translation initiation of each gene of this tricistronic unit two EMC-virus derived internal ribosomal entry sites (IRES - also termed Cap-independent translation enhancer (CITE)) are inserted (Jackson et al., Trends Biochem Sci (1990) 15, 477-83; Jang et al., J Virol (1988) 62, 2636-43)

The tricistronic expression unit is terminated by a simian virus 40 polyadenylation signal sequence. This composition permits the simultaneous expression of all three genes under the control of only one promoter. In contrast to monocistronic transcription units, which integrate independently from each other into different chromosomal locations during the process of stable cell line development, the tricistronic transcription unit integrates all containing genes in one and the same chromosomal locus. Due to the alignment of the genes, the blasticidin S deaminase gene is only transcribed in case a full length transcription takes place. Moreover the polarity of multicistronic transcription units (Moser, S. et al., Biotechnol Prog (2000) 16, 724-35) leads probably to a balanced stoichiometry of the receptor genes and their expression rates in the range of 1:0.7 up to 1:1 for the first two positions whereas the blasticidin S deaminase gene compared to the receptor genes in the third position is expressed to a lesser extend.

Assuming that for the functional heterodimeric receptor ht1R2/ht1R3 a 1:1 stoichiometry is needed the lesser polarity effects for the receptor genes promote the desired stoichiometry whereas the reduced expression of the deaminase promotes an integration locus with enhanced transcriptional activity. Generation of stable hT1R2/hT1R3 expressing cells are performed by culturing the transfected cells in the presence of blasticidine.

For measurement of human T1R2/T1R3 taste receptor dependent activity a control cell line without sweet receptor based on HEK 293 and a cell line expressing the sweet receptor hT1R2/hT1R3 based on HEK 293, respectively, are seeded into assay plates and labelled with the calcium sensitive fluorescence dye Fluo4-AM in a culture medium. The potential modulators, e.g. the test compound, are added to the control cell line and to the cell line expressing the sweet receptor hT1R2/hT1R3, respectively.

Response to different concentrations of the modulator in the presence of fructose is recorded as Fluo4-AM fluorescence increase initiated through the hT1R2/hT1R3 dependent increase of the second messenger calcium. The applied fructose concentration is chosen from the results of preexaminations showing that fructose, in these concentrations, is barely activating the sweet taste receptors within this cell based assay. Thus a sweetness enhancing property of a test compound is detectable in the presence of the sweetener fructose.

### 1.2 Preparation of the cell lines and cell culture

A stable cell line based on HEK293 was termed HEKα15#17 served as control for functional Gα-protein and a stable cell line HEKGα15#17R2R3b#8 C6 served as cell line expressing the sweet receptor hT1R2/hT1R3 based on HEKGα15#17.

The cells were cultured in DMEM/HG and 10% FCS Gold, 4mM L-Glutamin, 1,3mg/ml Geneticin (HEKGα15#17) or the same medium additionally containing 6µg/ml Blasticidin (HEKGα15#17R2R3b#8 C6). The medium for the assay was DMEM/HG (high glucose, 4.5 g/l glucose, Life Technologies) and 10% FCS Gold, 4mM L-Glutamin (HEKGα15#17) or DMEM/LG (low glucose, 1 g/l glucose; Life Technologies) and 10% FCS Gold (HEKGα15#17R2R3b#8C6), respectively.

### 1.3 Evaluation of the sweet receptor modulating activity of the compound of formula (Ia)

In order to evaluate the sweet receptor modulating activity of the compound of formula (Ia), compound of formula (Ia) was added to the cultures in a concentration of 25 µM in a volume of 50µl. Controls were KH-buffer (Krebs HEPES-buffer: 118mM NaCl, 4.7mM KCl, 1.2mM MgSO₄, 1.2mM KH₂PO₄, 4.2mM NaHCO₃, 1.3mM CaCl₂, 10mM Hepes, pH 7.4) either without or with sweetness enhancer. As natural and artificial sweeteners either fructose (20mM), acesulfame K (30mM) or sodium cyclamate (30 mM) were used.

Further controls for function of G-protein and receptor were isoproterenol (1µM, Sigma Aldrich), ionomycin (1µM, Calbiochem) and ATP (1µM, Applichem).

DMSO was diluted in KH-buffer/fructose (100mM stock solution = 50µM final test concentration = 1:400 dilution).

### 1.4 In vitro cell based assay procedure

For measurement of human T1R2/T1R3 taste receptor dependent activity, on day 1 of the test procedure the HEKα15#17 cells (control cell line without sweet receptor) and the HEKGα15#17R2R3b#8 C6 cells (cell line expressing the sweet receptor hT1R2/hT1R3), respectively, were transferred to low-glucose medium.

On day 2 the cells were seeded into assay plates at a density of 25,000 cells/well. Assay plates for fluorescence measurement (black clear bottom 96-cavity plates, Greiner Bio-One) were coated with poly-D-lysine. For direct comparison both cell lines (control cell line without sweet receptor and cell line expressing the sweet receptor hT1R2/hT1R3) were seeded into different cavities of the same assay plate. The plate was placed into the incubator for 48 hours at 37°C, 5% CO₂, 100% relative humidity.

On day 4 the calcium assay was performed. For this assay, the cells were marked with Fluo4-AM in medium and KH-buffer. To each cavity 100µl KH-buffer/250µM sulfinpyrazone/4µM Fluo4-AM were added gently to 100µl medium (final concentration of Fluo4-AM = 2µM, stock solution of Fluo4-AM 1mM in DMSO, excitation at 485 nm, emission at 520 nm, Fisher Scientific, 58239 Schwerte, Germany).

The plate was placed into the incubator for 1 hour. In the mean time the test substances ("ligands") were prepared in a separate plate (5 times concentrated, 150 µl/cavity, and then automatically 50µl/cavity were transferred into the screening cell plate by pipetting robot (FLEX-station, Molecular Devices, Sunnyvale, California).

The culture medium was carefully removed and 200µl KH-buffer/250µM sulfinpyrazone per cavity was added slowly. Cells were left in the FLEX-station for 20 minutes for adaptation, followed by measurement at 37°C.

### 1.5 Results of the in vitro cell based assay

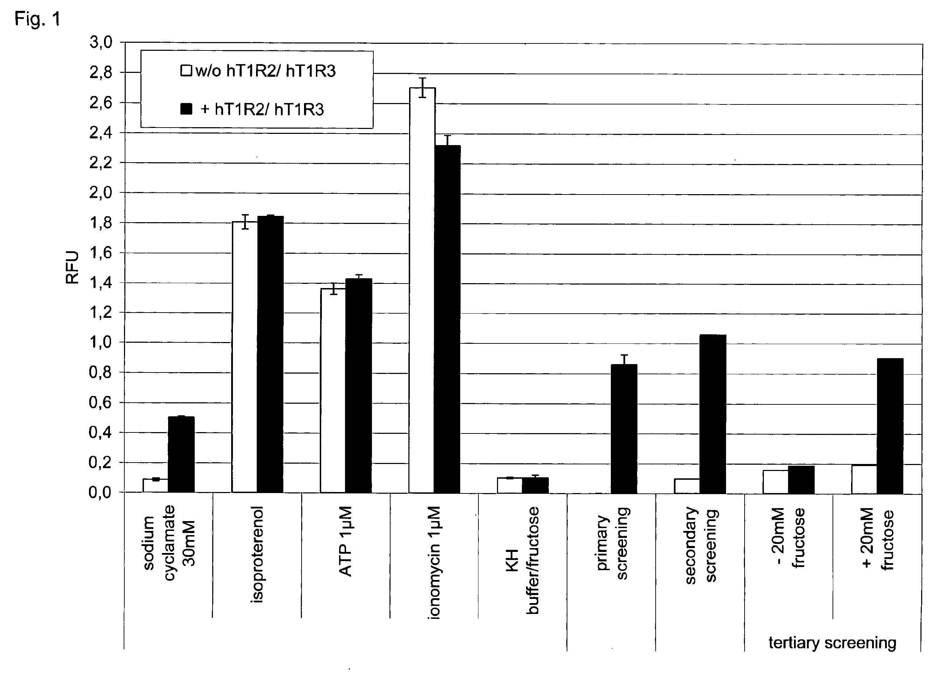

The results of this assay are shown in the bar diagram of Figure 1. In the bar diagram the grey bars (control--without hT1R2/hT1R3) indicate the measured Fluo4-AM fluorescence increase of the respective test compound added to the control cell line, the black bars (assay--with hT1R2/hT1R3) indicate the measured Fluo4-AM fluorescence increase of the respective test compound added to the cell line expressing the sweet receptor hT1R2/hT1R3.

The first bar (sodium cyclamate) shows that the control sweetener sodium cyclamate does not activate the control cell line, but activates the cell line expressing the sweet receptor hT1R2/hT1R3. Consequently, this bar shows that sodium cyclamate is a sweetener.

The second, third and fourth bars (isoproterenol, ATP and ionomycin) show that the respective control compounds activate both the control and the cell line expressing the sweet receptor hT1R2/hT1R3 and confirm the function of the G-protein and receptor.

The fifth bar (KH buffer/fructose) represents the result of a screening, in which fructose was added in a concentration in that it does neither activate the control cell line, nor the cell line expressing the sweet receptor hT1R2/hT1R3.

The sixth bar represents the result of the primary screening which was carried out with receptor carrying cells only and shows a strong cellular activation. The seventh bar (secondary screening) shows that the compound of formula (Ia) in the presence of fructose does not activate the control cell line but activates the cell line expressing the sweet receptor hT1R2/hT1R3. Consequently, the compound of formula (Ia) is a sweetener and/or a fructose enhancer.

The eighth bar (compound of formula (Ia) without fructose) shows that the compound of formula (Ia) does neither activate the control cell line, nor the cell line expressing the sweet receptor hT1R2/hT1R3. Consequently, the compound of formula (aI) does not serve as a sweetener at the indicated concentration.

The ninth bar (compound of formula (Ia) in the presence of fructose) shows that the compound of formula (Ia) does not activate the control cell line, but activates the cell line expressing the sweet receptor hT1R2/hT1R3 and enhances the activity of fructose (cf. also the fifth bar).

The inserted graph shows that the selective stimulation of receptor carrying cells by compound of formula (Ia) is verified by measuring the time response in the cell assay over a period of 78 seconds.

Consequently, the assay shows that the compound of formula (Ia) is, at the indicated concentration, a fructose enhancer.

### Example 2: Taste and spit assay with compound of formula (Ia)

The taste of a sample of compound of formula (Ia) with regard to sweetness and fructose enhancing features was assessed by using a panel of trained sensory evaluators experienced in the sweet taste estimation procedure. 5 individuals were asked to taste the quality of single samples of 10 ml volume.

### 2.1 General procedure

Panelists were asked to take a sample of the liquid to be assessed (20 µM test substance compound of formula (Ia) in 0.5 % ethanol) into the mouth and after some time allowed for taste perception to spit the sample out completely. Subsequently, the panelists were asked to rinse their mouth well with water or black tea to reduce any potential carry over effects. The tasting of a sample could be repeated if required.

### 2.2 Taste and spit phase I - Qualitative Assessment

In a first descriptive test 5 individuals were asked to taste the quality of single samples of 10 ml volume (maximum 3 subsequent samples). The samples were served at ambient temperature. The individuals of the taste panel were asked to answer the following questions with regard to the quality of taste: 1) does the sample taste sweet?, 2) is there another taste detectable (bitter, sour, salty, umami)?, 3) is there an off- or aftertaste?, 4) is there anything else remarkable about the perception of the sample?

**Results:** No characteristic taste was identified in the sample containing the compound of formula (Ia) at the indicated concentration. Importantly, no off-taste was detected by the taste panel. These results show that the compound of formula (Ia), beneficially, may be utilized as a sweetness enhancer without contributing off-tastes to the resultant product.

### 2.3 Taste and spit phase II - Assessment of fructose enhancing features

In the next step the panelists were asked to answer questions in a pairwise comparison test to determine the enhancement of sweet taste of the test substance with fructose relative to fructose only. Again, 5 individuals were given samples of 10 ml volume. This time two samples were prepared for direct comparison regarding sweetness. One sample contained fructose (4%) in solvent (0.5 % ethanol) and the other sample additionally contained 20 µM of the test substance compound of formula (Ia). Designation of the samples with A and B was randomized and decoded after the taste procedure. The questions to be answered were: 1) does one sample taste sweeter than the other?, 2) if so, which one?, 3) are there any other differences in the taste between the two samples?

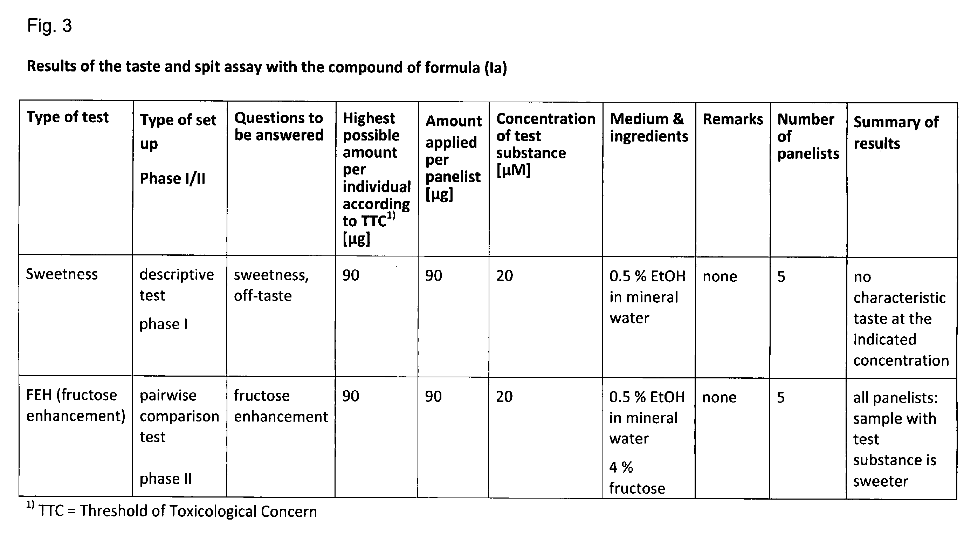

**Results:** The results of the taste and spit assay are based on a qualitative evaluation of the differences between the two samples (cf. Fig. 3). All 5 panelists identified the sample containing 4 % fructose with the compound of formula (Ia) as sweeter than the sample containing 4 % fructose only. Consequently, the compound of formula (Ia) is perceived as a fructose enhancer.

### Example 3: Fermentation, extraction and isolation of the compound of formula (Ia)

The compound of formula (Ia) can be isolated from the actinomycetes strain with the identification reference 01496axxx000004 and the accession number DSM 25420, which has been deposited on November 30, 2011, at the DSMZ-Deutsche Sammlung von Mikroorganismen und Zellkulturen GmbH, Inhoffenstr. 7 B, 38124 Braunschweig, Germany, by the AnalytiCon Discovery GmbH, Hermannswerder Haus 17, 14473 Potsdam, Germany according to the procedure described below.

The taxonomy of strain was determined at the DSMZ by 16S rDNA analysis. The result showed the closest match with *Streptomyces roseolilacinus* (98.9%, binary 98.8%).

### 3.1 Fermentation

### 3.1.1 Pre-culture

The culture is transferred from an agar plate with ISP3 medium into a static medium containing the ingredients 1 to 4, 5 and 6 mentioned below at 30°C for at least 7 days. The volume of the pre-culture is 125 ml in a 1000ml laboratory glass.

| | **Ingredient** | **Supplier** | **[g/l]** |
|---|---|---|---|
| 1 | Glucose | Fluka 49150 | 3.0 |
| 2 | Starch | Roth 4701 | 10.0 |
| 3 | Soy flour, defatted | Hensel | 4.0 |
| 4 | Yeast-Extract | Difco 212720 | 1.0 |
| 5 | pH - Value | | **pH 7.3** |
| 6 | Calcium carbonate | Merck 21060 | 1.0 |
| 7 | Agar agar | Roth 5210 | 2.75 |

### 3.1.2 Main culture

125 ml of the pre-culture are transferred into 10 1 of a liquid medium containing the ingredients 1 to 5 and 7 mentioned below.

| | **Ingredient** | **Supplier** | **[g/l]** | **[g/10l]** |
|---|---|---|---|---|
| 1 | Mannitol | Fluka 63565 | 16.0 | 160.0 |
| 2 | Glycerine 87% | Fluka 49781 | 8.0 | 80.0 |
| 3 | Cotton Seed Flour | Sigma C-4898 | 3.0 | 30.0 |
| 4 | Soy peptone | Sigma P0521 | 7.5 | 75.0 |
| 5 | Sodium chloride | n.n. | 1.0 | 10.0 |
| 6 | **pH-Value** | | **pH 7.0** | **pH 7.0** |
| 7 | Calcium chloride | Fluka 21060 | 1.0 | 10.0 |

### 3.2 Extraction

400 ml of HP20 are added to the fermentation broth and stirred for 45 min. The harvested culture broth with HP20 is centrifuged at 6000 x g for 15 min to remove solid components (biomass+ HP20). The supernatant is removed, decontaminated and discarded. The obtained biomass-/XAD mixture is transferred with acetone into an extraction vessel (glass or polypropylene).

The first extraction is performed with acetone. The biomass-/HP20-pellets are covered with approx. 2 cm acetone and completely soaked. The vessel is well shaken. The mixture is left standing overnight. Subsequently, the mixture is placed for 10 min in an ultra sonic bath and afterwards placed for 30 min at 110-120 rpm on a shaker. Finally, the solution is filtrated (paper). The second extraction is performed according to the procedure described before. Afterwards both extracts are combined.

### 3.3 Isolation of the compound of formula (Ia)

The compound of formula (I) is isolated from the combined extracts using MPLC (medium pressure liquid chromatography).

| | | | | | |
|---|---|---|---|---|---|
| MPLC System | Kronlab GmbH | | | | |
| Data System | Prepcon 4.47 | | | | |
| Stationary Phase | Polygoprep 60-50 RP-18 (Macherey & Nagel) | | | | |
| Mobile Phase | A: dest. water | | | | |
| | B: Methanol (p.a.) | | | | |
| | C: Isopropanol | | | | |

| Gradient | Time [min] | Flow rate [ml/min.] | % A | % B | % C |
|---|---|---|---|---|---|
| | 0.0 | 100 | 100 | 0 | 0 |
| | 5.0 | 100 | 100 | 0 | 0 |
| | 5.1 | 130 | 100 | 0 | 0 |
| | 10.0 | 130 | 100 | 0 | 0 |
| | 10.1 | 100 | 80 | 20 | 0 |
| **Individual** | **18.0** | **100** | **32** | **68** | **0** |
| **Gradient** | **51.0** | **100** | **10** | **80** | **0** |
| | 61.0 | 100 | 10 | 90 | 0 |
| | 61.1 | 150 | 0 | 100 | 0 |
| | 66.0 | 150 | 0 | 100 | 0 |
| | 66.1 | 30 | 0 | 0 | 100 |
| | 70.0 | 30 | 0 | 0 | 100 |
| | 70.1 | 75 | 0 | 0 | 100 |
| | 74.0 | 75 | 0 | 0 | 100 |

The second fraction (collected from 12 to 16 min) contains the novel compound of formula (Ia) with a purity of 79% (ELSD, evaporative light scattering detection) confirmed by NMR.

### 3.4 Spectral and physical data of the compound of formula (Ia)

The compound of formula (Ia) has been characterized by the following data:
Molecular formula: C₁₈H₃₂N₄O₉
Molecular weight [g/mol]: 448.47
Purity (LC/MS-ELSD): 79%

### Structure:

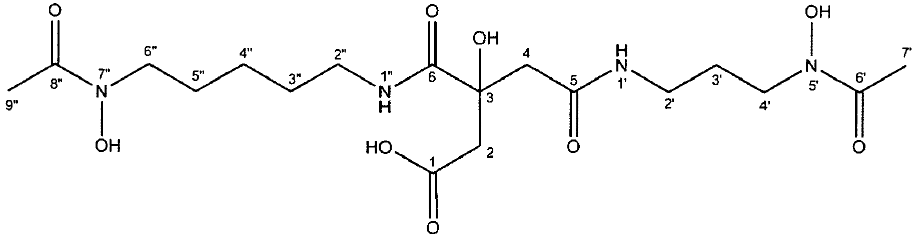

### LC/MS Assignment:

| **(+)-ESI** | | **(-)-ESI** | |
|---|---|---|---|
| m/z | Interpretation | m/z | Interpretation |
| | [M+Na]⁺ | 447 | [M-H]⁻ |
| 449 | [M+H]⁺ | | [M+HCOO]⁻ |

Assignment of the ¹H- and ¹³C-NMR-Signals (based on HH-COSY, HSQC, and HMBC experiments)¹:

| **Position** | **δ_{C} [ppm]** | **δ_{H} [ppm]** | **J [Hz]/ (INT)** | **HMBC (H -> C)** |
|---|---|---|---|---|
| 1 | 177.1 *s* | - | - | - |
| 2 | 43.6 *t* | 2.64 *d* | 15.6 | 1,3,4,6 |
| | | 2.43 *d* | 15.6 | 1,3,4,6 |
| 3 | 74.5 *s* | - | - | - |
| 4 | 44.5 *t* | 2.65 *d* | 14.2 | 2, 3, 5, 6 |
| | | 2.49 *d* | 14.2 | 2, 3, 5, 6 |
| 5 | 171.5 *s* | - | - | - |
| 6 | 175.9 *s* | - | - | - |
| 1' | - | - | - | - |
| 2' | 36.7 *t* | 3.17 *m* | (2H) | 5 |
| 3' | 26.7 *t* | 1.77 *m* | (2H) | 2', 4' |
| 4' | 45.6 *t* | 3.64 *m* | (2H) | 6' |
| 5' | - | - | - | - |
| 6' | 172.5 *s* | - | - | - |
| 7' | 19.2 *q* | 2.10 *s* | (3H) | 6' |
| 1" | - | - | - | - |
| 2" | 38.9 *t* | 3.22 *m* | (2H) | 6 |
| | | 3.15 *m* | | |
| 3" | 29.0 *t* | 1.53 *m* | (2H) | 2", 4", 5" |
| 4" | 23.8 *t* | 1.43 *m* | (2H) | - |
| 5" | 26.4 *t* | 1.62 *m* | (2H) | 6" |
| 6" | 47.5 *t* | 3.62 *m* | (2H) | 8" |
| | | 3.56 *m* | | |
| 7" | - | - | - | - |
| 8" | 172.5 *s* | - | - | - |
| 9" | 19.2 *q* | 2.10 *s* | (3H) | 8" |

| | | | | |
|---|---|---|---|---|
| 1 500 MHz Bruker-NMR in *d*₄-Methanol (δ_{C} = 48.5 ppm; δ_{H} = 3.3 ppm) | | | | |

### Example 4: Assessment of the stability of the compound of formula (Ia)

The stability of the compound of formula (Ia) was tested at 30°C at three different pH-values. The test ran over 8 weeks with the compound of formula (Ia) placed in temperature controlled ovens. Samples were taken according to the following scheme: Days 0, 3, 6, 10, 14, 21, and 28 followed by weeks 6 and 8. All samples were analyzed by HPLC. Reference samples for days 3, 6, 10, 14, 21, and 28 plus weeks 6 and 8 were kept at -20°C and analyzed together with the samples at 30°C.

### 4.1 Methods

Each individual sample of the compound of formula (Ia) contained 18µg in 200µl buffer (0.2 mM). All samples were prepared in 1.5 ml HPLC vials at the same day. The buffer composition is summarized in the table below. Samples for day 0 were immediately analyzed and all other samples placed in ovens. Reference samples were immediately frozen at -20°C.

**Table: Composition of buffers**

| | pH = 3.0 | pH = 4.5 | pH = 6.5 |
|---|---|---|---|
| Salt | Na-Citrate | Na-Citrate | NaH₂PO₄ + Na₂HPO₄ (1:1) |
| Concentration of salt | 1g/l | 1g/l | 1g/l |
| pH adjusted with | HCl/NaOH | HCl/NaOH | H₃PO₄/NaOH |

One reference sample per pH-value was used. Samples from the oven were taken and reference samples were added after thawing. The set of samples at 30° with reference sample was grouped into the HPLC autosampler to minimize the time gap between HPLC-analysis within each set of samples. All samples were analyzed by double injections.

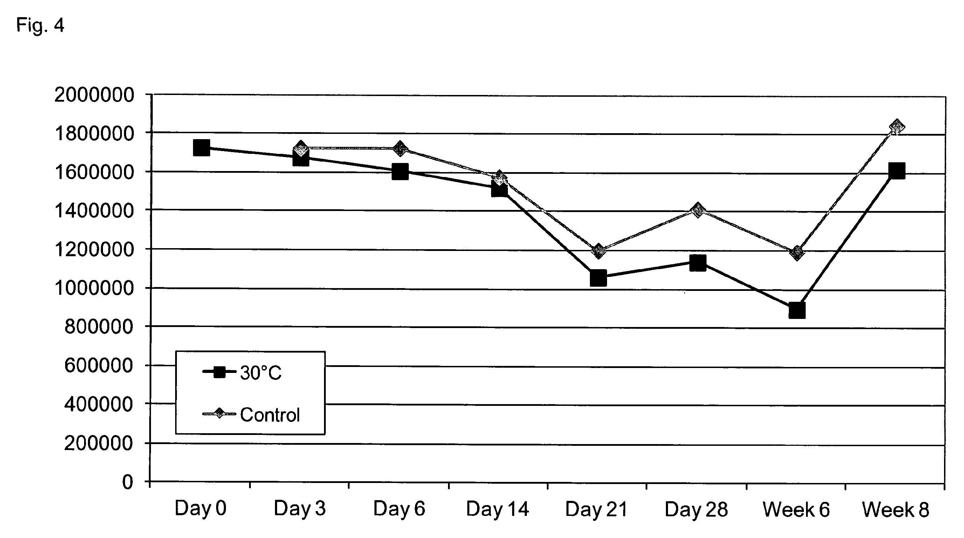

Samples were analyzed by the method shown below. The target peak was manually integrated and mean values of double injections were used to prepare graphs for assessment of the stability (see Figures 4 to 6).

### Analytical LC/MS/ELSD/UV Method (Chemodiversity-Profiling, Standard Method)

| | | | |
|---|---|---|---|
| HPLC System | Merck Hitachi | | |
| Data System | HPLC-Manager D-7000 HSM | | |
| Column | Merck Superspher 60 RP-select B 125x4 mm, 4 µm | | |
| Column oven T | 23°C | | |
| Flow Rate | 1 ml/min | | |
| Detection | UV(220nm) | | |
| Injection Volume | 50 µl | | |
| Mobile Phase: | A: 5 mM ammoniumformiate and 0.1 % formic acid | | |
| | B: acetonitrile / methanol = 1:1, 5 mM ammoniumformiate and 0.1 % formic acid (pH 3) | | |

| Gradient | time [min] | % A | % B |
|---|---|---|---|
| | 0 | 90 | 10 |
| | 15.0 | 40 | 60 |
| | 18.0 | 0 | 100 |
| | 18.1 | 90 | 10 |
| | 21.5 | 90 | 10 |

### 4.2 Results and Discussion

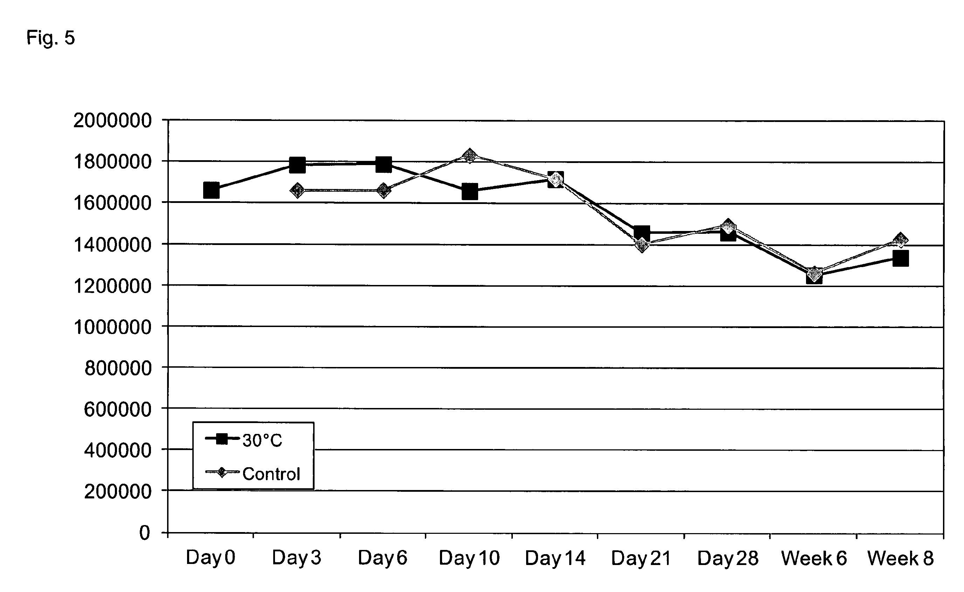

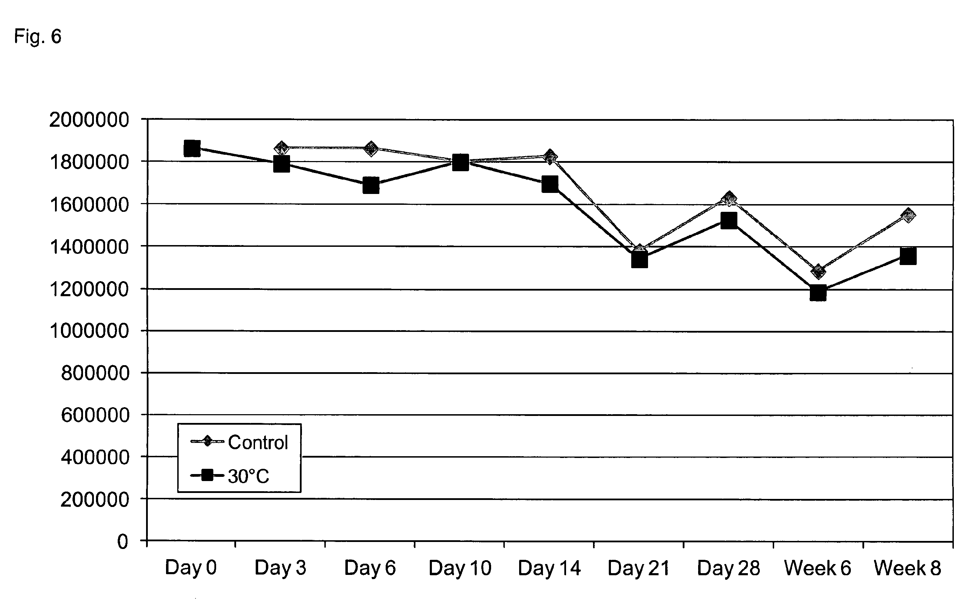

Figure 4 shows that the compound of formula (Ia) was stable during 8 weeks at 30 °C at pH = 3.0. Figure 5 shows that the compound of formula (Ia) was stable during 8 weeks at 30 °C at pH = 4.5. Figure 6 shows that the compound of formula (Ia) was stable during 8 weeks at 30 °C at pH = 6.5.

Consequently, the results show that the compound of formula (Ia) has an excellent temperature and pH stability.

### Example 5: Assessment of the solubility of the compound of formula (Ia)

The solubility properties of the compound of formula (Ia) has been assessed at six selected basic conditions:
- 100mM 100% water with buffer at pH 3.0 and 6.5
- 5mM 100% water with buffer at pH 3.0 and 6.5
- 1mM 100% water with buffer at pH 3.0 and 6.5

### 5.1 Experimental Set-up

### 5.1.1 Sample preparation

20 µmol (8.96 mg) of the compound of formula (Ia) were used for samples preparation. The samples were partitioned into two portions for each buffer system and placed into 1.6 ml glass vials. To each of the vials 100 µl of the respective buffer solution was added to prepare the 100mM samples. All Samples were thoroughly sonicated and vortexed to yield homogenous solutions or suspensions. The resulting set of samples consisted of:
- Compound of formula (Ia)_100mM, buffer 3.0
- Compound of formula (Ia)_100mM, buffer 6.5

In a second step from each of the prior listed samples 7.5µl were pipetted into 1.6 ml glass vials followed by addition of 142.5µl of the respective buffer solution. The resulting set of samples consisted of:
- Compound of formula (Ia)_5mM, buffer 3.0
- Compound of formula (Ia)_5mM, buffer 6.5

In a third step, after sonication and vortexing to yield homogenous solutions or suspensions, 25µl from each of the samples listed above were pipetted into 1.6 ml glass vials followed by addition of 100µl of the respective buffer solution. The resulting set of samples consisted of:
- Compound of formula (Ia)_1mM, buffer 3.0
- Compound of formula (Ia)_1mM, buffer 6.5

### 5.1.2 Determination of solubility

After the preparation of all 6 samples, the entire set was sonicated and vortexed again. The resulting solubility was checked by two persons independently and summarized in table 1.

Afterwards the set of 6 samples was placed into a deep freezer and stored at -22°C for 72h. After that period, the samples were thawed, sonicated, and vortexed. The resulting solubility was checked by two persons independently and summarized in table 2.

In a final step the set of 6 samples was placed into a dark box at 23°C for 72h. After that period, the samples were sonicated, and vortexed. The resulting solubility was checked by two persons independently and summarized in table 3.

### 5.2 Results

The results of the solubility study are shown in Tables 1 to 3 below.

**Table 1: Solubility directly after sample preparation**

| **Sample of compound of formula (Ia)** | **Immediately dissolved** |
|---|---|
| 100 mM, buffer 6.5 | yes |
| 100 mM, buffer 3.0 | yes |
| 5 mM, buffer 6.5 | yes |
| 5 mM, buffer 3.0 | yes |
| 1 mM, buffer 6.5 | yes |
| 1 mM, buffer 3.0 | yes |

**Table 2: Solubility of samples after freezing for three days and thawing**

| **Sample of compound of formula (Ia)** | **Dissolved after freezing/thawing** |
|---|---|
| 100mM, buffer 6,5 | yes |
| 100mM, buffer 3.0 | yes |
| 5mM, buffer 6,5 | yes |
| 5mM, buffer 3.0 | yes |
| 1 mM, buffer 6.5 | yes |
| 1mM, buffer 3.0 | yes |

**Table 3: Solubility of samples after standing at room temperature for three days**

| **Sample of compound of formula (Ia)** | **Dissolved after three days at room temperature** |
|---|---|
| 100mM, buffer 6.5 | yes |
| 100mM, buffer 3.0 | yes |
| 5mM, buffer 6.5 | yes |
| 5mM, buffer 3.0 | yes |
| 1 mM, buffer 6.5 | yes |
| 1 mM, buffer 3.0 | yes |

The results of the solubility study are documented in three tables. Three categories for the state of solution are in generally used: "Yes", "Incomplete" and "No". The category "Yes" is only used in case of a clear flawless solution. The category "Incomplete" is used in case of a visible strong reduction of undissolved matter. The category "No" was used for all other cases.

The study showed a convincing solubility for the compound of formula (Ia), which was immediately dissolved at all conditions.

The following items are also subject-matter of the present invention:
1. A compound of formula (I), 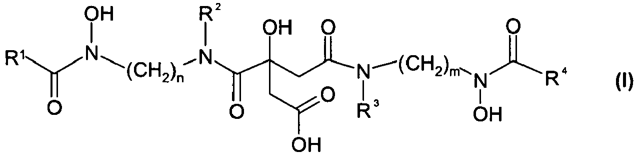 wherein
   R¹ and R⁴ are identical or different and are
      C₁-C₈-alkyl, C₁-C₈-alkoxy, C₂-C₈-alkenyl, C₂-C₈-alkynyl, C₃-C₈-cycloalkyl, C₃-C₈-cycloalkenyl, wherein each of the above mentioned radicals is unsubstituted or substituted by one or more radicals selected from the group consisting of halogen or hydroxyl,
      aryl, preferably phenyl or naphthyl, wherein the aryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy, or
      heteroaryl, wherein the heteroaryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy,
   R² and R³ are identical or different and are hydrogen or C₁-C₈-alkyl and,
   n and m are identical or different and are an integer from 1 to 5,
   or a stereoisomer or a salt or a hydrate thereof,
   with the exception of 4-[5-(acetylhydroxyamino)pentylamino]-2-[2-[5-(acetylhydroxyamino)pentylamino]-2-oxoethyl]-2-hydroxy-4-oxobutyric acid (Terregens factor, arthrobactin) and 3-[3-(acetylhydroxyamino)propylcarbamoyl]-2-[3-(acetylhydroxyamino)propylcarbamoylmethyl]-2-hydroxypropionic acid (schizokinen).
2. The compound of item 1, wherein
   R¹ and R⁴ are identical or different and are
      C₁-C₄-alkyl, C₁-C₄-alkoxy, C₂-C₄-alkenyl, C₂-C₄-alkynyl, C₃-C₆-cycloalkyl, C₃-C₆-cycloalkenyl, wherein each of the above mentioned radicals is unsubstituted or substituted by one or more radicals selected from the group consisting of halogen or hydroxyl,
      phenyl or naphthyl, wherein the phenyl or naphthyl is unsubstituted or substituted by one or more radicals selected from the group consisting of hydroxy, cyano, nitro, halogen, halogen-C₁-C₄-alkyl, halogen-C₁-C₄-alkoxy, C₁-C₄-alkyl and C₁-C₄-alkoxy, or
      heteroaryl, wherein the heteroaryl is unsubstituted or substituted by one or more radicals selected from the group consisting of hydroxy, cyano, nitro, halogen, halogen-C₁-C₄-alkyl, halogen-C₁-C₄-alkoxy, C₁-C₄-alkyl and C₁-C₄-alkoxy,
   R² and R³ are identical or different and are hydrogen or C₁-C₄-alkyl and,
   n and m are identical or different and are an integer from 1 to 5.
3. The compound of item 1 or 2, wherein
   R¹ and R⁴ are identical or different and are
      C₁-C₄-alkyl, C₁-C₄-alkoxy, C₃-C₆-cycloalkyl, wherein each of the above mentioned radicals is unsubstituted or substituted by one or more radicals selected from the group consisting of halogen or hydroxyl, or
      phenyl, wherein the phenyl is unsubstituted or substituted by one or more radicals selected from the group consisting of hydroxy, cyano, nitro, halogen, halogen-C₁-C₄-alkyl, halogen-C₁-C₄-alkoxy, C₁-C₄-alkyl and C₁-C₄-alkoxy,
   R² and R³ are identical or different and are hydrogen or methyl and,
   n and m are identical or different and are an integer from 3 to 5.
4. The compound of any one of items 1 to 3, wherein n and m are different.
5. The compound of any one of items 1 to 4, wherein n is 5 and m is 3.
6. The compound of any one of items 1 to 5, wherein R¹ and R⁴ are identical.
7. The compound of any one of items 1 to 6, wherein R¹ and R⁴ are C₁-C₄-alkyl.
8. The compound of any one of items 1 to 7, wherein R² and R³ are identical.
9. The compound of any one of items 1 to 8, wherein R² and R³ are hydrogen.
10. The compound of any one of items 1 to 9, wherein the compound of formula (I) is the compound of formula (Ia) 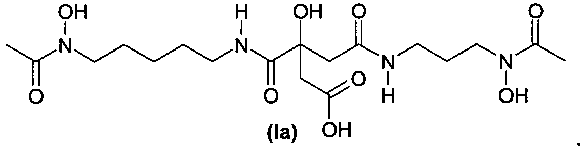
11. A sweetener composition, comprising
   at least one sweetener; and
   a compound of formula (I), 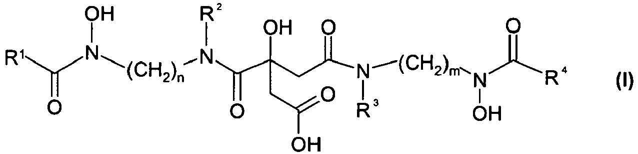 wherein
   R¹ and R⁴ are identical or different and are
      C₁-C₈-alkyl, C₁-C₈-alkoxy, C₂-C₈-alkenyl, C₂-C₈-alkynyl, C₃-C₈-cycloalkyl, C₃-C₈-cycloalkenyl, wherein each of the above mentioned radicals is unsubstituted or substituted by one or more radicals selected from the group consisting of halogen or hydroxyl,
      aryl, preferably phenyl or naphthyl, wherein the aryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy, or
      heteroaryl, wherein the heteroaryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy,
   R² and R³ are identical or different and are hydrogen or C₁-C₈-alkyl and,
   n and m are identical or different and are an integer from 1 to 5,
   or a stereoisomer or a salt or a hydrate thereof.
12. The sweetener composition of item 11, wherein 1 gram of the sweetener composition has a sweetness comparable to from one to three teaspoons of granulated sugar.
13. The sweetener composition of item 11 or 12, wherein 1 gram of the sweetener composition contains less calories and carbohydrates than about 1 gram of granulated sugar.
14. The sweetener composition of any one of items 11 to 13, wherein the compound of formula (I) or a stereoisomer or a salt or a hydrate thereof has no off-taste, as determined by a tasting panel, wherein the off-taste is selected from the group consisting of metallic off-taste, acidic off-taste, astringent off taste, throat-burning off taste or liquorice off-taste..
15. The sweetener composition of item 14, wherein the sweetener composition is substantially free of off-taste, as determined by a tasting panel, , wherein the off-taste is selected from the group consisting of metallic off-taste, acidic off-taste, astringent off taste, throat-burning off taste or liquorice off-taste.
16. The sweetener composition of any one of items 11 to 15, wherein the sweetener composition is a liquid at ambient conditions.
17. The sweetener composition of any one of items 11 to 15, wherein the sweetener composition is a solid at ambient conditions.
18. The sweetener composition of any one of items 11 to 17, wherein the sweetener composition comprises homogeneous particles comprising the sweetener and the compound of formula (I) or a stereoisomer or a salt or a hydrate thereof.
19. The sweetener composition of item 18, wherein the sweetener particles have an average particle size of between about 50 microns and about 1250 microns.
20. The sweetener composition of any one of items 11 to 19, wherein the sweetener composition comprises a mixture of first particles comprising the sweetener and second particles comprising the compound of formula (I) or a stereoisomer or a salt or a hydrate thereof.
21. The sweetener composition of any one of items 11 to 201, comprising from 0.0005 wt% to 1.0 wt% of the compound of formula (I) or a stereoisomer or a salt or hydrate thereof, based on the total weight of the sweetener composition.
22. The sweetener composition of any one of items 11 to 21, wherein the at least one sweetener is selected from the group consisting ofabiziasaponin, abrusosides, in particular abrusoside A, abrusoside B, abrusoside C, abrusoside D, acesulfame potassium, advantame, albiziasaponin, alitame, aspartame, superaspartame, bayunosides, in particular bayunoside 1, bayunoside 2, brazzein, bryoside, bryonoside, bryonodulcoside, carnosifloside, carrelame, curculin, cyanin, chlorogenic acid, cyclamates and its salts, cyclocaryoside I, dihydroquercetin-3-acetate, dihydroflavenol, dulcoside, gaudichaudioside, glycyrrhizin, glycyrrhetin acid, gypenoside, hematoxylin, isomogrosides, in particular iso-mogroside V, lugduname, magap, mabinlins, micraculin, mogrosides (lo han guo), in particular mogroside IV and mogroside V, monatin and its derivatives, monellin, mukurozioside, naringin dihydrochalcone (NarDHC), neohesperidin dihydrochalcone (NDHC), neotame, osladin, pentadin, periandrin I-V, perillartine, D-phenylalanine, phlomisosides, in particular phlomisoside 1, phlomisoside 2, phlomisoside 3, phlomisoside 4, phloridzin, phyllodulcin, polpodiosides, polypodoside A, pterocaryosides, rebaudiosides, in particular rebaudioside A, rebaudioside B, rebaudioside C, rebaudioside D, rebaudioside F, rebaudioside G, rebaudioside H), rubusosides, saccharin and its salts and derivatives, scandenoside, selligueanin A, siamenosides, in particular siamenoside I, stevia, steviolbioside, stevioside and other steviol glycosides, strogines, in particular strogin 1, strogin 2, strogin 4, suavioside A, suavioside B, suavioside G, suavioside H, suavioside I, suavioside J, sucralose, sucronate, sucrooctate, talin, telosmoside A₁₅, thaumatin, in particular thaumatin I and II, trans-anethol, trans-cinnamaldehyde, trilobatin, D-tryptophane, erythritol, galactitol, hydrogenated starch syrups including maltitol and sorbitol syrups, inositols, isomalt, lactitol, maltitol, mannitol, xylitol, arabinose, dextrin, dextrose, fructose, high fructose corn syrup, fructooligosaccharides, fructooligosaccharide syrups, galactose, galactooligosaccharides, glucose, glucose and (hydrogenated) starch syrups/hydrolysates, isomaltulose, lactose, hydrolysed lactose, maltose, mannose, rhamnose, ribose, sucrose, tagatose, trehalose and xylose.
23. The sweetener composition of item 22, wherein the sweetener is acesulfame potassium, sucrose or fructose.
24. The sweetener composition of any one of items 11 to 23, wherein the sweetener composition comprises a first sweetener and a second sweetener.
25. The sweetener composition of item 24, wherein the first sweetener is fructose.
26. The sweetener composition of any one of items 11 to 25, wherein the at least one sweetener is an artificial sweetener.
27. The sweetener composition of any one of items 11 to 25, wherein the sweetener is a natural sweetener.
28. The sweetener composition according to any one of items 11 to 26, wherein the sweetener is acesulfame potassium or sucrose.
29. The sweetener composition according to any one of items 1 to 28, wherein the sweetener composition comprises a pregelatinized starch.
30. The sweetener composition of item 29, comprising from 80 wt% to 95 wt% of pregelatinized starch based on the total weight of the sweetener composition.
31. The sweetener composition of item 29 or 30, wherein the sweetener is absorbed or adsorbed onto the pregelatinized starch.
32. The sweetener composition of any one of items 29 to 31, wherein the sweetener composition comprises homogeneous particles comprising the sweetener and the pregelatinized starch.
33. The sweetener composition of any one of items 29 to 32, wherein the pregelatinized starch has a specific surface less than or equal to 0.5 m²/g.
34. The sweetener composition of any one of items 29 to 32, wherein the pregelatinized starch has a specific surface ranging from 0.05 m²/g to 0.5 m²/g
35. The sweetener composition of any one of items 29 to 32, wherein the pregelatinized starch is non-granular.
36. The sweetener composition of any one of items 29 to 32, wherein the pregelatinized starch is granular.
37. The sweetener composition of any one of items 29 to 32, wherein the pregelatinized starch comprises particles and at least 50% of the pregelatinized starch particles have a particle size between 50 to 500 micrometers.
38. A tabletop sweetener composition, comprising:
   (a) at least one sugar sweetener;
   (b) at least one sugar alcohol;
   (c) a compound of formula (I) 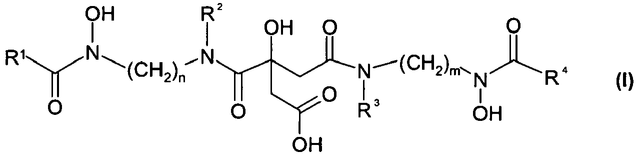 wherein
      R¹ and R⁴ are identical or different and are
         C₁-C₈-alkyl, C₁-C₈-alkoxy, C₂-C₈-alkenyl, C₂-C₈-alkynyl, C₃-C₈-cycloalkyl, C₃-C₈-cycloalkenyl, wherein each of the above mentioned radicals is unsubstituted or substituted by one or more radicals selected from the group consisting of halogen or hydroxyl,
         aryl, preferably phenyl or naphthyl, wherein the aryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy, or
         heteroaryl, wherein the heteroaryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy,
      R² and R³ are identical or different and are hydrogen or C₁-C₈-alkyl and,
      n and m are identical or different and are an integer from 1 to 5,
      or a stereoisomer or a salt or a hydrate thereof; and
   (d) cellulose.
39. The tabletop sweetener composition of item 38, comprising:
   (a) a disaccharide carbohydrate and/or fructose;
   (b) erythritol;
   (c) the compound of the formula (I) as defined in item 38,
   (d) cellulose.
40. The tabletop sweetener composition of item 38 or 39, wherein the disaccharide carbohydrate is selected from the group consisting of isomaltulose, lactose, maltose, sucrose, and trehalose
41. The tabletop sweetener composition of item 38 or 39, wherein the tabletop sweetener composition comprises from about 40 % by weight to about 70 % by weight erythritol.
42. The tabletop sweetener composition of item 38 or 39, wherein the tabletop sweetener composition comprises from about 27 % by weight to about 50 % by weight disaccharide.
43. The tabletop sweetener composition of item 38 or 39, wherein the tabletop sweetener composition comprises from about 0.5 % by weight to about 7.0 % by weight of a compound of formula (I) or a stereoisomer or a salt or a hydrate thereof.
44. The tabletop sweetener composition of item 38 or 39, wherein the tabletop sweetener composition comprises from about 0.4 % by weight to about 3.0 % by weight cellulose.
45. The tabletop sweetener composition of item 38 or 39 further comprising a sweetness modifier.
46. The tabletop sweetener composition of item 38 or 39 further comprising a mouthfeel enhancer.
47. The tabletop sweetener composition of item 38 or 39 further comprising a flavoring.
48. The tabletop sweetener composition of item 38 or 39, in the form of tabletop sweetener particles.
49. The tabletop sweetener composition of item 48, wherein the tabletop sweetener particles have an average particle size of between about 50 microns and about 1250 microns.
50. The tabletop sweetener composition of item 28, wherein the tabletop sweetener composition has less than about 5 calories per gram.
51. A tabletop sweetener composition, comprising:
   (a) a plurality of first sweetener particles, wherein the first sweetener particles have (i) a sugar alcohol core, (ii) a first sugar alcohol core-coating layer comprising a compound of the general formula (I), 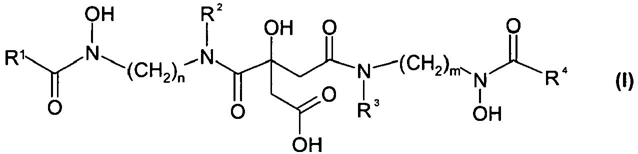 wherein
      R¹ and R⁴ are identical or different and are
         C₁-C₈-alkyl, C₁-C₈-alkoxy, C₂-C₈-alkenyl, C₂-C₈-alkynyl, C₃-C₈-cycloalkyl, C₃-C₈-cycloalkenyl, wherein each of the above mentioned radicals is unsubstituted or substituted by one or more radicals selected from the group consisting of halogen or hydroxyl,
         aryl, preferably phenyl or naphthyl, wherein the aryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy, or
         heteroaryl, wherein the heteroaryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy,
      R² and R³ are identical or different and are hydrogen or C₁-C₈-alkyl and,
      n and m are identical or different and are an integer from 1 to 5,
      or a stereoisomer or a salt or a hydrate thereof, and (iii) a second sugar alcohol core-coating layer comprising a sugar sweetener where the second sugar alcohol core-coating layer is disposed over the first sugar alcohol core-coating layer; and
   (b) a plurality of second sweetener particles, where the second sweetener particle has (i) a sugar sweetener core, (ii) a first sugar sweetener core-coating layer comprising the compound of formula (I) as defined above or a stereoisomer or a salt or a hydrate thereof and cellulose, and (iii) a second sugar sweetener core-coating layer comprising a disaccharide, where the second sugar sweetener core-coating layer is disposed over the first disaccharide core-coating layer.
52. The tabletop sweetener composition of item 51, comprising:
   (a) a plurality of first sweetener particles, wherein the first sweetener particles have (i) an erythritol core, (ii) a first erythritol core-coating layer comprising the compound of formula (I) as defined in item 51, or a stereoisomer or a salt or a hydrate thereof, and (iii) a second erythritol core-coating layer comprising a disaccharide carbohydrate, where the second erythritol core-coating layer lies outside of the first erythritol core-coating layer; and
   (b) a plurality of second sweetener particles, where the second sweetener particle has (i) a disaccharide core, (ii) a first disaccharide core-coating layer comprising a compound of formula (I) or a stereoisomer or a salt or a hydrate thereof and cellulose, and (iii) a second disaccharide core-coating layer comprising a disaccharide carbohydrate, where the second disaccharide core-coating layer lies outside of the first disaccharide core-coating layer.
53. The tabletop sweetener composition of item 51 or 52, wherein the disaccharide core comprises isomaltulose.
54. The tabletop sweetener composition of item 51 or 52, wherein the second erythritol core-coating layer comprises isomaltulose.
55. A tabletop sweetener composition of item 51 or 52, wherein the second disaccharide core-coating layer comprises isomaltulose.
56. A tabletop sweetener composition of item 51 or 52, wherein the first erythritol core-coating layer and the first disaccharide core-coating layer further comprise a flavoring.
57. The tabletop sweetener composition of item 51 or 52, wherein the first erythritol core-coating layer and the first disaccharide core-coating layer further comprise a mouthfeel enhancer.
58. The tabletop sweetener composition of item 51 or 52, wherein the first erythritol core-coating layer and the first disaccharide core-coating layer further comprise a sweetness modifier.
59. The tabletop sweetener composition of item 51 or 52, wherein the plurality of first sweetener particles and the plurality of second sweetener particles have an average particle size between about 50 microns and about 1250 microns.
60. A method of providing a sweetened consumable comprising the step of admixing with a consumable product a tabletop sweetener composition comprising:
   (i) at least one sugar sweetener;
   (ii) at least one sugar alcohol or polyol;
   (iii) a compound of the general formula (I), 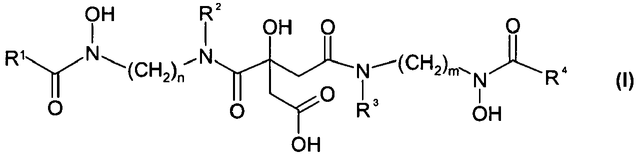 wherein
      R¹ and R⁴ are identical or different and are
         C₁-C₈-alkyl, C₁-C₈-alkoxy, C₂-C₈-alkenyl, C₂-C₈-alkynyl, C₃-C₈-cycloalkyl, C₃-C₈-cycloalkenyl, wherein each of the above mentioned radicals is unsubstituted or substituted by one or more radicals selected from the group consisting of halogen or hydroxyl,
         aryl, preferably phenyl or naphthyl, wherein the aryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy, or
         heteroaryl, wherein the heteroaryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy,
      R² and R³ are identical or different and are hydrogen or C₁-C₈-alkyl and,
      n and m are identical or different and are an integer from 1 to 5,
      or a stereoisomer or a salt or a hydrate thereof; and
   (iv) cellulose.
61. The method of providing a sweetened consumable of item 60 comprising the step of admixing with a consumable product a tabletop sweetener composition comprising:
   (i) a disaccharide carbohydrate and/or fructose;
   (ii) erythritol;
   (iii) the compound of formula (I) as defined in item 60, or a stereoisomer or a salt or a hydrate thereof; and
   (iv) cellulose.
62. A method of enhancing the taste sensations associated with flavor ingredients, comprising the step of admixing a compound of formula (I) 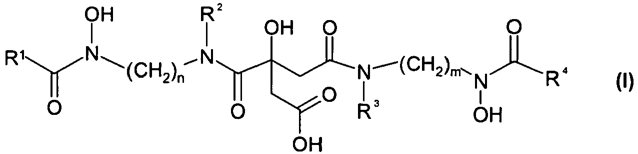 wherein
   R¹ and R⁴ are identical or different and are
      C₁-C₈-alkyl, C₁-C₈-alkoxy, C₂-C₈-alkenyl, C₂-C₈-alkynyl, C₃-C₈-cycloalkyl, C₃-C₈-cycloalkenyl, wherein each of the above mentioned radicals is unsubstituted or substituted by one or more radicals selected from the group consisting of halogen or hydroxyl,
      aryl, preferably phenyl or naphthyl, wherein the aryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy, or
      heteroaryl, wherein the heteroaryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy,
   R² and R³ are identical or different and are hydrogen or C₁-C₈-alkyl and,
   n and m are identical or different and are an integer from 1 to 5,
   or a stereoisomer or a salt or a hydrate thereof with one or more flavor ingredients to provide a flavor-enhanced composition or consumable product
63. The method of item 62, wherein the flavor ingredient is a sweetener.
64. The method of item 63, wherein the sweetener is selected from the group consisting of abiziasaponin, abrusosides, in particular abrusoside A, abrusoside B, abrusoside C, abrusoside D, acesulfame potassium, advantame, albiziasaponin, alitame, aspartame, superaspartame, bayunosides, in particular bayunoside 1, bayunoside 2, brazzein, bryoside, bryonoside, bryonodulcoside, carnosifloside, carrelame, curculin, cyanin, chlorogenic acid, cyclamates and its salts, cyclocaryoside I, dihydroquercetin-3-acetate, dihydroflavenol, dulcoside, gaudichaudioside, glycyrrhizin, glycyrrhetin acid, gypenoside, hematoxylin, isomogrosides, in particular iso-mogroside V, lugduname, magap, mabinlins, micraculin, mogrosides (lo han guo), in particular mogroside IV and mogroside V, monatin and its derivatives, monellin, mukurozioside, naringin dihydrochalcone (NarDHC), neohesperidin dihydrochalcone (NDHC), neotame, osladin, pentadin, periandrin I-V, perillartine, D-phenylalanine, phlomisosides, in particular phlomisoside 1, phlomisoside 2, phlomisoside 3, phlomisoside 4, phloridzin, phyllodulcin, polpodiosides, polypodoside A, pterocaryosides, rebaudiosides, in particular rebaudioside A, rebaudioside B, rebaudioside C, rebaudioside D, rebaudioside F, rebaudioside G, rebaudioside H), rubusosides, saccharin and its salts and derivatives, scandenoside, selligueanin A, siamenosides, in particular siamenoside I, stevia, steviolbioside, stevioside and other steviol glycosides, strogines, in particular strogin 1, strogin 2, strogin 4, suavioside A, suavioside B, suavioside G, suavioside H, suavioside I, suavioside J, sucralose, sucronate, sucrooctate, talin, telosmoside A₁₅, thaumatin, in particular thaumatin I and II, trans-anethol, trans-cinnamaldehyde, trilobatin, D-tryptophane, erythritol, galactitol, hydrogenated starch syrups including maltitol and sorbitol syrups, inositols, isomalt, lactitol, maltitol, mannitol, xylitol, arabinose, dextrin, dextrose, fructose, high fructose corn syrup, fructooligosaccharides, fructooligosaccharide syrups, galactose, galactooligosaccharides, glucose, glucose and (hydrogenated) starch syrups/hydrolysates, isomaltulose, lactose, hydrolysed lactose, maltose, mannose, rhamnose, ribose, sucrose, tagatose, trehalose and xylose.
65. A process for enhancing the sweetness of a sweetener composition comprising a sweetener, comprising the step of:
   (a) adding to the sweetener a compound of the general formula (I), 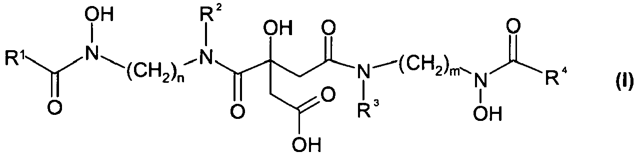 wherein
      R¹ and R⁴ are identical or different and are
         C₁-C₈-alkyl, C₁-C₈-alkoxy, C₂-C₈-alkenyl, C₂-C₈-alkynyl, C₃-C₈-cycloalkyl, C₃-C₈-cycloalkenyl, wherein each of the above mentioned radicals is unsubstituted or substituted by one or more radicals selected from the group consisting of halogen or hydroxyl,
         aryl, preferably phenyl or naphthyl, wherein the aryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy, or
         heteroaryl, wherein the heteroaryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy,
      R² and R³ are identical or different and are hydrogen or C₁-C₈-alkyl and,
      n and m are identical or different and are an integer from 1 to 5,
      or a stereoisomer or a salt or a hydrate thereof
   to form an enhanced sweetener composition.
66. The process of item 65, wherein the adding comprises adding to the sweetener a compound of formula (I) in an amount effective to increase the sweetness of the sweetener composition to an increased sweetness level.
67. A consumable product composition, comprising:
   a consumable product;
   a sweetener; and
   a compound of formula (I), 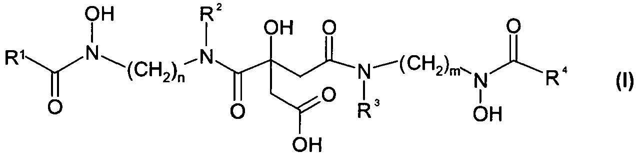 wherein
      R¹ and R⁴ are identical or different and are
         C₁-C₈-alkyl, C₁-C₈-alkoxy, C₂-C₈-alkenyl, C₂-C₈-alkynyl, C₃-C₈-cycloalkyl, C₃-C₈-cycloalkenyl, wherein each of the above mentioned radicals is unsubstituted or substituted by one or more radicals selected from the group consisting of halogen or hydroxyl,
         aryl, preferably phenyl or naphthyl, wherein the aryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy, or
         heteroaryl, wherein the heteroaryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy,
      R² and R³ are identical or different and are hydrogen or C₁-C₈-alkyl and,
      n and m are identical or different and are an integer from 1 to 5,
   or a stereoisomer or a salt or a hydrate thereof, present in an amount effective to increase a sweetness level of the composition.
68. The consumable product composition of item 67, wherein a compound of formula (I) or a stereoisomer or a salt or a hydrate thereof is present in the consumable product composition in a concentration from 0.1 wppm to 100 wppm.
69. The consumable product composition of item 67 or 68, wherein the consumable product is a water-based consumable product selected from the group consisting of beverages, water, aqueous beverages, enhanced/slightly sweetened water drinks, flavored carbonated and still mineral and table waters, carbonated beverages, non-carbonated beverages, carbonated waters, still waters, soft drinks, non-alcoholic drinks, alcoholic drinks, beer, wine, liquor, fruit drinks, juices, fruit juices, vegetable juices, broth drinks, coffees, teas, black teas, green teas, oolong teas, herbal infusions, cacoa, tea-based drinks, coffee-based drinks, cacao-based drinks, infusions, syrups, frozen fruits, frozen fruit juices, water-based ices, fruit ices, sorbets, dressings, salad dressings, jams, marmalades, canned fruit, savoury, delicatessen products like delicatessen salads, sauces, ketchup, mustard, pickles and marinated fish, sauces, soups, beverage botanical materials, sauces, soups, beverage botanical materials, and instant powders for reconstitution.
70. The consumable product composition of item 67 or 68, wherein the consumable product is a solid dry consumable product selected from the group consisting of cereals, baked food products, biscuits, breads, breakfast cereals, cereal bars, energy bars/nutritional bars, granolas, cakes, rice cakes, cookies, crackers, donuts, muffins, pastries, confection, chewing gums, chocolate products, chocolates, fondants, hard candies, marshmallows, pressed tablets, snack foods, botanical materials, and instant powders for reconstitution.
71. The consumable product composition of item 67 or 68, wherein the consumable product is a dairy product, dairy-derived product and/or dairy-alternative product selected from the group consisting of milk, fluid milk, cultured milk product, cultured and noncultured dairy-based drink, cultured milk product cultured with lactobacillus, yoghurt, yoghurt-based beverage, smoothie, lassi, milk shake, acidified milk, acidified milk beverage, butter milk, kefir, milk-based beverages, milk/juice blend, fermented milk beverage, icecream, dessert, sour cream, dip, salad dressing, cottage cheese, frozen yoghurt, soy milk, rice milk, soy drink, and rice milk drink.
72. The consumable product composition of item 67 or 68, wherein the consumable product is a carbonated drink.
73. The consumable product composition of item 67 or 68, wherein the consumable product is a non-carbonated drink.
74. The consumable product composition of item 67 or 68, wherein the consumable product is a cereal.
75. The consumable product composition of item 67 or 68, wherein the consumable product is a yoghurt.
76. The consumable product composition of item 67 or 68, wherein the consumable product is a chewing-gum.
77. The consumable product composition of item 67 or 68, wherein the consumable product is a dental product selected from the group consisting of toothpaste, dental floss, mouthwash, denture adhesive, enamel whitener, fluoride treatments and oral care gels.
78. The consumable product composition of item 67 or 68, wherein the consumable product is a toothpaste.
79. The consumable product composition of item 67 or 68, wherein the consumable product is a cosmetic product selected from the group consisting of lipstick, lip balm, lip gloss, and petroleum jelly.
80. The consumable product composition of item 67 or 68, wherein the consumable product is a pharmaceutical product selected from the group consisting of over-the-counter and prescription drugs, non-tobacco snuff, tobacco substitutes, chewable medications, cough syrups, throat sprays, throat lozenges, cough drops, antibacterial products, pill coatings, gel caplets, soluble fiber preparations, antacids, tablet cores, rapidly absorbed liquid compositions, stable foam compositions, rapidly disintegrating pharmaceutical dosage forms, beverage concentrates for medicinal purposes, aqueous pharmaceutical suspensions, liquid concentrate compositions, and stabilized sorbic acid solutions, phosphate buffers, saline solutions, emulsion, non-aqueous pharmaceutical solvents, aqueous pharmaceutical carriers, solid pharmaceutical carrier, and pharmaceutical preservatives/additives (antimicrobials, antioxidants, chelating agents, inert gases, flavoring agents, coloring agents).
81. The consumable product composition of item 67 or 68, wherein the consumable product is an animal feed or animal food.
82. The consumable product composition of any one of items 67 to 81, wherein the consumable product is an emulsion product comprising a sweetener, a compound of formula (I) or a stereoisomer or a salt or a hydrate thereof, a pregelatinized starch, and a carrier.
83. The consumable product composition of item 82, wherein the carrier comprises menthol.

## Claims

1. A compound of formula (I), 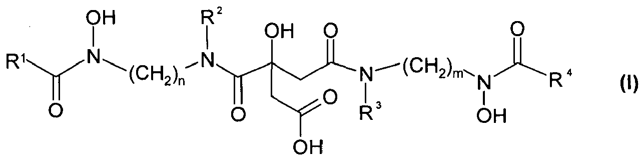 wherein
R¹ and R⁴ are identical or different and are
C₁-C₈-alkyl, C₁-C₈-alkoxy, C₂-C₈-alkenyl, C₂-C₈-alkynyl, C₃-C₈-cycloalkyl, C₃-C₈-cycloalkenyl, wherein each of the above mentioned radicals is unsubstituted or substituted by one or more radicals selected from the group consisting of halogen or hydroxyl,
aryl, preferably phenyl or naphthyl, wherein the aryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy, or
heteroaryl, wherein the heteroaryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy,
R² and R³ are identical or different and are hydrogen or C₁-C₈-alkyl and,
n and m are identical or different and are an integer from 1 to 5,
or a stereoisomer or a salt or a hydrate thereof.

2. The compound according to claim 1, wherein
R¹ and R⁴ are identical or different and are
C₁-C₄-alkyl, C₁-C₄-alkoxy, C₂-C₄-alkenyl, C₂-C₄-alkynyl, C₃-C₆-cycloalkyl, C₃-C₆-cycloalkenyl, wherein each of the above mentioned radicals is unsubstituted or substituted by one or more radicals selected from the group consisting of halogen or hydroxyl,
phenyl or naphthyl, wherein the phenyl or naphthyl is unsubstituted or substituted by one or more radicals selected from the group consisting of hydroxy, cyano, nitro, halogen, halogen-C₁-C₄-alkyl, halogen-C₁-C₄-alkoxy, C₁-C₄-alkyl and C₁-C₄-alkoxy, or
heteroaryl, wherein the heteroaryl is unsubstituted or substituted by one or more radicals selected from the group consisting of hydroxy, cyano, nitro, halogen, halogen-C₁-C₄-alkyl, halogen-C₁-C₄-alkoxy, C₁-C₄-alkyl and C₁-C₄-alkoxy,
R² and R³ are identical or different and are hydrogen or C₁-C₄-alkyl and,
n and m are identical or different and are an integer from 1 to 5.

3. The compound according to claim 1 or 2, wherein
R¹ and R⁴ are identical or different and are
C₁-C₄-alkyl, C₁-C₄-alkoxy, C₃-C₆-cycloalkyl, wherein each of the above mentioned radicals is unsubstituted or substituted by one or more radicals selected from the group consisting of halogen or hydroxyl, or
phenyl, wherein the phenyl is unsubstituted or substituted by one or more radicals selected from the group consisting of hydroxy, cyano, nitro, halogen, halogen-C₁-C₄-alkyl, halogen-C₁-C₄-alkoxy, C₁-C₄-alkyl and C₁-C₄-alkoxy,
R² and R³ are identical or different and are hydrogen or methyl and,
n and m are identical or different and are an integer from 3 to 5.

4. The compound according to any one of claims 1 to 3, wherein n and m are different, and/or
wherein n is 5 and m is 3, and/or
wherein R¹ and R⁴ are identical, in particular, wherein R¹ and R⁴ are C₁-C₄-alkyl, and/or
wherein R² and R³ are identical, in particular, wherein R² and R³ are hydrogen.

5. The compound according to any one of claims 1 to 4, wherein the compound of formula (I) is the compound of formula (Ia)

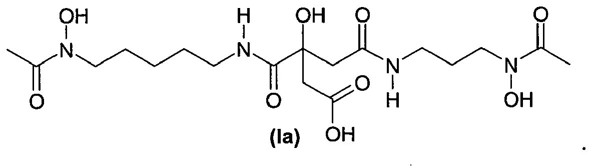

6. Use of a compound of formula (I) 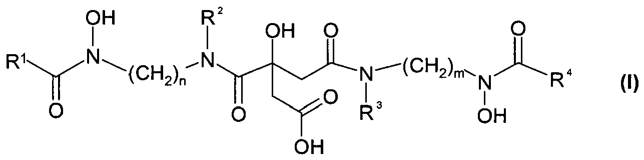 wherein
R¹ and R⁴ are identical or different and are
C₁-C₈-alkyl, C₁-C₈-alkoxy, C₂-C₈-alkenyl, C₂-C₈-alkynyl, C₃-C₈-cycloalkyl, C₃-C₈-cycloalkenyl, wherein each of the above mentioned radicals is unsubstituted or substituted by one or more radicals selected from the group consisting of halogen or hydroxyl,
aryl, preferably phenyl or naphthyl, wherein the aryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy, or
heteroaryl, wherein the heteroaryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy,
R² and R³ are identical or different and are hydrogen or C₁-C₈-alkyl and,
n and m are identical or different and are an integer from 1 to 5,
or a stereoisomer or a salt or a hydrate thereof as a sweetness enhancer.

7. A sweetener composition comprising
(a) at least one sweetener; and
(b) a compound of formula (I) 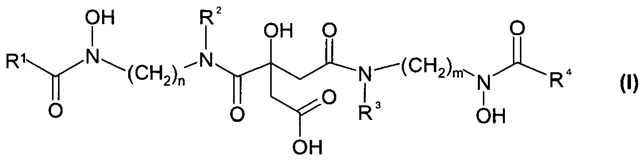 wherein
R¹ and R⁴ are identical or different and are
C₁-C₈-alkyl, C₁-C₈-alkoxy, C₂-C₈-alkenyl, C₂-C₈-alkynyl, C₃-C₈-cycloalkyl, C₃-C₈-cycloalkenyl, wherein each of the above mentioned radicals is unsubstituted or substituted by one or more radicals selected from the group consisting of halogen or hydroxyl,
aryl, preferably phenyl or naphthyl, wherein the aryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy, or
heteroaryl, wherein the heteroaryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy,
R² and R³ are identical or different and are hydrogen or C₁-C₈-alkyl and,
n and m are identical or different and are an integer from 1 to 5,
or a stereoisomer or a salt or a hydrate thereof.

8. The sweetener composition according to claim 7, wherein the compound of formula (I) or a stereoisomer or a salt or a hydrate thereof is a sweetness enhancer, wherein the at least one sweetener is preferably selected from the group consisting of abiziasaponin, abrusosides, in particular abrusoside A, abrusoside B, abrusoside C, abrusoside D, acesulfame potassium, advantame, albiziasaponin, alitame, aspartame, superaspartame, bayunosides, in particular bayunoside 1, bayunoside 2, brazzein, bryoside, bryonoside, bryonodulcoside, carnosifloside, carrelame, curculin, cyanin, chlorogenic acid, cyclamates and its salts, cyclocaryoside I, dihydroquercetin-3-acetate, dihydroflavenol, dulcoside, gaudichaudioside, glycyrrhizin, glycyrrhetin acid, gypenoside, hematoxylin, isomogrosides, in particular iso-mogroside V, lugduname, magap, mabinlins, micraculin, mogrosides (lo han guo), in particular mogroside IV and mogroside V, monatin and its derivatives, monellin, mukurozioside, naringin dihydrochalcone (NarDHC), neohesperidin dihydrochalcone (NDHC), neotame, osladin, pentadin, periandrin I-V, perillartine, D-phenylalanine, phlomisosides, in particular phlomisoside 1, phlomisoside 2, phlomisoside 3, phlomisoside 4, phloridzin, phyllodulcin, polpodiosides, polypodoside A, pterocaryosides, rebaudiosides, in particular rebaudioside A, rebaudioside B, rebaudioside C, rebaudioside D, rebaudioside F, rebaudioside G, rebaudioside H), rubusosides, saccharin and its salts and derivatives, scandenoside, selligueanin A, siamenosides, in particular siamenoside I, stevia, steviolbioside, stevioside and other steviol glycosides, strogines, in particular strogin 1, strogin 2, strogin 4, suavioside A, suavioside B, suavioside G, suavioside H, suavioside I, suavioside J, sucralose, sucronate, sucrooctate, talin, telosmoside A₁₅, thaumatin, in particular thaumatin I and II, trans-anethol, trans-cinnamaldehyde, trilobatin, D-tryptophane, erythritol, galactitol, hydrogenated starch syrups including maltitol and sorbitol syrups, inositols, isomalt, lactitol, maltitol, mannitol, xylitol, arabinose, dextrin, dextrose, fructose, high fructose corn syrup, fructooligosaccharides, fructooligosaccharide syrups, galactose, galactooligosaccharides, glucose, glucose and (hydrogenated) starch syrups/hydrolysates, isomaltulose, lactose, hydrolysed lactose, maltose, mannose, rhamnose, ribose, sucrose, tagatose, trehalose and xylose, and/or
wherein the at least one sweetener is acesulfame potassium or sucrose, and/or
wherein the sweetener composition comprises (c) a pregelatinized starch, and/or
wherein the composition further comprises at least one additional ingredient selected from bubble forming agents, bulking agents, carriers, fibers, sugar alcohols, flavorings, flavor enhancers, flavor stabilizers, acidulants, anti-caking and free-flow agents.

9. A tabletop sweetener composition comprising
(a) at least one sugar sweetener, which is selected from the group consisting of monosaccharides, disaccharides, oligosaccharides and polysaccharides, preferably the at least one sugar sweetener is selected from the group consisting of arabinose, dextrin, dextrose, fructose, high fructose corn syrup, fructooligosaccharides, fructooligosaccharide syrups, galactose, galactooligosaccharides, glucose, glucose and (hydrogenated) starch syrups/hydrolysates, isomaltulose, lactose, hydrolysed lactose, maltose, mannose, rhamnose, ribose, sucrose, stachyose, tagatose, trehalose, xylose, and combinations thereof, most preferably the at least one sugar sweetener is a disaccharide and/or fructose;
(b) at least one sugar alcohol (or polyol), which is selected from the group consisting of erythritol, galactitol, hydrogenated starch syrups including maltitol and sorbitol syrups, inositols, isomalt, lactitol, maltitol, mannitol, xylitol, and combinations thereof, preferably the at least one sugar alcohol is erythritol;
(c) a compound of formula (I) 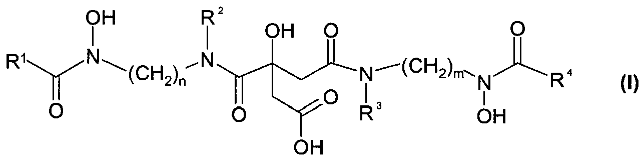 wherein
R¹ and R⁴ are identical or different and are
C₁-C₈-alkyl, C₁-C₈-alkoxy, C₂-C₈-alkenyl, C₂-C₈-alkynyl, C₃-C₈-cycloalkyl, C₃-C₈-cycloalkenyl, wherein each of the above mentioned radicals is unsubstituted or substituted by one or more radicals selected from the group consisting of halogen or hydroxyl,
aryl, preferably phenyl or naphthyl, wherein the aryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy, or
heteroaryl, wherein the heteroaryl is unsubstituted or substituted by one or more radicals selected from the group consisting of hydroxy, cyano, nitro, halogen, halogen-C₁-C₈-alkyl, halogen-C₁-C₈-alkoxy, C₁-C₈-alkyl and C₁-C₈-alkoxy,
R² and R³ are identical or different and are hydrogen or C₁-C₈-alkyl and,
n and m are identical or different and are an integer from 1 to 5,
or a stereoisomer or a salt or a hydrate thereof, and
(d) a taste-improving amount of cellulose.

10. The tabletop sweetener composition according to claim 9 comprising
(a) a disaccharide carbohydrate and/or fructose;
(b) erythritol;
(c) the compound of formula (I) as defined claim 9 or a stereoisomer or a salt or a hydrate thereof; and
(d) a taste-improving amount of cellulose.

11. The tabletop sweetener composition according to claim 9 or 10, wherein the disaccharide carbohydrate is selected from the group consisting of isomaltulose, lactose, maltose, sucrose, and trehalose, and/or
further comprising a sweetness modifier and/or
a mouthfeel enhancer and/or a flavoring, and/or
wherein the tabletop sweetener composition substantially comprises sweetener particles.

12. A consumable comprising
(a) a consumable product; and
(b) a sweetener composition as defined in claim 7 or 8.

13. A consumable comprising
(a) a consumable product; and
(b) a tabletop sweetener composition as defined in any one of claims 9 to 11.

14. The consumable according to claim 12 or 13, wherein the consumable product is selected from water-based consumables, solid dry consumables, dairy products, dairy-derived products and dairy-alternative products, and/or
wherein the consumable product is a water-based consumable product selected from the group consisting of beverage, water, aqueous beverage, enhanced/slightly sweetened water drink, flavored carbonated and still mineral and table water, carbonated beverage, non-carbonated beverage, carbonated water, still water, soft drink, non-alcoholic drink, alcoholic drink, beer, wine, liquor, fruit drink, juice, fruit juice, vegetable juice, broth drink, coffee, tea, black tea, green tea, oolong tea, herbal infusion, cacao, in particular water-based cacao, tea-based drink, coffee-based drinks, cacao-based drink, syrup, frozen fruit, frozen fruit juice, water-based ice, fruit ice, sorbet, dressing, salad dressing, jams, marmalades, canned fruit, savoury, delicatessen products like delicatessen salads, sauces, ketchup, mustard, pickles and marinated fish, sauce, soup, and beverage botanical materials, in particular whole or ground beverage botanical materials, and instant powder for reconstitution, in particular coffee beans, ground coffee, instant coffee, cacao beans, cacao powder, instant cacao, tea leaves, instant tea powder, and/or
wherein the consumable product is a solid dry consumable product selected from the group consisting of cereals, baked food products, biscuits, bread, breakfast cereal, cereal bar, energy bars/nutritional bars, granola, cakes, rice cakes, cookies, crackers, donuts, muffins, pastries, confectionaries, chewing gum, chocolate product, chocolate, fondant, hard candy, marshmallow, pressed tablets, snack foods, botanical materials, in particular whole or ground botanical materials, and instant powders for reconstitution, and/or
wherein the consumable product is a dairy product, dairy-derived product and/or dairy-alternative product selected from the group consisting of milk, fluid milk, cultured milk product, cultured and noncultured dairy-based drink, cultured milk product cultured with lactobacillus, yoghurt, yoghurt-based beverage, smoothie, lassi, milk shake, acidified milk, acidified milk beverage, butter milk, kefir, milk-based beverages, milk/juice blend, fermented milk beverage, ice cream, dessert, sour cream, dip, salad dressing, cottage cheese, frozen yoghurt, soy milk, rice milk, soy drink, and rice milk drink, and/or
wherein the consumable product is a carbonated drink or a non-carbonated drink or a cereal or yoghurt or a chewing-gum.

15. The consumable according to claim 12, wherein the consumable product is a dental product selected from the group consisting of toothpaste, dental floss, mouthwash, denture adhesive, enamel whitener, fluoride treatments and oral care gels, preferably toothpaste, or
wherein the consumable product is a cosmetic product selected from the group consisting of lipstick, lip balm, lip gloss and petroleum jelly, or
wherein the consumable product is a pharmaceutical product selected from the group consisting of over-the-counter and prescription drugs, non-tobacco snuff, tobacco substitutes, chewable medications, cough syrups, throat sprays, throat lozenges, cough drops, antibacterial products, pill coatings, gel caplets, soluble fiber preparations, antacids, tablet cores, rapidly absorbed liquid compositions, stable foam compositions, rapidly disintegrating pharmaceutical dosage forms, beverage concentrates for medicinal purposes, aqueous pharmaceutical suspensions, liquid concentrate compositions, and stabilized sorbic acid solutions, phosphate buffers, saline solutions, emulsion, non-aqueous pharmaceutical solvents, aqueous pharmaceutical carriers, solid pharmaceutical carrier, and pharmaceutical preservatives/additive, in particular pharmaceutical preservatives/additives such as antimicrobials, antioxidants, chelating agents, inert gases, flavoring agents and coloring agents, or
wherein the consumable product is animal feed or animal food.

## Patentansprüche

1. Verbindung der Formel (I), 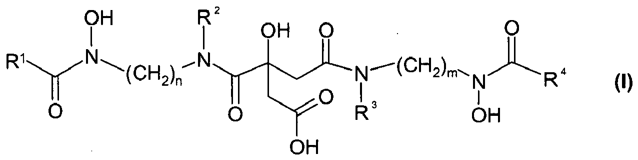 wobei
R¹ und R⁴ identisch oder unterschiedlich sind und
C₁-C₈-Alkyl, C₁-C₈-Alkoxy, C₂-C₈-Alkenyl, C₂-C₈-Alkinyl, C₃-C₈-Cycloalkyl, C₃-C₈-Cycloalkenyl sind, wobei jedes der oben genannten Radikale unsubstituiert oder mit einem oder mehreren Radikal/en ausgewählt aus der Gruppe bestehend aus Halogen oder Hydroxyl substituiert ist,
Aryl, vorzugsweise Phenyl oder Naphthyl sind, wobei das Aryl unsubstituiert oder mit einem oder mehren Radikal/en ausgewählt aus der Gruppe bestehend aus Hydroxy, Cyano, Nitro, Halogen, Halogen-C₁-C₈-Alkyl, Halogen-C₁-C₈-Alkoxy, C₁-C₈-Alkyl und C₁-C₈-Alkoxy substituiert ist, oder
Heteroaryl sind, wobei das Heteroaryl unsubstituiert oder mit einem oder mehreren Radikal/en ausgewählt aus der Gruppe bestehend aus Hydroxy, Cyano, Nitro, Halogen, Halogen-C₁-C₈-Alkyl, Halogen-C₁-C₈-Alkoxy, C₁-C₈-Alkyl und C₁-C₈-Alkoxy substituiert ist,
R² und R³ identisch oder unterschiedlich sind und Wasserstoff oder C₁-C₈ Alkyl sind und,
n und m identisch oder unterschiedlich sind und eine ganze Zahl von 1 bis 5 sind,
oder ein Stereoisomer oder ein Salz oder ein Hydrat davon sind.

2. Verbindung gemäß Anspruch 1, wobei
R¹ und R⁴ identisch oder unterschiedlich sind und
C₁-C₄-Alkyl, C₁-C₄-Alkoxy, C₂-C₄-Alkenyl, C₂-C₄-Alkinyl, C₃-C₆-Cycloalkyl, C₃-C₆-Cycloalkenyl sind, wobei jedes der oben genannten Radikale unsubstituiert oder mit einem oder mehreren Radikal/en ausgewählt aus der Gruppe bestehend aus Halogen oder Hydroxyl substituiert ist,
Phenyl oder Naphthyl sind, wobei das Phenyl oder Naphthyl unsubstituiert ist oder mit einem oder mehreren Radikal/en ausgewählt aus der Gruppe bestehend aus Hydroxy, Cyano, Nitro, Halogen, Halogen-C₁-C₄-Alkyl, Halogen-C₁-C₄-Alkoxy, C₁-C₄-Alkyl und C₁-C₄-Alkoxy substituiert ist, oder
Heteroaryl sind, wobei das Heteroaryl unsubstituiert oder mit einem oder mehreren Radikal/en ausgewählt aus der Gruppe bestehend aus Hydroxy, Cyano, Nitro, Halogen, Halogen-C₁-C₄-Alkyl, Halogen-C₁-C₄-Alkoxy, C₁-C₄-Alkyl und C₁-C₄-Alkoxy substituiert ist,
R² und R³ identisch oder unterschiedlich sind und Wasserstoff oder C₁-C₄-Alkyl sind, und
n und m identisch oder unterschiedlich sind und eine ganze Zahl von 1 bis 5 sind.

3. Verbindung gemäß Anspruch 1 oder 2, wobei
R¹ und R⁴ identisch oder unterschiedlich sind und
C₁-C₄-Alkyl, C₁-C₄-Alkoxy, C₃-C₆-Cycloalkyl sind, wobei jedes der oben genannten Radikale unsubstituiert oder mit einem oder mehreren Radikal/en ausgewählt aus der Gruppe bestehend aus Halogen oder Hydroxyl substituiert ist, oder
Phenyl sind, wobei das Phenyl unsubstituiert oder mit einem oder mehreren Radikal/en ausgewählt aus der Gruppe bestehend aus Hydroxy, Cyano, Nitro, Halogen, Halogen-C₁-C₄-Alkyl, Halogen-C₁-C₄-Alkoxy, C₁-C₄-Alkyl und C₁-C₄-Alkoxy substituiert ist,
R² und R³ identisch oder unterschiedlich sind und Wasserstoff oder Methyl sind, und
n und m identisch oder unterschiedlich sind und eine ganze Zahl von 3 bis 5 sind.

4. Verbindung gemäß irgendeinem der Ansprüche 1 bis 3, wobei n und m unterschiedlich sind und/oder
wobei n 5 ist und m 3 ist, und/oder
wobei R¹ und R⁴ identisch sind, insbesondere wobei R¹ und R⁴ C₁-C₄-Alkyl sind, und/oder
wobei R² und R³ identisch sind, insbesondere wobei R² und R³ Wasserstoff sind.

5. Verbindung gemäß irgendeinem der Ansprüche 1 bis 4, wobei die Verbindung der Formel (I) eine Verbindung der Formel (la) 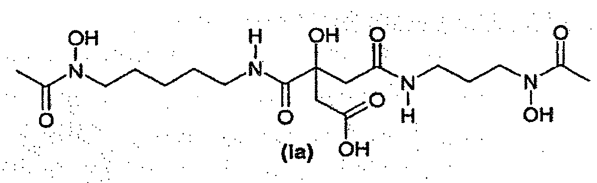 ist.

6. Verwendung der Verbindung der Formel (I) 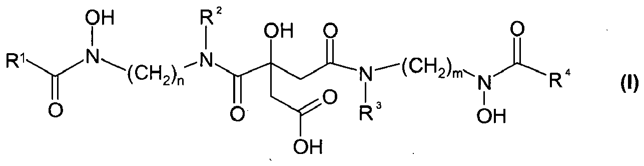 wobei
R¹ und R⁴ identisch oder unterschiedlich sind und
C₁-C₈-Alkyl, C₁-C₈-Alkoxy, C₂-C₈-Alkenyl, C₂-C₈-Alkinyl, C₃-C₈-Cycloalkyl, C₃-C₈-Cycloalkenyl sind, wobei jedes der oben genannten Radikale unsubstituiert oder mit einem oder mehreren Radikal/en ausgewählt aus der Gruppe bestehend aus Halogen oder Hydroxyl substituiert ist,
Aryl, vorzugsweise Phenyl oder Naphthyl, sind, wobei das Aryl unsubstituiert oder mit einem oder mehreren Radikal/en ausgewählt aus der Gruppe bestehend aus Hydroxy, Cyano, Nitro, Halogen, Halogen-C₁-C₈-Alkyl, Halogen-C₁-C₈-Alkoxy, C₁-C₈-Alkyl und C₁-C₈-Alkoxy substituiert ist, oder
Heteroaryl sind, wobei das Heteroaryl unsubstituiert oder mit einem oder mehreren Radikal/en ausgewählt aus der Gruppe bestehend aus Hydroxy, Cyano, Nitro, Halogen, Halogen-C₁-C₈-Alkyl, Halogen-C₁-C₈-Alkoxy, C₁-C₈-Alkyl und C₁-C₈-Alkoxy substituiert ist,
R² und R³ identisch oder unterschiedlich sind und Wasserstoff oder C₁-C₈-Alkyl sind, und
n und m identisch oder unterschiedlich und eine ganze Zahl von 1 bis 5 sind,
oder ein Stereoisomer oder ein Salz oder ein Hydrat davon als ein Süßkraftverstärker.

7. Süßstoffzusammensetzung umfassend
(a) mindestens einen Süßstoff; und
(b) eine Verbindung der Formel (I) 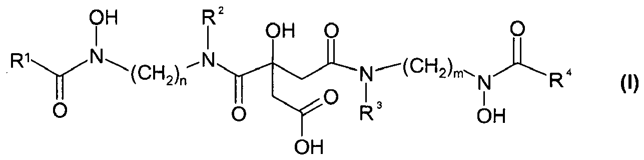 wobei
R¹ und R⁴ identisch oder unterschiedlich sind und
C₁-C₈-Alkyl, C₁-C₈-Alkoxy, C₂-C₈-Alkenyl, C₂-C₈-Alkinyl, C₃-C₈-Cycloalkyl, C₃-C₈-Cycloalkenyl sind, wobei jedes der oben genannten Radikale unsubstituiert oder mit einem oder mehreren Radikalen ausgewählt aus der Gruppe bestehend aus Halogen oder Hydroxyl substituiert ist,
Aryl, vorzugsweise Phenyl oder Naphthyl, sind, wobei das Aryl unsubstituiert oder mit einem oder mehreren Radikal/en ausgewählt aus der Gruppe bestehend aus Hydroxy, Cyano, Nitro, Halogen, Halogen-C₁-C₈-Alkyl, Halogen-C₁-C₈-Alkoxy, C₁-C₈-Alkyl und C₁-C₈-Alkoxy substituiert ist, oder
Heteroaryl sind, wobei das Heteroaryl unsubstituiert oder mit einem oder mehreren Radikalen ausgewählt aus der Gruppe bestehend aus Hydroxy, Cyano, Nitro, Halogen, Halogen-C₁-C₈-Alkyl, Halogen-C₁-C₈-Alkoxy, C₁-C₈-Alkyl und C₁-C₈-Alkoxy substituiert ist,
R² und R³ identisch oder unterschiedlich sind und Wasserstoff oder C₁-C₈-Alkyl sind, und
n und m identisch oder unterschiedlich sind und eine ganze Zahl von 1 bis 5 sind,
oder ein Stereoisomer oder ein Salz oder ein Hydrat davon.

8. Süßstoffzusammensetzung gemäß Anspruch 7, wobei die Verbindung der Formel (I) oder ein Stereoisomer oder ein Salz oder ein Hydrat davon ein Süßkraftverstärker ist, wobei der mindestens eine Süßstoff vorzugsweise ausgewählt ist aus der Gruppe bestehend aus Abiziasaponin, Abrusosiden, insbesondere Abrusosid A, Abrusosid B, Abrusosid C, Abrusosid D, Acesulfamkalium, Advantam, Albiziasaponin, Alitam, Aspartam, Superaspartam, Bayunosiden, insbesondere Bayunosid 1, Bayunosid 2, Brazzein, Bryosid, Bryonosid, Bryonodulcosid, Carnosiflosid, Carrelam, Curculin, Cyanin, Chlorogensäure, Cyclamaten und deren Salzen, Cyclocaryosid I, Dihydroquercetin-3-acetat, Dihydroflavenol, Dulcosid, Gaudichaudiosid, Glycyrrhizin, Glycyrrhetinsäure, Gypenosid, Hämatoxylin, Isomogrosiden, insbesondere Iso-Mogrosid V, Lugdunam, Magap, Mabinlinen, Micraculin, Mogrosiden (lo han guo), insbesondere Mogrosid IV und Mogrosid V, Monatin und dessen Derivaten, Monellin, Mukuroziosid, Naringindihydrochalcon (NarDHC), Neohesperidindihydrochalcon (NDHC), Neotam, Osladin, Pentadin, Periandrin I-V, Perillartin, D-Phenylalanin, Phlomisosiden, insbesondere Phlomisosid 1, Phlomisosid 2, Phlomisosid 3, Phlomisosid 4, Phloridzin, Phyllodulcin, Polpodiosiden, Polypodosid A, Pterocarysiden, Rebaudiosiden, insbesondere Rebaudiosid A, Rebaudiosid B, Rebaudiosid C, Rebaudiosid D, Rebaudiosid F, Rebaudiosid G, Rebaudiosid H, Rubusosiden, Saccharin und dessen Salzen und Derivaten, Scandenosid, Selligueanin A, Siamenosiden, insbesondere Siamenosid I, Stevia, Steviolbiosid, Steviosid und anderen Steviolglycosiden, Stroginen, insbesondere Strogin 1, Strogin 2, Strogin 34, Suaviosid A, Suaviosid B, Suaviosid G, Suaviosid H, Suaviosid I, Suaviosid J, Sucralose, Sucronat, Sucrooctat, Talin, Telosmosid A₁₅, Thaumatin, insbesondere Thaumatin I und II, Transanethol, Trans-Cinnamaldehyd, Trilobatin, D-Tryptophan, Erythritol, Galactitol, hydrierten Stärkesirupen einschließlich Maltitol- und Sorbitolsirupen, Inositolen, Isomalz, Lacatitol, Maltitol, Mannitol, Xylitol, Arabinose, Dextrin, Dextrose, Fructose, Maissirup mit hohem Fruchtzuckergehalt, Fructooligosacchariden, Fructooligosaccharidsirupen, Galactose, Galactooligosacchariden, Glucose, Glucose und (hydrierten) Stärkesirupen/Hydrolysaten, Isomaltulose, Lactose, hydrolysierter Lactose, Maltose, Mannose, Rhamnose, Ribose, Sucrose, Tagatose, Trehalose und Xylose, und/oder
wobei der mindestens eine Süßstoff Acesulfamkalium oder Saccharose ist, und/oder
wobei die Süßstoffzusammensetzung (c) eine Quellstärke umfasst, und/oder
wobei die Zusammensetzung ferner mindestens einen zusätzlichen Inhaltsstoff, ausgewählt aus blasenbildenden Mitteln, Füllstoffen, Trägern, Fasern, Zuckeralkoholen, Aromastoffen, Aromaverstärkern, Aromastabilisatoren, Säuerungsmitteln, Antibackmitteln und Fließhilfsmitteln, umfasst.

9. Haushalts-Süßstoff-Zusammensetzung umfassend
(a) mindestens einen Zuckersüßstoff, der ausgewählt ist aus der Gruppe bestehend aus Monosacchariden, Disacchariden, Oligosacchariden und Polysacchariden, vorzugsweise ist der mindestens eine Zuckersüßstoff ausgewählt aus der Gruppe bestehend aus Arabinose, Dextrin, Dextrose, Fructose, Maissirup mit hohem Fruchtzuckergehalt, Fructooligosacchariden, Fructooligosaccharidesirupen, Galactose, Galactooligosacchariden, Glucose, Glucose und (hydrierten) Stärkesirupen/Hydrolysaten, Isomaltulose, Lactose, hydrolysierter Lactose, Maltose, Mannose, Rhamnose, Ribose, Sucrose, Stachyose, Tagatose, Trehalose, Xylose und Kombinationen davon, am meisten bevorzugte ist der mindestens eine Zuckersüßstoff ein Disaccharid und/oder Fructose;
(b) mindestens einen Zuckeralkohol (oder Polyol), der ausgewählt ist aus der Gruppe bestehend aus Erythritol, Galactitol, hydrierten Stärkesirupen inklusive Maltitol- und Sorbitolsirupen, Inositolen, Isomalz, Lactitol, Maltitol, Mannitol, Xylitol und Kombinationen davon, vorzugsweise ist der mindestens eine Zuckeralkohol Erythritol;
(c) eine Verbindung der Formel (I) 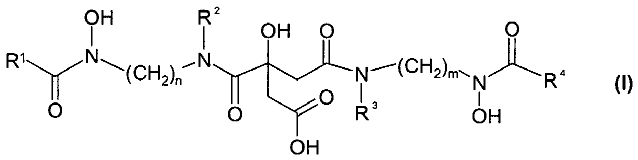 wobei
R¹ und R⁴ identisch oder unterschiedlich sind und
C₁-C₈-Alkyl, C₁-C₈-Alkoxy, C₂-C₈Alkenyl, C₂-C₈-Alkinyl, C₃-C₈-Cycloalkyl, C₃-C₈-Cycloalkenyl sind, wobei jedes der oben genannten Radikale unsubstituiert oder mit einem oder mehreren Radikal/en ausgewählt aus der Gruppe bestehend aus Halogen oder Hydroxyl substituiert ist, und
Aryl, vorzugsweise Phenyl oder Naphthyl, sind, wobei das Aryl unsubstituiert oder mit einem oder mehreren Radikal/en ausgewählt aus der Gruppe bestehend aus Hydroxy, Cyano, Nitro, Halogen, Halogen-C₁-C₈-Alkyl, Halogen-C₁-C₈-Alkoxy, C₁-C₈-Alkyl und C₁-C₈-Alkoxy substituiert ist, oder
Heteroaryl sind, wobei das Heteroaryl unsubstituiert oder mit einem oder mehreren Radikal/en ausgewählt aus der Gruppe bestehend aus Hydroxy, Cyano, Nitro, Halogen, Halogen-C₁-C₈-Alkyl, Halogen-C₁-C₈-Alkoxy, C₁-C₈-Alkyl und C₁-C₈-Alkoxy substituiert ist,
R² und R³ identisch oder unterschiedlich sind und Wasserstoff oder C₁-C₈-Alkyl sind, und
n und m identisch oder unterschiedlich sind und eine ganze Zahl von 1 bis 5 sind,
oder ein Stereoisomer oder ein Salz oder ein Hydrat davon sind, und
(d) eine geschmacksverbessernde Menge an Cellulose.

10. Haushalts-Süßstoff-Zusammensetzung gemäß Anspruch 9 umfassend
(a) ein Disaccharidcarbohydrat und/oder Fructose;
(b) Erythritol;
(c) die Verbindung der Formel (I) wie in Anspruch 9 definiert oder ein Stereoisomer oder ein Salz oder ein Hydrat davon; und
(d) eine geschmacksverbessernde Menge an Cellulose.

11. Haushalts-Süßstoff-Zusammensetzung gemäß Anspruch 9 oder 10, wobei das Disaccharidcarbohydrat ausgewählt ist aus der Gruppe bestehend aus Isomaltulose, Lactose, Maltose, Sucrose und Trehalose, und/oder
ferner umfassend einen Süßstoffmodifikator und/oder
einen Mundgefühlverbesserer und/oder einen Aromastoff, und/oder
wobei die Haushalts-Süßstoff-Zusammensetzung im Wesentlichen Süßstoffpartikel umfasst.

12. Verbrauchsartikel umfassend
(a) ein verzehrfähiges Produkt; und
(b) eine Süßstoffzusammensetzung wie in Anspruch 7 oder 8 definiert.

13. Verbrauchsartikel umfassend
(a) ein verzehrfähiges Produkt; und
(b) eine theoretische Süßstoffzusammensetzung wie in irgendeinem der Ansprüche 9 bis 11 definiert.

14. Verbrauchsartikel gemäß Anspruch 12 oder 13, wobei das verzehrfähige Produkt ausgewählt ist aus wasserbasierten Verbrauchsartikeln, festen trockenen Verbrauchsartikeln, Milchprodukten, von Milch abgeleiteten Produkten und Milch-Alternativprodukten, und/oder
wobei das verzehrfähige Produkt ein wasserbasiertes verzehrfähiges Produkt ist, ausgewählt aus der Gruppe bestehend aus Getränken, Wasser, wässerigen Getränken, stark/leicht gesüßten Wassergetränken, aromatisiertem kohlensäurehaltigem und stillem Mineral- und Tafelwasser, kohlensäurehaltigen Getränken, nicht kohlensäurehaltigen Getränken, kohlensäurehaltigem Wasser, stillem Wasser, Soft-Getränken, nichtalkoholischen Getränken, alkoholischen Getränken, Bier Wein, Schnaps, Fruchtgetränken, Saft, Fruchtsaft, Gemüsesaft, Brottrunk, Kaffee, Tee, schwarzem Tee, grünem Tee, Oolong-Tee, Kräutertee, Kakao, insbesondere wasserbasiertem Kakao, teebasierten Getränken, kaffeebasierten Getränken, kakaobasierten Getränken, Sirup, gefrorenem Obst, gefrorenem Fruchtsaft, wasserbasiertem Eis, Fruchteis, Sorbet, Dressing, Salatdressing, Konfitüren, Marmeladen, Obstkonserven, Häppchen (savoury), Delikatessprodukten wie Delikatesssalaten, Saucen, Ketchup, Senf, Pickles und mariniertem Fisch, Sauce, Suppe und Getränken botanischer Materialien, insbesondere Getränken ganzer oder gemahlener botanischer Materialien, und Instantpulver zur Wiederherstellung, insbesondere Kaffeebohnen, gemahlenem Kaffee, Instant-Kaffee, Kakaobohnen, Kakaopulver, Instant-Kakao, Teeblättern, Instant-Teepulver, und/oder
wobei das verzehrfähige Produkt ein festes trockenes verzehrfähiges Produkt, ausgewählt aus der Gruppe bestehend aus Zerealien, gebackenen Lebensmitteln, Keksen, Brot, Frühstückszerealien, Zerealienriegeln, Energieriegeln/Müsliriegeln, Müsli, Kuchen, Reiskuchen, Keksen, Crackern, Donuts, Muffin, Gebäck, Konfekt, Kaugummi, Schokoladeprodukten, Schokolade, Fondant, Bonbons, Marshmallow, gepressten Tabletten, Imbisswaren, botanischen Materialien, insbesondere ganzen oder gemahlenen botanischen Materialien und Instant-Pulvern zur Wiederherstellung, und/oder
wobei das verzehrfähige Produkt ein Milchprodukt, von Milch abgeleitetes Produkt und/oder Milch-Alternativprodukt ausgewählt aus der Gruppe bestehend aus Milch, flüssiger Milch, Sauermilchprodukt, vergorenem und nicht vergorenem milchbasiertem Getränk, mit Lactobacillus vergorenen Sauermilchprodukten , Joghurt, joghurtbasiertem Getränk, Smoothie, Lassi, Milchshake, angesäuerter Milch, angesäuertem Milchgetränk, Buttermilch, Kefir, milchbasierten Getränken, Milch/Saft-Mischung, fermentiertem Milchgetränk, Eiskrem, Dessert, Sauerrahm, Dip, Salatdressing, Hüttenkäse, gefrorenem Joghurt, Sojamilch, Reismilch, Sojadrink und Reismilchgetränk, und/oder
wobei das verzehrfähige Produkt ein kohlensäurehaltiges Getränk oder ein nicht kohlensäurehaltiges Getränk oder eine Zerealie oder ein Joghurt oder ein Kaugummi ist.

15. Verbrauchsartikel gemäß Anspruch 12, wobei das verzehrfähige Produkt ein Dentalprodukt ausgewählt aus der Gruppe bestehend aus Zahnpasta, Zahnseide, Mundwasser, Zahnprotheseklebemitteln, Zahnschmelzaufhellern, Fluoridbehandlungsmitteln und oralen Pflegegelen, vorzugsweise Zahnpasta ist, oder
wobei das verzehrfähige Produkt ein Kosmetikprodukt ausgewählt aus der Gruppe bestehend aus Lippenstift, Lippenbalsam, Lip-Gloss und Vaseline ist, oder
wobei das verzehrfähige Produkt ein pharmazeutisches Produkt ausgewählt aus der Gruppe bestehend aus nicht verschreibungspflichtigen und verschreibungspflichtigen Arzneimitteln, nicht auf Tabak basiertem Schnupftabak, Tabakersatzstoffen, kaubaren Arzneimitteln, Hustensirupen, Halssprays, Halsbonbons, Hustentropfen, antibakteriellen Produkten, Tablettenüberzügen, Gelkapseln, löslichen Faserpräparaten, Magensäuremitteln, Tablettenkernen, schnell absorbierten flüssigen Zusammensetzungen, stabilen Schaumzusammensetzungen, schnell sich auflösenden pharmazeutischen Dosierungsformen, Getränkkonzentraten für medizinische Zwecke, wässrigen pharmazeutischen Suspensionen, flüssigen Konzentratzusammensetzungen und stabilisierten Sorbinsäurelösungen, Phosphatpuffer, Laugelösungen, Emulsion, nicht-wässrigen pharmazeutischen Lösungsmitteln, wässrigen pharmazeutischen Trägern, festen pharmazeutischen Trägern und pharmazeutischen Konservierungsmitteln/Additiven, insbesondere pharmazeutischen Konservierungsmitteln/Additiven, wie antimikrobiellen Stoffen, Antioxidanzien, chelatisierenden Mitteln, Inertgasen, Geschmacksstoffen und Farbstoffen, oder
wobei das verzehrfähige Produkt Tierfuttermittel oder Tierfutter ist.

## Revendications

1. Composé de formule (I), 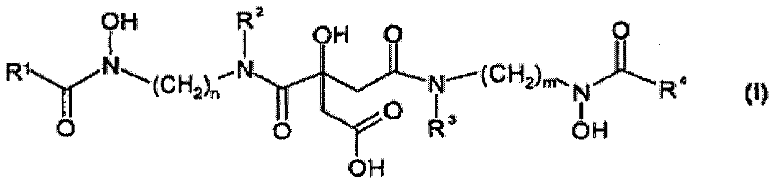 dans laquelle
R¹ et R⁴ sont identiques ou différents et sont
un alkyle en C₁ à C₈, un alcoxy en C₁ à Cg, un alcényle en C₂ à C₈, un alcynyle en C₂ à C₈, un cycloalkyle en C₃ à C₈, un cycloalcényle en C₃ à C₈, dans lequel chacun des radicaux susmentionnés est non substitué ou substitué avec un ou plusieurs radicaux choisis dans le groupe constitué par un halogène ou un hydroxyle,
un aryle, de préférence le phényle ou le naphtyle, dans lequel l'aryle est non substitué ou substitué avec un ou plusieurs radicaux choisis dans le groupe constitué par un hydroxy, un cyano, un nitro, un halogène, un halogène-alkyle en C₁ à C₈, un halogène-alcoxy en C₁ à C₈, un alkyle en C₁ à C₈ et un alcoxy en C₁ à C₈, ou
un hétéroaryle, dans lequel l'hétéroaryle est non substitué ou substitué avec un ou plusieurs radicaux choisis dans le groupe constitué par un hydroxy, un cyano, un nitro, un halogène, un halogène-alkyle en C₁ à C₈, un halogène-alcoxy en C₁ à C₈, un alkyle en C₁ à C₈ et un alcoxy en C₁ à C₈,
R² et R³ sont identiques ou différents et sont un hydrogène ou un alkyle en C₁ à C₈, et n et m sont identiques ou différents et sont des nombres entiers allant de 1 à 5,
ou un stéréoisomère ou un sel ou un hydrate de celui-ci.

2. Composé selon la revendication **1**, dans lequel
R¹ et R⁴ sont identiques ou différents et sont
un alkyle en C₁ à C₄, un alcoxy en C₁ à C₄, un alcényle en C₂ à C₄, un alcynyle en C₂ à C₄, un cycloalkyle en C₃ à C₆, un cycloalcényle en C₃ à C₆, dans lequel chacun des radicaux susmentionnés est non substitué ou substitué avec un ou plusieurs radicaux choisis dans le groupe constitué par un halogène ou un hydroxyle,
un phényle ou un naphtyle, dans lequel le phényle ou le naphtyle est non substitué ou substitué avec un ou plusieurs radicaux choisis dans le groupe constitué par un hydroxy, un cyano, un nitro, un halogène, un halogène-alkyle en C₁ à C₄, un halogène-alcoxy en C₁ à C₄, un alkyle en C₁ à C₄ et un alcoxy en C₁ à C₄, ou
un hétéroaryle, dans lequel l'hétéroaryle est non substitué ou substitué avec un ou plusieurs radicaux choisis dans le groupe constitué par un hydroxy, un cyano, un nitro, un halogène, un halogène-alkyle en C₁ à C₄, un halogène-alcoxy en C₁ à C₄, un alkyle en C₁ à C₄ et un alcoxy en C₁ à C₄,
R² et R³ sont identiques ou différents et sont un hydrogène ou un alkyle en C₁ à C₄ et, n et m sont identiques ou différents et sont des nombres entiers allant de 1 à 5.

3. Composé selon la revendication **1** ou **2**, dans lequel
R¹ et R⁴ sont identiques ou différents et sont
un alkyle en C₁ à C₄, un alcoxy en C₁ à C₄, un cycloalkyle en C₃ à C₆, dans lequel chacun des radicaux susmentionnés est non substitué ou substitué avec un ou plusieurs radicaux choisis dans le groupe constitué par un halogène ou un hydroxyle, ou
un phényle, dans lequel le phényle est non substitué ou substitué avec un ou plusieurs radicaux choisis dans le groupe constitué par un hydroxy, un cyano, un nitro, un halogène, un halogène-alkyle en C₁ à C₄, un halogène-alcoxy en C₁ à C₄, un alkyle en C₁ à C₄ et un alcoxy en C₁ à C₄,
R² et R³ sont identiques ou différents et sont un hydrogène ou un méthyle, et
n et m sont identiques ou différents et sont des nombres entiers allant de 3 à 5.

4. Composé selon l'une quelconque des revendications **1** à **3**, dans lequel n et m sont différents, et/ou
dans lequel n est 5 et m est 3, et/ou
dans lequel R¹ et R⁴ sont identiques, en particulier, dans lequel R¹ et R⁴ sont un alkyle en C₁ à C₄, et/ou
dans lequel R² et R³ sont identiques, en particulier, dans lequel R² et R³ sont un hydrogène.

5. Composé selon l'une quelconque des revendications 1 à 4, dans lequel le composé de formule (I) est le composé de formule (Ia)

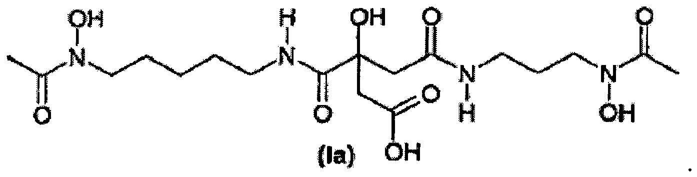

6. Utilisation d'un composé de formule (I) 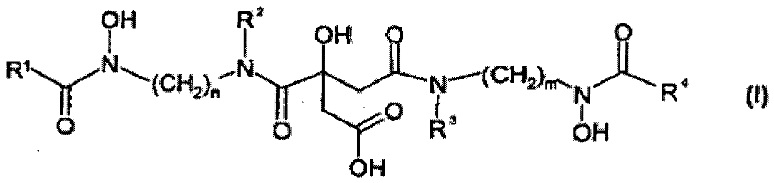 dans laquelle
R¹ et R⁴ sont identiques ou différents et sont
un alkyle en C₁ à C₈, un alcoxy en C₁ à C₈, un alcényle en C₂ à C₈, un alcynyle en C₂ à C₈, un cycloalkyle en C₃ à C₈, un cycloalcényle en C₃ à C₈, dans lequel chacun des radicaux susmentionnés est non substitué ou substitué avec un ou plusieurs radicaux choisis dans le groupe constitué par un halogène ou un hydroxyle,
un aryle, de préférence le phényle ou le naphtyle, dans lequel l'aryle est non substitué ou substitué avec un ou plusieurs radicaux choisis dans le groupe constitué par un hydroxy, un cyano, un nitro, un halogène, un halogène-alkyle en C₁ à C₈, un halogène-alcoxy en C₁ à C₈, un alkyle en C₁ à C₈ et un alcoxy en C₁ à C₈, ou
un hétéroaryle, dans lequel l'hétéroaryle est non substitué ou substitué avec un ou plusieurs radicaux choisis dans le groupe constitué par un hydroxy, un cyano, un nitro, un halogène, un halogène-alkyle en C₁ à C₈, un halogène-alcoxy en C₁ à C₈, un alkyle en C₁ à C₈ et un alcoxy en C₁ à C₈,
R² et R³ sont identiques ou différents et sont un hydrogène ou un alkyle en C₁ à C₈, et n et m sont identiques ou différents et sont des nombres entiers allant de 1 à 5,
ou d'un stéréoisomère ou d'un sel ou d'un hydrate de celui-ci en tant qu'exhausteur de goût sucré.

7. Composition édulcorante comprenant :
(a) au moins un édulcorant ; et
(b) un composé de formule (I) 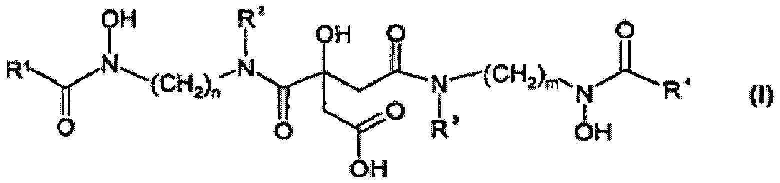 dans laquelle
R¹ et R⁴ sont identiques ou différents et sont
un alkyle en C₁ à C₈, un alcoxy en C₁ à C₈, un alcényle en C₂ à C₈, un alcynyle en C₂ à C₈, un cycloalkyle en C₃ à C₈, un cycloalcényle en C₃ à C₈, dans lequel chacun des radicaux susmentionnés est non substitué ou substitué avec un ou plusieurs radicaux choisis dans le groupe constitué par un halogène ou un hydroxyle,
un aryle, de préférence le phényle ou le naphtyle, dans lequel l'aryle est non substitué ou substitué avec un ou plusieurs radicaux choisis dans le groupe constitué par un hydroxy, un cyano, un nitro, un halogène, un halogène-alkyle en C₁ à C₈, un halogène-alcoxy en C₁ à C₈, un alkyle en C₁ à C₈ et un alcoxy en C₁ à C₈, ou
un hétéroaryle, dans lequel l'hétéroaryle est non substitué ou substitué avec un ou plusieurs radicaux choisis dans le groupe constitué par un hydroxy, un cyano, un nitro, un halogène, un halogène-alkyle en C₁ à C₈, un halogène-alcoxy en C₁ à C₈, un alkyle en C₁ à C₈ et un alcoxy en C₁ à C₈,
R² et R³ sont identiques ou différents et sont un hydrogène ou un alkyle en C₁ à C₈, et n et m sont identiques ou différents et sont des nombres entiers allant de 1 à 5,
ou un stéréoisomère ou un sel ou un hydrate de celui-ci.

8. Composition édulcorante selon la revendication **7**, dans laquelle le composé de formule (I) ou un stéréoisomère ou un sel ou un hydrate de celui-ci est un exhausteur de goût sucré, dans lequel ledit au moins un édulcorant est choisi de préférence dans le groupe constitué par l'abiziasaponine, les abrusosides, en particulier l'abrusoside A, l'abrusoside B, l'abrusoside C, l'abrusoside D, l'acésulfame de potassium, l'advantame, l'albiziasaponine, l'alitame, l'aspartame, le superaspartame, les bayunosides, en particulier le bayunoside 1, le bayunoside 2, la brazzéine, le bryoside, le bryonoside, le bryonodulcoside, le carnosifloside, le carrélame, la curculine, la cyanine, l'acide chlorogénique, les cyclamates et leurs sels, le cyclocaryoside I, la dihydroquercétine-3-acétate, le dihydroflavénol, le dulcoside, le gaudichaudioside, la glycyrrhizine, l'acide glycyrrhétinique, le gypénoside, l'hématoxyline, les isomogrosides, en particulier l'isomogroside V, le lugduname, le magap, les mabinlines, la micraculine, les mogrosides (lo han guo), en particulier le mogroside IV et le mogroside V, la monatine et ses dérivés, la monelline, le mukurozioside, la naringine dihydrochalcone (NarDHC), la néohespéridine dihydrochalcone (NDHC), le néotame, l'osladine, la pentadine, la périandrine I-V, la périllartine, la D-phénylalanine, les phlomisosides, en particulier le phlomisoside 1, le phlomisoside 2, le phlomisoside 3, le phlomisoside 4, la phloridzine, la phyllodulcine, les polpodiosides, le polypodoside A, les ptérocaryosides, les rébaudiosides, en particulier le rébaudioside A, le rébaudioside B, le rébaudioside C, le rébaudioside D, le rébaudioside F, le rébaudioside G, le rébaudioside H, les rubusosides, la saccharine et ses sels et dérivés, le scandénoside, la selliguéanine A, les siaménosides, en particulier le siaménoside I, la stévia, le stéviolbioside, le stévioside et d'autres glycosides de stéviol, les strogines, en particulier la strogine 1, la strogine 2, la strogine 4, le suavioside A, le suavioside B, le suavioside G, le suavioside H, le suavioside I, le suavioside J, le sucralose, le sucronate, le sucrooctate, la taline, le télosmoside A₁₅, la thaumatine, en particulier les thaumatines I et II, le trans-anéthol, le trans-cinnamaldéhyde, la trilobatine, le D-tryptophane, l'érythritol, le galactitol, un sirop d'amidon hydrogéné tel que les sirops de maltitol et de sorbitol, les inositols, l'isomalt, le lactitol, le maltitol, le mannitol, le xylitol, l'arabinose, la dextrine, le dextrose, le fructose, un sirop de maïs à teneur élevée en fructose, les fructooligosaccharides, les sirops de fructooligosaccharide, le galactose, les galactooligosaccharides, le glucose, les sirops/hydrolysats de glucose et d'amidon (hydrogéné), l'isomaltulose, le lactose, le lactose hydrolysé, le maltose, le mannose, le rhamnose, le ribose, le saccharose, le tagatose, le tréhalose et le xylose, et/ou
dans laquelle ledit au moins un édulcorant est l'acésulfame de potassium ou le saccharose, et/ou
dans laquelle la composition édulcorante comprend (c) un amidon prégélatinisé, et/ou dans laquelle la composition comprend en outre au moins un ingrédient supplémentaire choisi parmi les agents formant des bulles, les agents de charge, les supports, les fibres, les alcools de sucre, les arômes, les exhausteurs d'arôme, les stabilisants d'arôme, les acidulants, les agents anti-agglomérant et les agents fluidisant.

9. Composition édulcorante de table, comprenant :
a) au moins un édulcorant de sucre, qui est choisi dans le groupe constitué par les monosaccharides, les disaccharides, les oligosaccharides et les polysaccharides, de préférence ledit au moins un édulcorant de sucre est choisi dans le groupe constitué par l'arabinose, la dextrine, le dextrose, le fructose, un sirop de maïs à teneur élevée en fructose, les fructooligosaccharides, les sirops de fructooligosaccharide, le galactose, les galactooligosaccharides, le glucose, les sirops/hydrolysats de glucose et d'amidon (hydrogéné), l'isomaltulose, le lactose, le lactose hydrolysé, le maltose, le mannose, le rhamnose, le ribose, le saccharose, le stachyose, le tagatose, le tréhalose, le xylose et leurs combinaisons, de manière préférée entre toutes ledit au moins un édulcorant de sucre est un disaccharide et/ou le fructose ;
(b) au moins un alcool de sucre (ou polyol), qui est choisi dans le groupe constitué par l'érythritol, le galactitol, les sirops d'amidon hydrogéné tels que les sirops de maltitol et de sorbitol, les inositols, l'isomalt, le lactitol, le maltitol, le mannitol, le xylitol et leurs combinaisons, de préférence ledit au moins un alcool de sucre est l'érythritol ;
(c) un composé de formule (I) 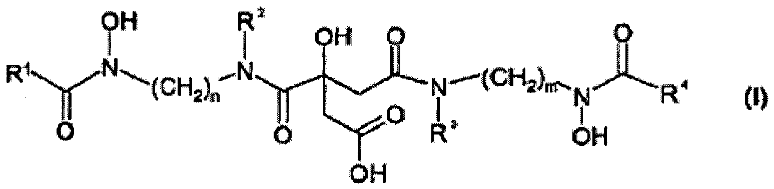 dans laquelle
R¹ et R⁴ sont identiques ou différents et sont
un alkyle en C₁ à C₈, un alcoxy en C₁ à C₈, un alcényle en C₂ à C₈, un alcynyle en C₂ à C₈, un cycloalkyle en C₃ à C₈, un cycloalcényle en C₃ à C₈, dans lequel chacun des radicaux susmentionnés est non substitué ou substitué avec un ou plusieurs radicaux choisis dans le groupe constitué par un halogène ou un hydroxyle,
un aryle, de préférence le phényle ou le naphtyle, dans lequel l'aryle est non substitué ou substitué avec un ou plusieurs radicaux choisis dans le groupe constitué par un hydroxy, un cyano, un nitro, un halogène, un halogène-alkyle en C₁ à C₈, un halogène-alcoxy en C₁ à C₈, un alkyle en C₁ à C₈ et un alcoxy en C₁ à C₈, ou
un hétéroaryle, dans lequel l'hétéroaryle est non substitué ou substitué avec un ou plusieurs radicaux choisis dans le groupe constitué par un hydroxy, un cyano, un nitro, un halogène, un halogène-alkyle en C₁ à C₈, un halogène-alcoxy en C₁ à C₈, un alkyle en C₁ à C₈ et un alcoxy en C₁ à C₈,
R² et R³ sont identiques ou différents et sont un hydrogène ou un alkyle en C₁ à C₈ et, n et m sont identiques ou différents et sont des nombres entiers allant de 1 à 5,
ou un stéréoisomère ou un sel ou un hydrate de celui-ci, et
(d) une quantité de cellulose améliorant le goût.

10. Composition édulcorante de table selon la revendication **9,** comprenant
(a) un disaccharide et/ou du fructose ;
(b) de l'érythritol ;
(c) le composé de formule (I) tel que défini dans la revendication **9** ou un stéréoisomère ou un sel ou un hydrate de celui-ci ; et
(d) une quantité de cellulose améliorant le goût.

11. Composition édulcorante de table selon la revendication **9** ou **10**, dans laquelle le disaccharide est choisi dans le groupe constitué par l'isomaltulose, le lactose, le maltose, le saccharose et le tréhalose, et/ou
comprenant en outre un modificateur d'édulcorant, et/ou
un exhausteur de sensation en bouche et/ou un arôme, et/ou
dans laquelle la composition édulcorante de table comprend essentiellement des particules d'édulcorant.

12. Consommable comprenant
(a) un produit consommable ; et
(b) une composition édulcorante telle que définie dans la revendication **7** ou **8**.

13. Consommable comprenant
(a) un produit consommable ; et
(b) une composition édulcorante de table telle que définie dans l'une quelconque des revendications **9** à **11**.

14. Consommable selon la revendication **12** ou **13**, dans lequel le produit consommable est choisi parmi les consommables à base d'eau, les consommables secs et solides, les produits laitiers, les produits dérivés du lait et les substituts de produits laitiers, et/ou dans lequel le produit consommable est un produit consommable à base d'eau choisi dans le groupe constitué par une boisson, l'eau, une boisson aqueuse, une boisson aqueuse améliorée/légèrement sucrée, une eau gazeuse aromatisée et une eau minérale plate aromatisée et une eau de table aromatisée, une boisson gazeuse, une boisson non gazeuse, une eau gazeuse, une eau plate, un soda, une boisson sans alcool, une boisson alcoolisée, une bière, un vin, une liqueur, une boisson au fruit, un jus, un jus de fruit, un jus de légume, un bouillon, un café, un thé, un thé noir, un thé vert, un thé oolong, une infusion de plante, du cacao, en particulier du cacao à base d'eau, une boisson à base de thé, une boisson à base de café, une boisson à base de cacao, un sirop, un fruit congelé, un jus de fruit congelé, une glace à base d'eau, une glace au fruit, un sorbet, une vinaigrette, une vinaigrette pour salade, une confiture, une marmelade, un fruit en conserve, un mets salé, un produit de traiteur tel que une salade traiteur, les sauces, le ketchup, la moutarde, les marinades et le poisson mariné, une sauce, une soupe et les matériels botaniques pour boisson, en particulier les matériels botaniques entiers ou broyés pour boisson, et une poudre instantanée pour reconstitution, en particulier les grains de café, le café moulu, le café instantané, les fèves de cacao, la poudre de cacao, le cacao instantané, les feuilles de thé, la poudre de thé instantané, et/ou dans lequel le produit consommable est un produit consommable sec et solide choisi dans le groupe constitué par les céréales, les produits alimentaires cuits au four, les biscuits, le pain, les céréales pour petit-déjeuner, une barre de céréale, les barres énergétiques/barres nutritionnelles, le granola, les gâteaux, les gâteaux de riz, les cookies, les craquelins, les beignets, les muffins, les viennoiseries, les confiseries, le chewing-gum, un produit au chocolat, le chocolat, un fondant, un bonbon dur, la guimauve, les comprimés, les aliments à grignoter, les matériels botaniques, en particulier les matériels botaniques entiers ou broyés, et les poudres instantanées pour reconstitution, et/ou
dans lequel le produit consommable est un produit laitier, un produit dérivé du lait et/ou un substitut de produit laitier choisi dans le groupe constitué par le lait, le lait de consommation, un produit laitier de culture, une boisson à base de lait cultivé et non cultivé, un produit à base de lait cultivé avec un lactobacillus, un yaourt, une boisson à base de yaourt, un smoothie, le lassi, le lait fouetté, le lait acidifié, une boisson à base de lait acidifié, le babeurre, le kéfir, les boissons à base de lait, un mélange lait/jus, une boisson à base de lait fermenté, une crème glacée, un dessert, une crème aigre, une sauce froide, une vinaigrette pour salade, le fromage blanc, le yaourt glacé, le lait de soja, le lait de riz, une boisson au soja et une boisson au lait de riz, et/ou dans lequel le produit consommable est une boisson gazeuse ou une boisson non gazeuse ou une céréale ou un yaourt ou un chewing-gum.

15. Consommable selon la revendication **12**, dans lequel le produit consommable est un produit dentaire choisi dans le groupe constitué par la pâte dentifrice, le fil dentaire, un bain de bouche, un adhésif pour prothèse dentaire, un blanchisseur d'émail, les traitements au fluor et les gels de soin buccal, de préférence la pâte dentifrice, ou
dans lequel le produit consommable est un produit cométique choisi dans le groupe constitué par le rouge à lèvres, une pommade pour les lèvres, un brillant à lèvres et une gelée de pétrole, ou
dans lequel le produit consommable est un produit pharmaceutique choisi dans le groupe constitué par les médicaments sans ordonnance et sur ordonnance, une substance à priser sans tabac, les substituts de tabac, les médicaments à croquer, les sirops contre la toux, les pulvérisations pour la gorge, les losanges pour la gorge, les gouttes contre la toux, les produits antibactériens, les enrobages de pilules, les comprimés en gel, les préparations de fibres solubles, les antiacides, les noyaux de comprimé, les compositions liquides rapidement absorbées, les compositions stables de mousse, les formes posologiques à délitement rapide, les concentrés buvables ayant des applications médicales, les suspensions pharmaceutiques aqueuses, les compositions liquides concentrées et les solutions d'acide sorbique stabilisées, les tampons au phosphate, les solutions salines, une émulsion, les solvants pharmaceutiques non aqueux, les supports pharmaceutiques aqueux, un support pharmaceutique solide et les conservateurs/additifs pharmaceutiques, en particulier les conservateurs/additifs pharmaceutiques tels que les antimicrobiens, les antioxydants, les agents de chélation, les gaz inertes, les agents aromatisants et les agents colorants, ou
dans lequel le produit consommable est un aliment pour animal ou de la nourriture pour animal.
